# EUROPEAN PATENT SPECIFICATION

(11) **EP 3 418 285 B1**
(45) Date of publication and mention of the grant of the patent: **06.05.2020**
(21) Application number: 17176829.4
(22) Date of filing: 20.06.2017
(51) Int. Cl.: C07F 15/00, C07D 495/06, C07D 519/00, C09K 11/06

(54) **COMPOSITION COMPRISING A SUBSTITUTED IR COMPLEX AND A PHENYLQUINAZOLINE BRIDGED WITH A HETEROATOM**
ZUSAMMENSETZUNG MIT EINEM SUBSTITUIERTEN IR-KOMPLEX UND EINEM MIT EINEM HETEROATOM VERBRÜCKTEN PHENYLCHINAZOLIN
COMPOSITION COMPRENANT UN COMPLEXE D'IR SUBSTITUÉ ET UNE PHENYLQUINAZOLINE PONTÉE AVEC UN HÉTÉROATOME

(43) Date of publication of application: 26.12.2018
(73) Proprietor: Idemitsu Kosan Co., Ltd., Chiyoda-ku, Tokyo 100-8321 (JP)
(72) Inventor: NISHIMAE, Yuichi, 4058 Basel (CH); NAKANO, Yuki, Sodegaura, Chiba, 299-0293 (JP); NAGASHIMA, Hideaki, Sodegaura, Chiba, 299-0293 (JP); KAWAMURA, Masahiro, 4057 Basel (CH); HAKETA, Tasuku, Sodegaura, Chiba, 299-0293 (JP); WOLLEB, Annemarie, 4232 Fehren (CH)
(74) Representative: Hollah, Dorothee

(56) References cited:
- EP-A1- 3 168 221
- WO-A1-2016/072743
- WO-A1-2017/109727
- KR-A- 20150 111 106

## Description

The present invention relates to compositions comprising a substituted Ir complex of formula (XX) and a compound of the general formula (I), to an emissive layer comprising said composition and to an electronic device comprising said composition.

Quinazolines and their use in electronic devices are known from the related art.

KR 20150111106 A discloses compounds according to the following formulae 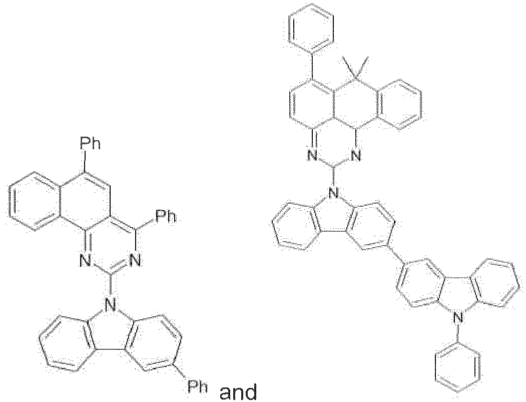 and their use as electroluminescent device materials.

EP 3 168 221 A1 relates to compounds of formula 1 and organic optoelectronic devices comprising the same:

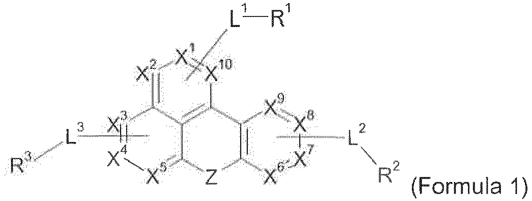

In all exemplified compounds Z is N-L⁴-R^{b}: As emitter, only (piq)₂Ir(acac) is mentioned in EP 3 168 221 A1. WO2017/109727 concerns compounds of general formula (I) 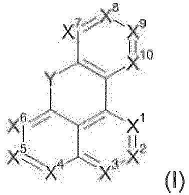 and their use in electronic devices. In the examples in PCT/IB2016/057889, the compounds of formula (I) are employed in combination with the emitter:

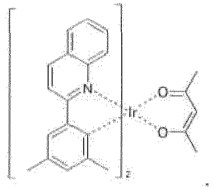

WO2016/072743 discloses an iridium derived complex which is however used in combination with a host quite different from the present one. There remains a need for electronic devices comprising new compositions, which are ideally matched, especially as an emitter material and a host material, to provide electronic devices having a good overall performance, especially improved efficiency and/or driving voltage.

Accordingly, it is an object of the present invention, with respect to the aforementioned related art, to provide compositions suitable for use in electronic devices, preferably OLEDs, and further applications in organic electronics, which are ideally matched. More particularly, it should be possible to provide electronic devices comprising new compositions for use in the light-emitting layer in OLEDs, especially as host material in combination with at least one substituted Ir complex which is preferably used as emitter.

Furthermore, the compositions should be suitable for providing electronic devices, preferably OLEDs, which ensure good efficiencies and/or a low use and operating voltage of the OLEDs. Said object is solved by a composition comprising
i) at least one Ir complex represented by the following formula (XX)

   IrLg₁Lg₂Lg₃ (XX)

   wherein
   Lg₁, Lg₂ and Lg₃ are selected from the following groups A-1, A-2 and A-3 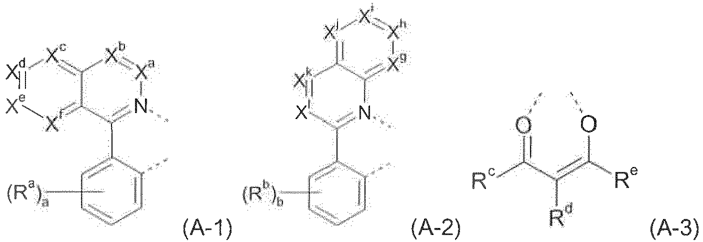
   wherein one or two of Lg₁, Lg₂ and Lg₃, preferably one, are a group A-3, and one or two of Lg₁, Lg₂ and Lg₃, preferably two, are selected from the group consisting of A-1 and A-2,
   wherein
   X^{a} is CR^{a'} or N;
   X^{b} is CR^{b'} or N;
   X^{c} is CR^{c'} or N;
   X^{d} is CR^{d'} or N;
   X^{e} is CR^{e'} or N;
   X^{f} is CR^{f} or N;
   X^{g} is CR^{g} or N;
   X^{h} is CR^{h} or N;
   Xⁱ is CRⁱ or N;
   X^{j} is CR^{j} or N;
   X^{k} is CR^{k} or N; and
   X^{l} is CR^{l} or N;
   wherein at least one of X^{a}, X^{b}, X^{c}, X^{d}, X^{e} and X^{f} is CR^{a'}, CR^{b'}, CR^{c'}, CR^{d'}, CR^{e'} respectively CR^{f}, and at least one of X^{g}, X^{h}, Xⁱ, X^{j}, X^{k} and X^{l} is CR^{g}, CR^{h}, CRⁱ, CR^{j}, CR^{k} respectively CR^{l};
   R^{a'}, R^{b'}, R^{e'}, R^{d'}, R^{e'}, R^{f}, R^{g}, R^{h}, Rⁱ, R^{j}, R^{k} and R^{l} each independently represent hydrogen, deuterium, a halogen, a cyano group, a substituted or unsubstituted C₁-C₂₅alkyl group, a substituted or unsubstituted C₃-C₂₅cycloalkyl group, a substituted or unsubstituted C₁-C₂₅Silyl group, a substituted or unsubstituted C₆-C₃₀aryl group, or a substituted or unsubstituted C₁-C₃₀ heteroaryl group;
   wherein at least one of R^{a'}, R^{b'}, R^{c'}, R^{d'}, R^{e'} and R^{f}, and at least one of R^{g}, R^{h}, Rⁱ, R^{j}, R^{k} and R^{l} is a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably a substituted or unsubstituted C₁-C₂₅alkyl group;
   R^{a} to R^{e} each independently represent hydrogen, deuterium, a halogen, a cyano group, a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably, R^{a} to R^{e} each independently represent hydrogen, deuterium, a substituted or unsubstituted C₁-C₁₀alkyl group, a substituted or unsubstituted C₃-C₁₀cycloalkyl group, a substituted or unsubstituted C₆-C₂₀ary group, or a substituted or unsubstituted C₁-C₂₀ heteroaryl group;
   a and b each independently represent an integer of 0 to 5; and
   each of R^{a} and R^{b} is the same or different;
   wherein the dotted lines are bonding sites;
   and
ii) at least one compound represented by the general formula (I) 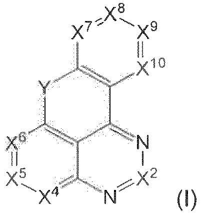 wherein
   X² is CR²,
   X⁴ is CR⁴ or N,
   X⁵ is CR⁵ or N,
   X⁶ is CR⁶ or N,
   X⁷ is CR⁷ or N,
   X⁸ is CR⁸ or N,
   X⁹ is CR⁹ or N,
   X¹⁰ is CR¹⁰ or N,
   Y is O or S,
   R², R⁴, R⁵, R⁶, R⁷, R⁸, R⁹, and R¹⁰ are independently of each other selected from H, deuterium, E, a C₆-C₃₀aryl group which is unsubstituted or substituted by at least one group E, C₂-C₂₄heteroaryl group which is unsubstituted or substituted by at least one group E a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
   at least two of R⁴, R⁵, R⁶, R⁷, R⁸, R⁹ or R¹⁰, if present at adjacent carbon atoms, together form at least one C₆-C₁₈aryl or C₂-C₁₈heteroaryl ring or ring system,
   D is independently of each other -CO-, -COO-, -S-, -SO-, -SO₂-, -O-,-CR¹⁵=CR¹⁶-, -NR¹⁷-,-SiR²²R²³-, -POR²⁵-, -C≡C-,
   E is independently of each other -OR²¹, -SR²¹, -NR¹⁷R¹⁸, -COR²⁰, -COOR¹⁹, -CONR¹⁷R¹⁸, -CN,-SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group,
   R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
   R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
   R¹⁷ and R¹⁸ together form a five or six membered aliphatic, aromatic or heteroaromatic ring, R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
   R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
   R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
   R²², R²³ and R²⁴ are independently of each other H, a C₁-C₁₈alkyl group, a C₆-C₁₈aryl group which is unsubstituted or substituted by at least one C₁-C₁₈alkyl group, and R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
   wherein one of R², R⁴, R⁵, R⁶, R⁷, R⁸, R⁹, R¹⁰, preferably R², in the compound of formula (I) is a N-heteroaryl group according to general formulae (XIII), (XV) or (XXI), preferably, R² in the compound of formula (I) is a N-heteroaryl group according to general formulae (XIII), (XV) or (XXI); 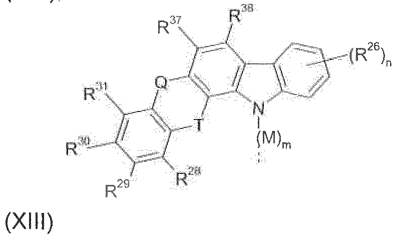
   wherein n is 0, 1, 2, 3 or 4,
   m is 0, 1, 2, 3 or 4,
   M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
   R²⁶ is independently of each other selected from E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
   or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
   R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
   or
   at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and
   Q and T are independently of each other selected from direct bond, S, O, SiR³²R³³, CR³⁴R³⁵ and NR³⁶, wherein Q and T are not at the same time a direct bond;
   wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, more preferably H, methyl, ethyl, or phenyl,
   R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E or a spiro group, or R³⁴ and R³⁵ together may form a five or six membered, substituted or unsubstituted, aliphatic ring, preferably R³⁴ and R³⁵ are independently of each other H, methyl, ethyl, phenyl or a spiro group, or R³⁴ and R³⁵ together may form a five or six membered, substituted or unsubstituted, aliphatic ring,
   R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group,
   wherein the dotted line is a bonding site; 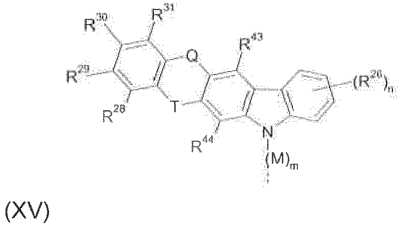
   wherein n is 0, 1, 2, 3 or 4,
   m is 0, 1, 2, 3 or 4,
   M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
   R²⁶ is independently of each other selected from E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
   or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
   or
   at least two of R²⁸, R²⁹, R³⁰ or R³¹, if present at adjacent carbon atoms, together may form at least one C₆-C₁₈aryl or C₂-C₁₈heteroaryl ring or ring system, and
   Q and T are independently of each other selected from direct bond, S, O, SiR³²R³³, CR³⁴R³⁵ and NR³⁶, wherein Q and T are not at the same time a direct bond;
   wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, more preferably H, methyl, ethyl or phenyl,
   R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E or a spiro group, or R³⁴ and R³⁵ together may form a five or six membered, substituted or unsubstituted, aliphatic ring, preferably, R³⁴ and R³⁵ are independently of each other H, ethyl, ethyl, phenyl or a spiro group, or R³⁴ and R³⁵ together may form a five or six membered, substituted or unsubstituted, aliphatic ring,
   R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, for example phenyl,
   wherein the dotted line is a bonding site, 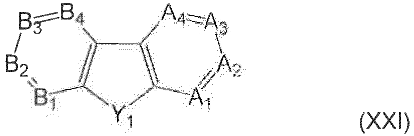
   wherein
   A¹ is CR⁶² or N,
   A² is CR⁶³ or N,
   A³ is CR⁶⁴ or N,
   A⁴ is CR⁶⁵ or N,
   B¹ is CR⁶⁶ or N,
   B² is CR⁶⁷ or N,
   B³ is CR⁶⁸ or N,
   B⁴ is CR⁶⁹ or N,
   Y¹ is NR⁷⁰, CR⁷¹R⁷², O or S;
   R⁶², R⁶³, R⁶⁴, R⁶⁵, R⁶⁶, R⁶⁷, R⁶⁸ and R⁶⁹ are independently of each other H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   or
   at least two of R⁶², R⁶³, R⁶⁴ and R⁶⁵ may be directly bonded to the moiety represented by the general formula (XXII) via the two *-locations; 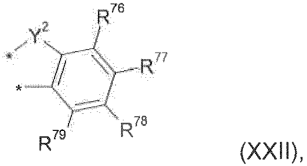
   wherein
   Y² is NR⁷³, CR⁷⁴R⁷⁵, O or S,
   or
   at least two of R⁶⁶, R⁶⁷, R⁶⁸ and R⁶⁹ may be directly bonded to the moiety represented by the general formula (XXIII) via the two *-locations. 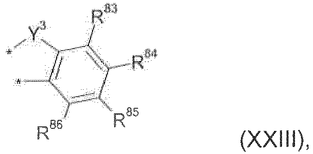
   wherein
   Y³ is NR⁸⁰, CR⁸¹R⁸², O or S,
   R⁷⁰, R⁷³ and R⁸⁰ are independently of each other a direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   R⁷¹, R⁷², R⁷⁴, R⁷⁵, R⁸¹ and R⁸² are, independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
   or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
   R⁷⁶, R⁷⁷, R⁷⁸, R⁷⁹, R⁸³, R⁸⁴, R⁸⁵ and R⁸⁶ are independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
   or
   R⁷⁶ and R⁷⁷, R⁷⁷ and R⁷⁸ and/or R⁷⁸ and R⁷⁹ together may form at least one C₆-C₁₈aryl or C₂-C₁₈heteroaryl ring or ring system, and/or
   R⁸³ and R⁸⁴, R⁸⁴ and R⁸⁵ and/or R⁸⁵ and R⁸⁶ together form at least one C₆-C₁₈aryl or C₂-C₁₈heteroaryl ring or ring system,
   wherein the heterocyclic group according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, wherein this respective residue R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
   m is 1, 2, 3 or 4,
   M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

The residues mentioned in the specification of the present application generally have the following preferred meanings, if said residues are not further specified in specific embodiments mentioned below:
Halogen is fluorine, chlorine, bromine and iodine.

C₁-C₂₅alkyl, preferably C₁-C₁₈alkyl, is typically linear or branched, where possible. Examples are methyl, ethyl, n-propyl, isopropyl, n-butyl, sec.-butyl, isobutyl, tert.-butyl, n-pentyl, 2-pentyl, 3-pentyl, 2,2-dimethylpropyl, 1,1,3,3-tetramethylpentyl, n-hexyl, 1-methylhexyl, 1,1,3,3,5,5-hexamethylhexyl, n-heptyl, isoheptyl, 1,1,3,3-tetramethylbutyl, 1-methylheptyl, 3-methylheptyl, n-octyl, 1,1,3,3-tetramethylbutyl and 2-ethylhexyl, n-nonyl, decyl, undecyl, dodecyl, tridecyl, tetradecyl, pentadecyl, hexadecyl, heptadecyl, or octadecyl. C₁-C₈alkyl is typically methyl, ethyl, n-propyl, isopropyl, n-butyl, sec.-butyl, isobutyl, tert.-butyl, n-pentyl, 2-pentyl, 3-pentyl, 2,2-dimethyl-propyl, n-hexyl, n-heptyl, n-octyl, 1,1,3,3-tetramethylbutyl and 2-ethylhexyl. C₁-C₄alkyl is typically methyl, ethyl, n-propyl, isopropyl, n-butyl, sec.-butyl, isobutyl, tert.-butyl.

C₁-C₂₅alkoxy groups, preferably C₁-C₁₈alkoxy groups, are straight-chain or branched alkoxy groups, e.g. methoxy, ethoxy, n-propoxy, isopropoxy, n-butoxy, sec-butoxy, tert-butoxy, amyloxy, isoamyloxy or tert-amyloxy, heptyloxy, octyloxy, isooctyloxy, nonyloxy, decyloxy, un-decyloxy, dodecyloxy, tetradecyloxy, pentadecyloxy, hexadecyloxy, heptadecyloxy and octadecyloxy. Examples of C₁-C₈alkoxy are methoxy, ethoxy, n-propoxy, isopropoxy, n-butoxy, sec.-butoxy, isobutoxy, tert.-butoxy, n-pentyloxy, 2-pentyloxy, 3-pentyloxy, 2,2-dimethylpropoxy, n-hexyloxy, n-heptyloxy, n-octyloxy, 1,1,3,3-tetramethylbutoxy and 2-ethylhexyloxy, preferably C₁-C₄alkoxy such as typically methoxy, ethoxy, n-propoxy, isopropoxy, n-butoxy, sec.-butoxy, isobutoxy, tert.-butoxy.

C₆-C₆₀aryl, preferably C₆-C₂₄aryl, particularly preferably C₆-C₁₈aryl, which optionally can be substituted, is typically phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, which may be unsubstituted or substituted. Phenyl, 1-naphthyl and 2-naphthyl are examples of a C₆-C₁₀aryl group.

C₆-C₂₄aryloxy, which optionally can be substituted, is typically C₆-C₁₀aryloxy, which optionally can be substituted by one, or more C₁-C₈alkyl and/or C₁-C₈alkoxy groups, such as, for example, phenoxy, 1-naphthoxy, or 2-naphthoxy.

C₁-C₆₀heteroaryl, preferably C₂-C₃₀heteroaryl, particularly preferably C₂-C₁₃heteroaryl, which optionally can be substituted, represents a ring with five to seven ring atoms or a condensed ring system, wherein nitrogen, oxygen or sulfur are the possible hetero atoms, and is typically a heterocyclic group with 5 to 40 atoms having at least six conjugated π-electrons such as thienyl, benzothiophenyl, dibenzothiophenyl, thianthrenyl, furyl, furfuryl, 2H-pyranyl, benzofuranyl, iso-benzofuranyl, dibenzofuranyl, phenoxythienyl, pyrrolyl, imidazolyl, pyrazolyl, pyridyl, bipyridyl, triazinyl, pyrimidinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbazolyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, 4-imidazo[1,2-a]benzimidazoyl, 5-benzimidazo[1,2-a]benzimidazoyl, benzimidazolo[2,1-b][1,3]benzothiazolyl, carbazolyl, or phenoxazinyl, which can be unsubstituted or substituted. Benzimidazo[1,2-a]benzimidazo-5-yl, benzimidazo[1,2-a]benzimidazo-2-yl, carbazolyl and dibenzofuranyl are examples of a C₂-C₁₄heteroaryl group.

C₇-C₂₅aralkyl, which optionally can be substituted, is for example benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl, ω-phenyl-octadecyl, ω-phenyl-eicosyl or ω-phenyl-docosyl, preferably C₇-C₁₈aralkyl such as benzyl, 2-benzyl-2-propyl, β-phenyl-ethyl, α,α-dimethylbenzyl, ω-phenyl-butyl, ω,ω-dimethyl-ω-phenyl-butyl, ω-phenyl-dodecyl or ω-phenyl-octadecyl, and particularly preferred C₇-C₁₂aralkyl such as benzyl, 2-benzyl-2-propyl, β-phenyl-ethyl, α,α-dimethylbenzyl, ω-phenyl-butyl, or ω,ω-dimethyl-ω-phenyl-butyl, in which both the aliphatic hydrocarbon group and aromatic hydrocarbon group may be unsubstituted or substituted. Preferred examples are benzyl, 2-phenylethyl, 3-phenylpropyl, naphthylethyl, naphthylmethyl, and cumyl.

C₅-C₁₂cycloalkyl, which optionally can be substituted, is for example cyclopentyl, cyclohexyl, cycloheptyl, cyclooctyl, cyclononyl, cyclodecyl, cycloundecyl, cyclododecyl, preferably cyclopentyl, cyclohexyl, cycloheptyl, or cyclooctyl, which may be unsubstituted or substituted.

Examples of the alkylene group (i.e. alkane-diyl group) having 1 to 25 carbon atoms represented include methylene group, ethylene group, n-propylene group, isopropylene group, n-butylene group, s-butylene group, isobutylene group, t-butylene group, n-pentylene group, n-hexylene group, n-heptylene group, n-octylene group, n-nonylene group, n-decylene group, n-undecylene group, n-dodecylene group, n-tridecylene group, n-tetradecylene group, n-pentadecylene group, n-hexadecylene group, n-heptadecylene group, n-octadecylene group, neopentylene group, 1-methylpentylene group, with methylene group, ethylene group, n-propylene group, isopropylene group, n-butylene group, s-butylene group, isobutylene group, t-butylene group being preferred. The alkylene group is substituted or unsubstituted.

Examples of the cycloalkylene group (i.e. cycloalkane-diyl group) having 5 to 12 carbon atoms include cyclopropylene group, cyclobutylene group, cyclopentylene group, cyclohexylene group, cyclooctylene group, and adamantylene group, with cyclopentylene group, and cyclohexylene group being preferred. The cycloalkylene group is substituted or unsubstituted.

The arylene group is preferably a divalent aromatic hydrocarbon group having 6 to 60 ring carbon atoms may be a non-condensed divalent aromatic hydrocarbon group or a condensed divalent aromatic hydrocarbon group. Specific examples thereof include phenylene group, naphthylene group, phenanthrylene group, biphenyl-diyl group, terphenyl-diyl group, quaterphenyl-diyl group, fluoranthen-diyl group, triphenylenylene-diyl group, phenanthrene-diyl group, fluorene-diyl group, spirofluorene-diyl group, 9,9-diphenylfluorene-diyl group, 9,9'-spirobi[9H-fluorene]-2-diyl group, 9,9-dimethylfluorene-diyl group, benzo[c]phenanthrene-diyl group, benzo[a]triphenylene-diyl group, naphtho[1,2-c]phenanthrene-diyl group, naphtho[1,2-a]triphenylenylene-diyl group, dibenzo[a,c]triphenylenylene-diyl group, and benzo[b]fluoranthene-diyl group, with phenylene group, naphthylene group, biphenyl-diyl group, terphenyl-diyl group, phenanthryl-diyl group, triphenylenylen-diyl group, fluorene-diyl group, spirobifluorene-diyl group, and fluoranthene-diyl group being preferred, and 1,2-phenylene group, 1,3-phenylene group, 1,4-phenylene group, 1,4-naphthylene group, 1,8-naphthylene group, 2,6-naphthylene group, 2,7-naphthylene group, biphenyl-2,2'-diyl group, biphenyl-2,3'-diyl group, biphenyl-2,4'-diyl group, biphenyl-2,5'-diyl group, biphenyl-2,6'-diyl group, biphenyl-3,3'-diyl group, biphenyl-3,4'-diyl group, biphenyl-3,5'-diyl group, biphenyl-3,6'-diyl group, biphenyl-4,4'-diyl group, biphenyl-4,5'-diyl group, biphenyl-4,6'-diyl group, biphenyl-5,5'-diyl group, biphenyl-5,6'-diyl group, biphenyl-6,6'-diyl group, phenanthrene-9,10-diyl group, phenanthrene-2,3-diyl group, phenanthrene-2,7-diyl group, phenanthrene-2,8-diyl group, phenanthrene-2,6-diyl group, phenanthrene-2,9-diyl group, phenanthrene-2,10-diyl group, phenanthrene-3,9-diyl group, phenanthrene-3,10-diyl group, triphenylene-2,3-diyl group, triphenylene-2,5-diyl group, triphenylene-2,6-diyl group, triphenylene-2,7-diyl group, triphenylene-2,8-diyl group, 9,9-dimethylfluorene-2,7-diyl group, 9,9-dimethylfluorene-3,7-diyl group, 9,9-dimethylfluorene-1,4-diyl group, fluoranthene-3,9-diyl group, fluoranthene-3,8-diyl group, fluoranthene-3,4-diyl group, fluoranthene-3,5-diyl group, fluoranthene-3,6-diyl group, fluoranthene-2,9-diyl group, fluoranthene-2,8-diyl group, fluoranthene-2,4-diyl group, fluoranthene-2,5-diyl group, fluoranthene-2,6-diyl group, fluoranthene-1,9-diyl group, fluoranthene-1,8-diyl group, fluoranthene-1,4-diyl group, fluoranthene-1,5-diyl group, fluoranthene-1,6-diyl group being more preferred. The arylene group is substituted or unsubstituted.

The heteroarylene group is preferably a divalent heterocyclic group having 5 to 30 ring atoms may be a non-condensed heterocyclic group or a condensed heterocyclic group. Specific examples thereof include the divalent residues of pyrrole ring, isoindole ring, benzofuran ring, iso-benzofuran ring, dibenzothiophene ring, isoquinoline ring, quinoxaline ring, phenanthridine ring, phenanthroline ring, pyridine ring, pyrazine ring, pyrimidine ring, pyridazine ring, triazine ring, indole ring, quinoline ring, acridine ring, pyrrolidine ring, dioxane ring, piperidine ring, morpholine ring, piperazine ring, carbazole ring, furan ring, thiophene ring, oxazole ring, oxadiazole ring, benzoxazole ring, thiazole ring, thiadiazole ring, benzothiazole ring, triazole ring, imidazole ring, benzimidazole ring, pyran ring, dibenzofuran ring, and benzo[c]dibenzofuran ring, and the divalent residues of derivatives of these rings, with the divalent residues of dibenzofuran ring, carbazole ring, dibenzothiophene ring, and derivatives of these divalent rings being preferred, and the dibenzofuran-diyl group, 9-phenylcarbazole-diyl group and dibenzothiophene-diyl group being more preferred. The heteroarylene group is substituted or unsubstituted.

The abovementioned groups are substituted or unsubstituted. Possible preferred optional substituents of the above-mentioned groups are C₁-C₈alkyl, a hydroxyl group, a mercapto group, C₁-C₈alkoxy, C₁-C₈alkylthio, halogen, halo-C₁-C₈alkyl, C₆-C₂₄aryl, C₂-C₃₀heteroaryl, or a cyano group.

The optional substituents mentioned above may be further substituted by one or more of the optional substituents mentioned above.

The number of the optional substituents depends on the group which is substituted by said substituent(s). Preferred are 1, 2, 3 or 4 optional substituents, more preferred are 1, 2 or 3 optional substituents, most preferred are 1 or 2 optional substituents. In a further preferred embodiment, the groups mentioned above are unsubstituted.

The "carbon number of a to b" in the expression of "X group having a to b carbon atoms" is the carbon number of the unsubstituted X group and does not include the carbon atom(s) of an optional substituent.

The hydrogen atom referred to herein includes isotopes different from neutron numbers, i.e., light hydrogen (protium), heavy hydrogen (deuterium) and tritium. Therefore, one or more or all of the hydrogen atoms in the residues mentioned above and below may be replaced by protium, deuterium or tritium, preferably by deuterium.

### The compound of formula (I)

The present invention relates to a composition comprising at least one compound of general formula (I) 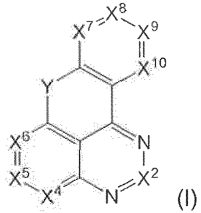 wherein X², X⁴, X⁵ X⁶, X⁷, X⁸, X⁹, X¹⁰ and Y have the meanings as mentioned above. Preferred embodiments are explained in the following.

According to one embodiment of the present invention Y in general formula (I) is O.

According to another embodiment of the present invention Y in general formula (I) is S.

Therefore the present invention preferably relates to compounds according to the following general formulae (Ia) and (Ib) 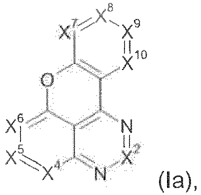 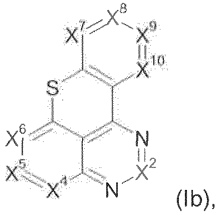 wherein independently of each other X², X⁴, X⁵, X⁶, X⁷, X⁸, X⁹, X¹⁰ and R¹¹ have the same meanings as mentioned above.

More preferably, 0, 1 or 2 of X⁴, X⁵, X⁶, X⁷, X⁸, X⁹, X¹⁰ in the compounds of formula (I) are N, most preferably, 0 of X⁴, X⁵, X⁶, X⁷, X⁸, X⁹, X¹⁰ are N, i.e. X⁴, X⁵, X⁶, X⁷, X⁸, X⁹, X¹⁰ are CR⁴, CR⁵, CR⁶, CR⁷, CR⁸, CR⁹ and CR¹⁰.

More preferred compounds of formula (I) are therefore compounds of the following general formulae (Ia1) and (Ib1): 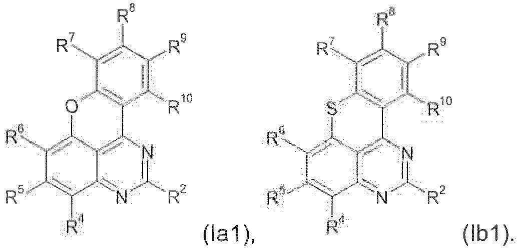
R², R⁴, R⁵, R⁶, R⁷, R⁸, R⁹, and R¹⁰ are independently of each other selected from H, deuterium, E, a C₆-C₂₄aryl group which is unsubstituted or substituted by at least one group E, C₂-C₂₄heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R⁴, R⁵, R⁶, R⁷, R⁸, R⁹ or R¹⁰, if present at adjacent carbon atoms, together form at least one C₆-C₁₈aryl or C₂-C₁₈heteroaryl ring or ring system,wherein one of R², R⁴, R⁵, R⁶, R⁷, R⁸, R⁹, R¹⁰, preferably R², in the compound of formula (I) is a N-heteroaryl group according to general formulae (XIII), (XV) or (XXI) as described below.
   D is independently of each other -CO-, -COO-, -S-, -SO-, -SO₂-, -O-,-CR¹⁵=CR¹⁶⁻, -NR¹⁷-,-SiR²²R²³⁻, -POR²⁵-, -C≡C-, preferably -O-, -NR¹⁷-, -SiR²²R²³-,
E is independently of each other -OR²¹, -SR²¹, -NR¹⁷R¹⁸, -COR²⁰, -COOR¹⁹, -CONR¹⁷R¹⁸, -CN,-SiR²²R²³R²⁴, POR²⁵R²⁷, halogen, a C₆-C₆₀aryl group which is unsubstituted or is substituted by at least one -F, -CF₃, -CF₂CF₃, -CF₂CF₂CF₃, -CF(CF₃)2, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one -F, -CF₃, -CF₂CF₃, -CF₂CF₂CF₃, -CF(CF₃)₂, -(CF₂)₃CF₃, -C(CF₃)₃, a C₁-C₁₈ alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, preferably E is independently of each other selected from -NR¹⁷R¹⁸, -CN, -SiR²²R²³R²⁴, -POR²⁵R²⁶, a C₁-C₁₈ alkyl group, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one C₁-C₁₈alkyl group, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group.

R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O, preferably H.

R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H or a C₁-C₁₈ alkyl group.

R¹⁷ and R¹⁸ together form a five or six membered aliphatic, aromatic or heteroaromatic ring, preferably five or six membered aliphatic ring.

R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O, preferably H, a C₆-C₁₈aryl group which is unsubstituted or a C₁-C₁₈alkyl group.

R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group.

R²², R²³ and R²⁴ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group.

R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E, preferably H, a C₆-C₁₈aryl group which is unsubstituted or a C₁-C₁₈alkyl group.

Preferably, R², R⁴, R⁵, R⁶, R⁷, R⁸, R⁹ and R¹⁰ are independently of each other selected from H, a C₆-C₂₄aryl group which is unsubstituted or substituted by at least one group E, C₂-C₂₄heteroaryl group which is unsubstituted or substituted by at least one group E, a C₁-C₂₅alkyl group, which is unsubstituted or substituted by at least one group E and/or interrupted by at least one group D a C₅-C₁₂cycloalkyl group which is unsubstituted or substituted by at least one group E, wherein one of R², R⁴, R⁵, R⁶, R⁷, R⁸, R⁹, R¹⁰, preferably R², in the compound of formula (I) is a N-heteroaryl group according to general formulae (XIII), (XV) or (XXI) as described below.

More preferably, R², R⁴, R⁵, R⁶, R⁷, R⁸, R⁹ and R¹⁰ are independently of each other selected from H, a C₆-C₂₄aryl group which is unsubstituted or substituted by at least one group E or C₂-C₂₄heteroaryl group which is unsubstituted or substituted by at least one group E,
wherein one of R², R⁴, R⁵, R⁶, R⁷, R⁸, R⁹, R¹⁰, preferably R², in the compound of formula (I) is a N-heteroaryl group according to general formulae (XIII), (XV) or (XXI) as described below.

Most preferably, one of (R², R⁴, R⁵, R⁶, R⁷, R⁸, R⁹, R¹⁰, preferably R², in the compound of formula (I) is a N-heteroaryl group according to general formulae (XIII), (XV) or (XXI) as described below, and the remaining of R², R⁴, R⁵, R⁶, R⁷, R⁸, R⁹ and R¹⁰ are H.

Further most preferably, R² in the compound of formula (I) is a N-heteroaryl group according to general formulae (XIII), (XV) or (XXI) as described below, and R⁴, R⁵, R⁶, R⁷, R⁸, R⁹ and R¹⁰ are H.

Most preferred are therefore compounds of formula (Ia1a) and (Ib1a): 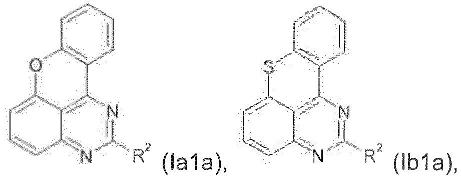 wherein R² is a N-heteroaryl group according to general formulae (XIII), (XV) or (XXI) as described below.

According to a further embodiment of the present invention in the compound according to general formula (I) at least two of R⁴, R⁵, R⁶, R⁷, R⁸, R⁹ or R¹⁰, if present at adjacent carbon atoms, together form at least one C₆-C₁₈aryl or C₁-C₁₈heteroaryl ring or ring system, preferably at least one C₆-C₁₈aryl or C₂-C₁₈heteroaryl ring or ring system.

According to this embodiment, two of R⁴, R⁵, R⁶, R⁷, R⁸, R⁹ or R¹⁰, if present at adjacent carbon atoms, together form one C₆-C₁₈aryl or C₁-C₁₈heteroaryl ring or ring system, preferably at least one C₆-C₁₈aryl or C₂-C₁₈heteroaryl ring or ring system. At the same time further two of R⁴, R⁵, R⁶, R⁷, R⁸, R⁹ or R¹⁰, if present at adjacent carbon atoms, may together form at least one further C₆-C₁₈aryl or C₁-C₁₈heteroaryl ring or ring system, preferably at least one C₆-C₁₈aryl or C₂-C₁₈heteroaryl ring or ring system. According to the present invention two of R⁴, R⁵, R⁶, R⁷, R⁸, R⁹ or R¹⁰ in each case, if present at adjacent carbon atoms, can together form at least one, two, three or four C₆-C₁₈aryl or C₁-C₁₈heteroaryl rings or ring systems, preferably at least one, two, three or four C₆-C₁₈aryl or C₂-C₁₈heteroaryl ring or ring system.

Preferred C₆-C₁₈aryl or C₁-C₁₈heteroaryl rings or ring systems, preferably C₆-C₁₈aryl or C₂-C₁₈heteroaryl ring or ring system are for example fused phenylene or naphthylene rings, five or six membered fused C₁-C₁₈heteroarylene rings or ringsystems, preferably fused phenylene or naphthylene rings, five or six membered fused C₂-C₁₈heteroarylene rings or ringsystems.

In the compounds of formula (I), one of R², R⁴, R⁵, R⁶, R⁷, R⁸, R⁹, R¹⁰, preferably R², in the compound of formula (I) is a N-heteroaryl group according to general formulae (XIII), (XV) or (XXI). The N-hereoaryl groups according to general formulae (XIII), (XV) and (XXI) are described in the following.

### N-hereoaryl groups according to general formula (XXI)

In one embodiment of the present invention, the N-hereoaryl group is represented by the general formula (XXI) 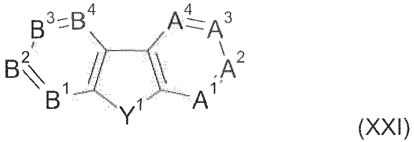 wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is NR⁷⁰, CR⁷¹R⁷², O or S
R⁶², R⁶³, R⁶⁴, R⁶⁵, R⁶⁶, R⁶⁷, R⁶⁸ and R⁶⁹ are independently of each other selected from H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
and/or
at least two of R⁶², R⁶³, R⁶⁴ and R⁶⁵, if present are directly bonded to the moiety represented by the general formula (XXII) by the two *-locations. 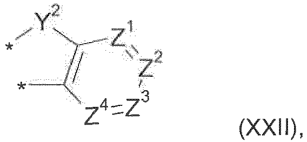
wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and/or
R⁶², R⁶³, R⁶⁴ or R⁶⁵, if present at adjacent carbon atoms, together form at least one C₆-C₁₈aryl or C₂-C₁₈heteroaryl ring or ring system,
and/or
at least two of R⁶⁶, R⁶⁷, R⁶⁸ and R⁶⁹, if present are directly bonded to the moiety represented by the general formula (XXIII) by the two *-locations. 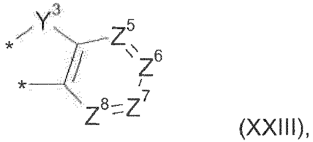
wherein
Y³ is NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and/or
at least two of R⁶⁶, R⁶⁷, R⁶⁸ or R⁶⁹, if present at adjacent carbon atoms, together form at least one C₆-C₁₈aryl or C₂-C₁₈heteroaryl ring or ring system,
R⁷⁰, R⁷³ and R⁸⁰ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
R⁷¹, R⁷², R⁷⁴, R⁷⁵, R⁸¹ and R⁸² are, independently of each other selected from H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached,
R⁷⁶, R⁷⁷, R⁷⁸, R⁷⁹, R⁸³, R⁸⁴, R⁸⁵ and R⁸⁶ are independently of each other selected from H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
and/or
at least two of R⁷⁶, R⁷⁷, R⁷⁸, R⁷⁹, R⁸³, R⁸⁴, R⁸⁵ or R⁸⁶, if present at adjacent carbon atoms, together form at least one C₆-C₁₈aryl or C₂-C₁₈heteroaryl ring or ring system,
wherein the substituent according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, preferably R⁷⁰, R⁷³ or R⁸⁰, if present, wherein this respective R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, preferably R⁷⁰, R⁷³ or R⁸⁰, is a direct bond, optionally interrupted by a group of formula -(M)ₘ-, in this case,
m is an integer of 0 to 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
wherein D and E have the same meanings as mentioned above.

According to the present invention the heterocyclic group according to general formula (XXI), which may be for example a dibenzofuran, dibenzothiophene or benzofurodibenzofuran group, can be attached to the compound of general formula (I) via any atom present in the heterocyclic ring system. Therefore, any of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, if present, can be a direct bond, optionally interrupted by a group of formula -(M)ₘ-, with which the heterocyclic group according to formula (XXI) can be attached to the compound of general formula (I).

Preferably, the heterocyclic group according to general formula (XXI) is represented by any one of general formula (XXIa), (XXIb) or (XXIc) 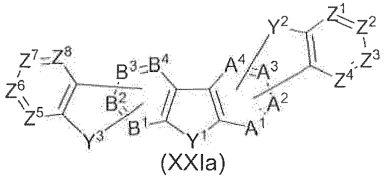 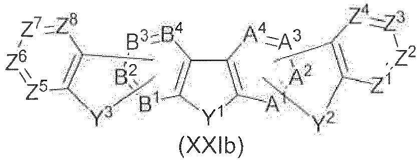 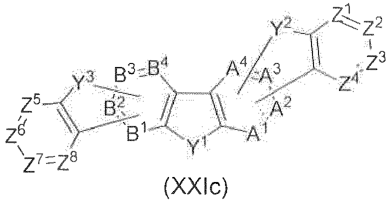 wherein A¹, A², A³, A⁴, B¹, B², B³, B⁴, Z¹, Z², Z³, Z⁴, Z⁵, Z⁶, Z⁷, Z⁸, Y¹, Y² and Y³ have the same meanings as defined above.

According to a further preferred embodiment of the present invention one of Y¹, Y² and Y³ in the compounds according general formulae (XXI), (XXIa), (XXIb) and/or (XXIc) is NR⁷⁰, NR⁷³ or NR⁸⁰ respectively.

According to a further preferred embodiment of the present invention two of Y¹, Y² and Y³ in the compound according to general formulae (XXI), (XXIa), (XXIb) and/or (XXIc) are NR⁷⁰, NR⁷³ or NR⁸⁰ respectively.

Preferably, the N-heteroaryl group according to general formula (XXI) corresponds to a N-heteroaryl group according to general formula (XXI) 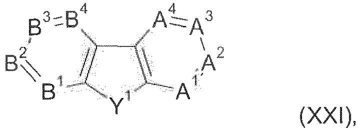
wherein A¹ is CR⁶²,
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
R⁶⁶, R⁶⁷, R⁶⁸ and R⁶⁹ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
and
at least two of R⁶², R⁶³, R⁶⁴ and R⁶⁵ are direct bond and, if present at adjacent carbon atoms, are directly connected to the two *-locations in the moiety of general formula (XXII) 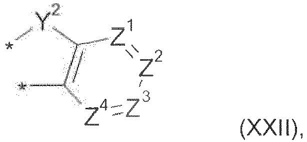
wherein
Y² is NR⁷³, CR⁷⁴R⁷⁵, O or S,
Z¹ is CR⁷⁶,
Z² is CR⁷⁷,
Z³ is CR⁷⁸,
Z⁴ is CR⁷⁹, and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or carbazolyl,
wherein R⁷⁶, R⁷⁷, R⁷⁸ and R⁷⁹ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably hydrogen,
R73, R⁷⁴ and R⁷⁵ have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰, CR⁷¹R⁷², O or S
at least two of R⁶², R⁶³, R⁶⁴ and R⁶⁵ are direct bond and, if present at adjacent carbon atoms, are directly connected to the two *-locations in the moiety of general formula (XXII) 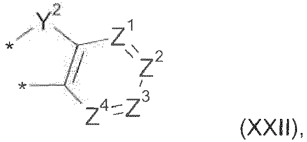 wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
and
at least two of R⁶⁶, R⁶⁷, R⁶⁸ and R⁶⁹, if present at adjacent carbon atoms, are directly connected to the two *-locations in the moiety of general formula (XXIII) 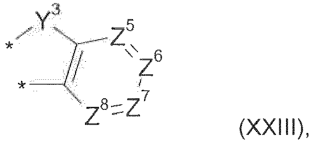
wherein
Y³ is NR⁸⁰,
Z⁵ is CR⁸³,
Z⁶ is CR⁸⁴,
Z⁷ is CR⁸⁵,
Z⁸ is CR⁸⁶,
and the remaining of R⁶⁶, R⁶⁷, R⁶⁸ and R⁶⁹ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁷⁸, R⁷⁹, R⁸³, R⁸⁴, R⁸⁵ and R⁸⁶ are independently of each other selected from direct bond, H, E, a C₆-C₄₀aryl group which is unsubstituted or substituted by at least one group E, C₁-C₃₉₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁰, R⁷¹, R⁷², R⁷³, R⁷⁴ and R⁷⁵ have the same meanings as mentioned above,
and the substituent according to general formula (XXI) is connected to the compound according to general formula (I) via R⁸⁰ being a direct bond, which is optionally interrupted by a group of formula -(M)ₘ-, wherein M and m have the same meanings as mentioned above, preferably m is 0 or 1.

In a further preferred embodiment, the N-heteroaryl group according to general formula (XXI) corresponds to a N-heteroaryl group according to general formula (XXI), wherein
A² is CR⁶³ or N preferably CR⁶³,
A³ is CR⁶⁴ or N, preferably CR⁶⁴,
A⁴ is CR⁶⁵ or N, preferably CR⁶⁵,
B¹ is CR⁶⁶ or N, preferably CR⁶⁶,
B² is CR⁶⁷ or N, preferably CR⁶⁷,
B³ is CR⁶⁸ or N, preferably CR⁶⁸,
B⁴ is CR⁶⁹ or N, preferably CR⁶⁹,
Y¹ is NR⁷⁰,
at least two of R⁶², R⁶³, R⁶⁴ and R⁶⁵ are direct bond and, if present at adjacent carbon atoms, are directly connected to the two *-locations in the moiety of general formula (XXII) 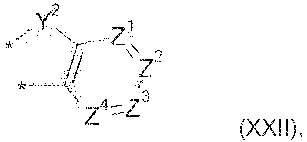 wherein
Y² is NR⁷³, CR⁷⁴R⁷⁵, O or S,
Z¹ is CR⁷⁶,
Z² is CR⁷⁷,
Z³ is CR⁷⁸
Z⁴ is CR⁷⁹,
and the remaining of R⁶², R⁶³, R⁶⁴ and R⁶⁵ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
and
at least two of R⁶⁶, R⁶⁷, R⁶⁸ and R⁶⁹, if present at adjacent carbon atoms, are directly connected to the two *-locations in the moiety of general formula (XXIII) 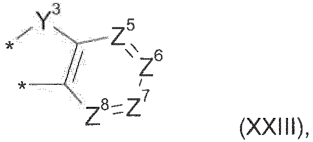
wherein
Y³ is NR⁸⁰, CR⁸¹R⁸², O or S,
Z⁵ is CR⁸³,
Z⁶ is CR⁸⁴,
Z⁷ is CR⁸⁵,
Z⁸ is CR⁸⁶,
and the remaining of R⁶⁶, R⁶⁷, R⁶⁸ and R⁶⁹ that are not direct bond are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷⁶, R⁷⁷, R⁷⁸, R⁷⁹, R⁸³, R⁸⁴, R⁸⁵ and R⁸⁶ are independently of each other selected from direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H or phenyl,
R⁷³, R⁷⁴, R⁷⁵, R⁸⁰, R⁸¹ and R⁸² have the same meanings as mentioned above,
and the substituent according to general formula (XXI) is connected to the compound according to general formula (I) via R⁷⁰ being a direct bond, which is optionally interrupted by a group of formula -(M)ₘ-, wherein M and m have the same meanings as mentioned above, preferably m is 0 or 1.

### N-heteroaryl groups according to general formula (XIII)

In one embodiment of the present invention, the N-heteroaryl group is represented by the general formula (XIII) 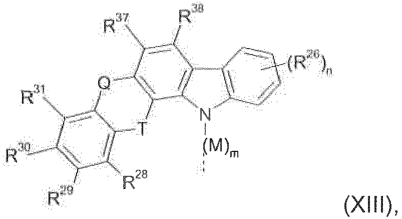
wherein M, m and R²⁶ have the meanings as mentioned above, preferably m is 0;
n is 0, 1, 2, 3 or 4, preferably n is 0;
R²⁸, R²⁹, R³⁰, R³¹, R³⁷ and R³⁸ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl, biphenyl, more preferably H;
or
at least two of R²⁸, R²⁹, R³⁰, R³¹, R³⁷ or R³⁸, if present at adjacent carbon atoms, together form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, preferably NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, or spiro group, wherein R³⁴ and R³⁵ together form a five or six membered, substituted or unsubstituted, aliphatic ring, preferably H, methyl, ethyl, phenyl, or spiro group, wherein R³⁴ and R³⁵ together form a five or six membered, substituted or unsubstituted, aliphatic ring,
R³⁶ is a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, a C₆-C₁₈aryl group which is unsubstituted or a C₁-C₁₈alkyl group, more preferably a phenyl group;
wherein D and E have the same meanings as mentioned above.

Preferred compounds of formula (XIII) are compounds of the following formula (XIIIa), wherein T is a direct bond, and compounds of formula (Xlllb), wherein Q is a direct bond: 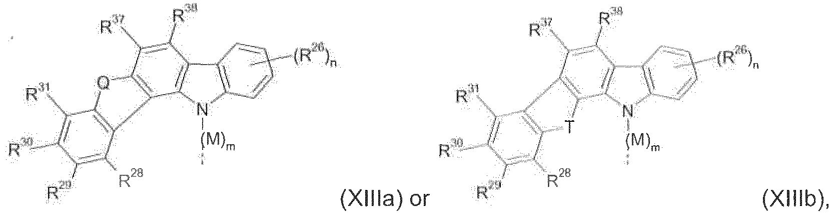 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring, which is preferably present in position of R³⁰ and R³¹.

More preferred compounds of formula (XIII) are the following compounds: 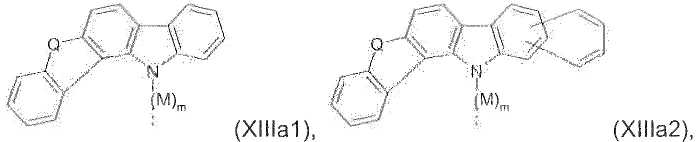 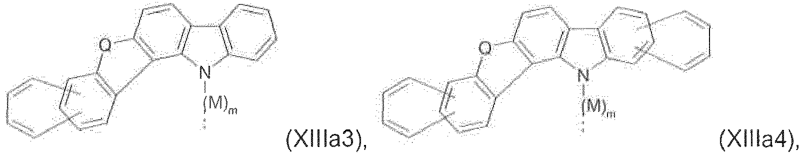 preferably 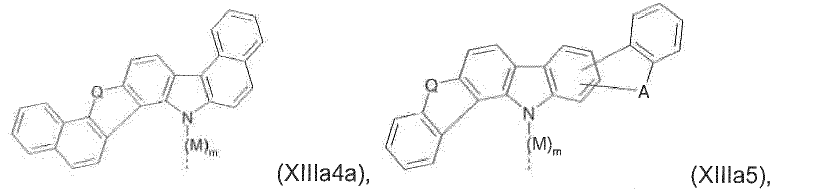 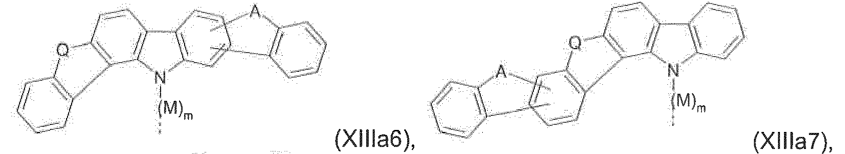 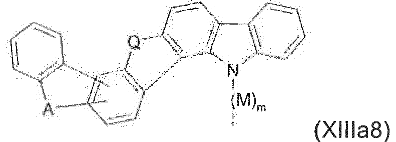 or 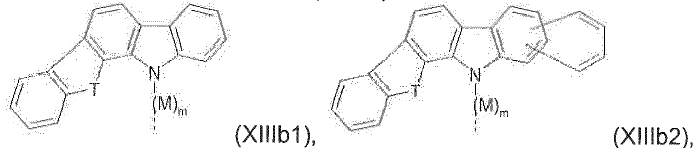 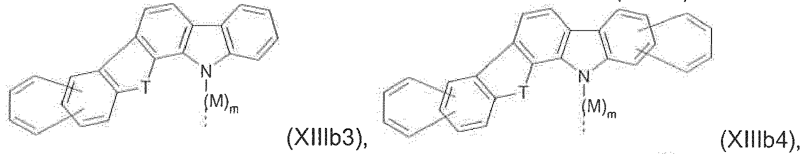 preferably 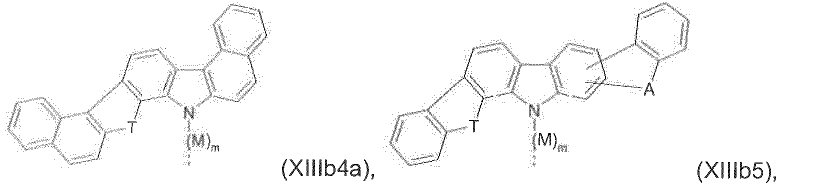 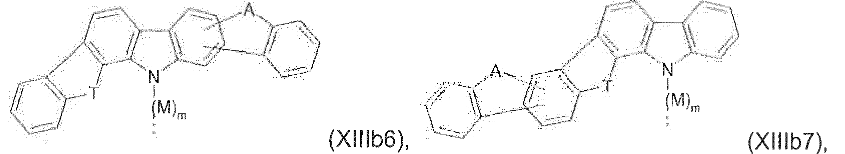 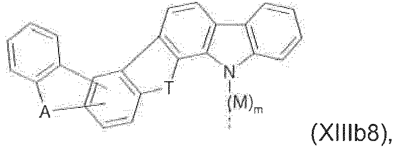 wherein
A is S, O, SiR³²R³³, CR³⁴R³⁵ or NR³⁶, preferably, and
Q and T are independently of each other S, O, SiR³²R³³, CR³⁴R³⁵ or NR³⁶, preferably NR³⁶.

According to a particularly preferred embodiment of the present invention, in the compound of formula (XIII), Q is direct bond, T is S, R³⁹, R⁴⁰, R⁴¹ and R⁴² are H, m is 0 or 1, n is 0 and R²⁸, R²⁹, R³⁰ and R³¹ are H.

According to a further particularly preferred embodiment of the present invention, in the compound of formula (XIII), Q is direct bond, T is NR³⁶, R³⁶ is phenyl, R³⁹, R⁴⁰, R⁴¹ and R⁴² are H, m is 0, n is 0 and R²⁸, R²⁹, R³⁰ and R³¹ are H.

According to a particularly preferred embodiment of the present invention, in the compound of formula (XIII), Q is direct bond, T is CR³⁴R³⁵, R³⁴ and R³⁵ are methyl, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is S, T is direct bond, R³⁹, R⁴⁰, R⁴¹ and R⁴² are H, m is 0, n is 0 and R²⁸, R²⁹, R³⁰ and R³¹ are H.

According to a further particularly preferred embodiment of the present invention, in the compound of formula (XIII), Q is NR³⁶, T is direct bond, R³⁶ is phenyl, R³⁹, R⁴⁰, R⁴¹ and R⁴² are H, m is 0, n is 0 and R²⁸, R²⁹, R³⁰ and R³¹ are H.

Most preferred compounds of formula (XIII) are compounds of formula (XIII), wherein Q is a direct bond, especially preferred are therefore compounds (Xlllb), more preferably (XIIIb1) to (XIIIb12).

According to a further preferred embodiment, the heteroaryl group according to general formula (XIII) corresponds to general formula (XIV) 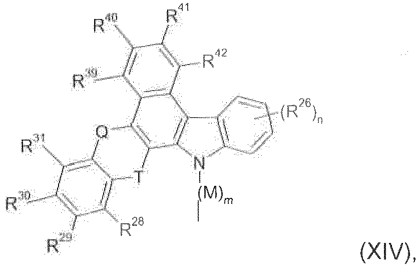
wherein R²⁶, R²⁸, R²⁹, R³⁰, R³¹, M, Q, T, n and m have the same meanings as mentioned above and
R³⁹, R⁴⁰, R⁴¹, R⁴² are independently of each other selected from H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, preferably R³⁹, R⁴⁰, R⁴¹ and R⁴² are H.

Preferred compounds of formula (XIV) are compounds of the following formula, wherein T is a direct bond, and compounds of formula (XIVb), wherein Q is a direct bond: 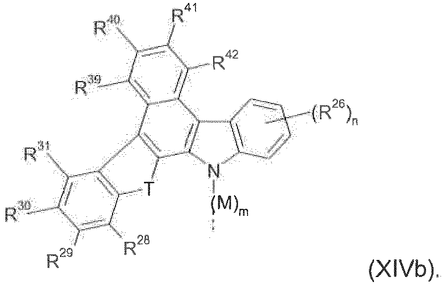 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring, which is preferably present in position of R³⁰ and R³¹ .

More preferred compounds of formula (XIV) are the following compounds : 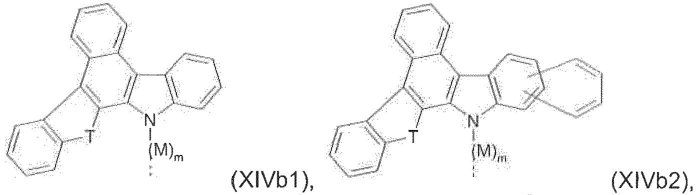 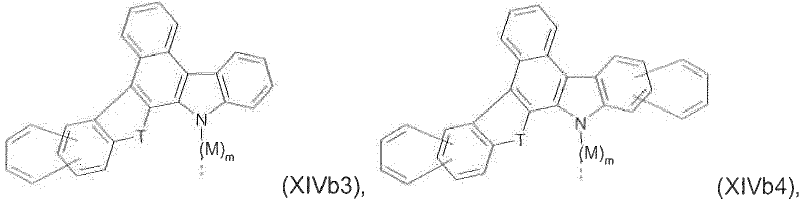 preferably 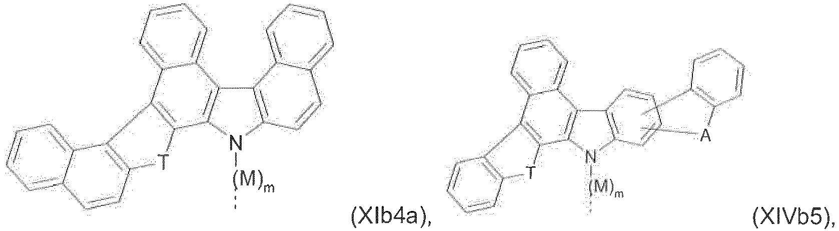 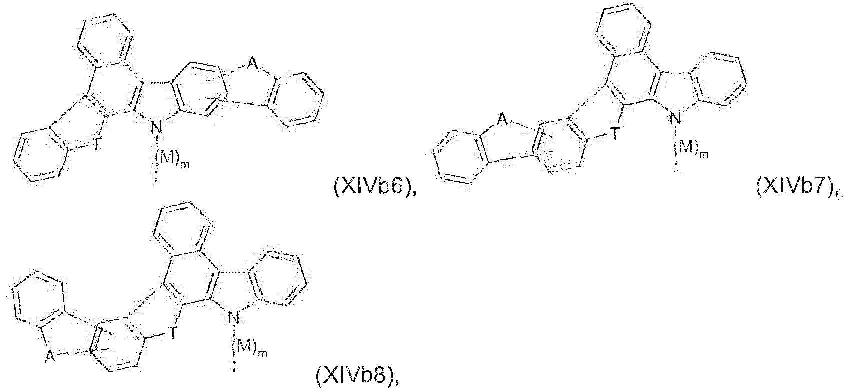
wherein A is S, O, SiR³²R³³, CR³⁴R³⁵ or NR³⁶, preferably, and
Q and T are independently of each other S, O, SiR³²R³³, CR³⁴R³⁵ or NR³⁶, preferably NR³⁶.

### N-heteroaryl groups according to general formula (XV)

In one embodiment of the present invention, the N-heteroaryl group is represented by the general formula 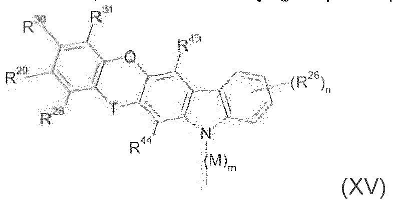
wherein n is 0, 1, 2, 3 or 4,
m is 0, 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, or at least two of R²⁸, R²⁹, R³⁰ or R³¹, if present at adjacent carbon atoms, together may form at least one C₆-C₁₈aryl or C₂-C₁₆heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E or a spiro group, or R³⁴ and R³⁵ together may form a five or six membered, substituted or unsubstituted, aliphatic ring, preferably, R³⁴ and R³⁵ are independently of each other H, ethyl, ethyl, phenyl or a spiro group, or R³⁴ and R³⁵ together may form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, for example phenyl,
wherein the dotted line is a bonding site;
wherein D and E have the same meanings as mentioned above.

Preferred compounds of formula (XV) are compounds of the following formulae (XVa), wherein T is a direct bond, and compounds of formula (XVb), wherein Q is a direct bond: 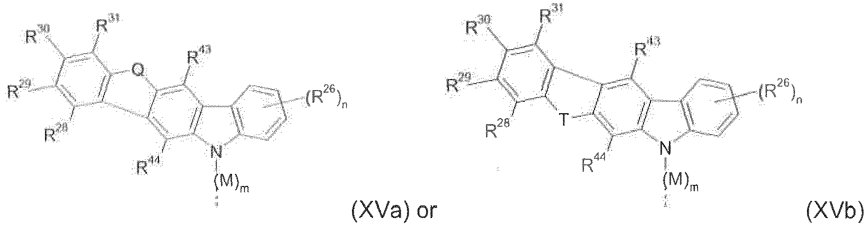 wherein residues, symbols and indices have the same meanings as mentioned above.

Preferably, R²⁸, R²⁹, R³⁰, R³¹, R³⁷ and R³⁸ are H.

R²⁶ is preferably H or two R²⁶ that are present at adjacent carbon atoms together form a fused phenyl ring, which is preferably present in position of R³⁰ and R³¹.

More preferred compounds of formula (XV) are the following compounds: 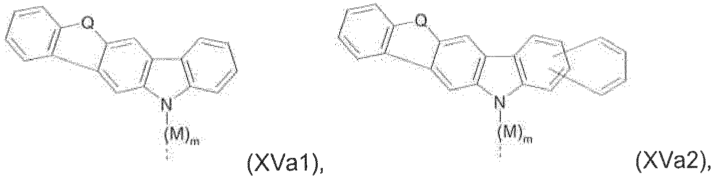 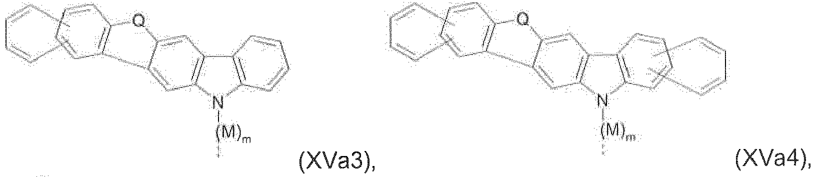 preferably 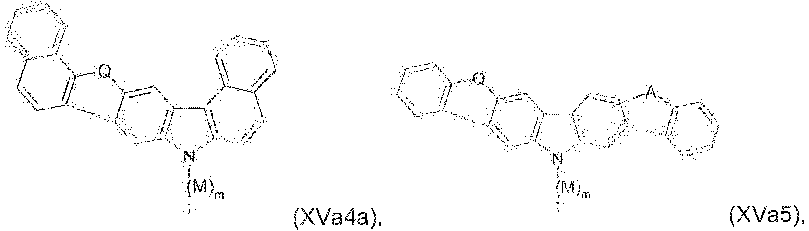 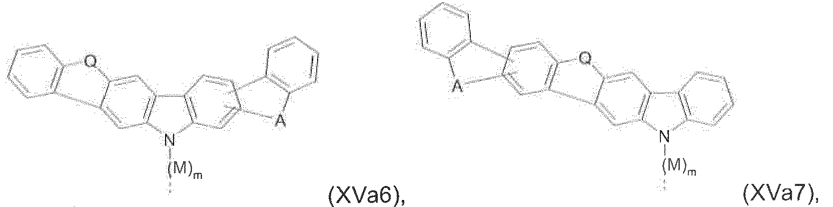 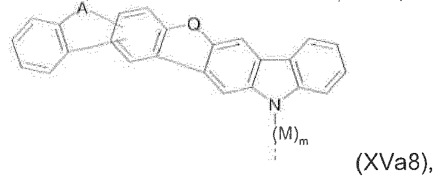 or 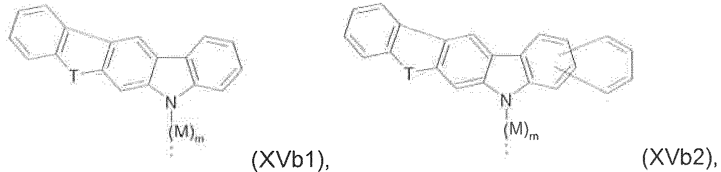 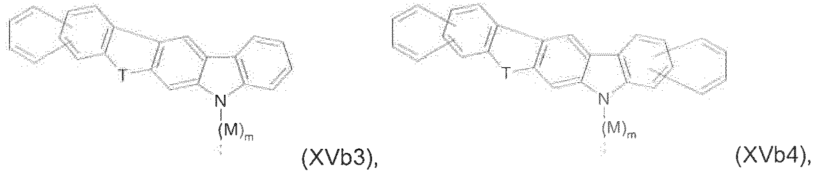 preferably 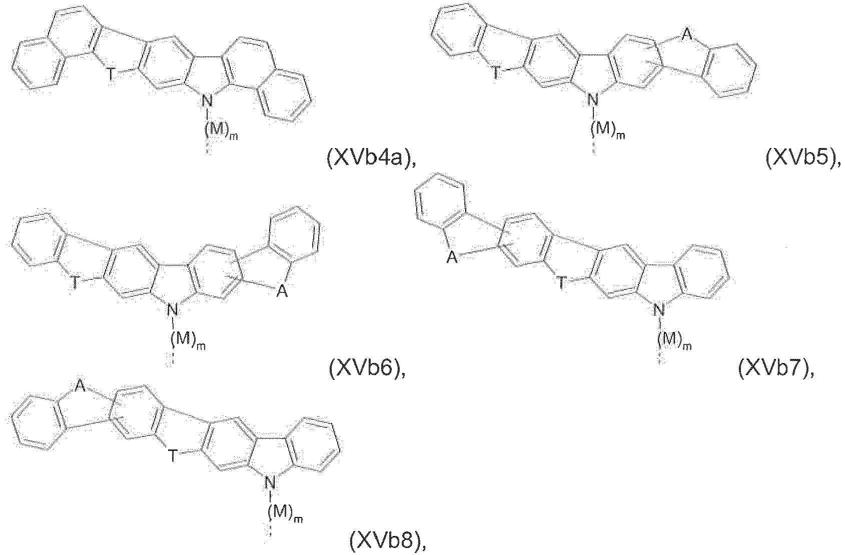
wherein A is S, O, SiR³²R³³, CR³⁴R³⁵ or NR³⁶, and
Q and T are independently of each other S, O, SiR³²R³³, CR³⁴R³⁵ or NR³⁶, preferably NR³⁶.

According to a particular preferred embodiment of the present invention in substituent according to general formula (XV), m is 0, n is 0, R²⁸, R²⁹, R³⁰, R³¹, R⁴³ and R⁴⁴ are H, Q is a direct bond, T is CR³⁴R³⁵, R³⁴ and R³⁵ are methyl.

According to a further particular preferred embodiment of the present invention in substituent according to general formula (XV), m is 0, n is 2, R²⁸, R²⁹, R³⁰, R³¹, R⁴³ and R⁴⁴ are H, two R²⁶ that are present at adjacent carbon atoms together form a fused phenyl ring, which is preferably present in position of R³⁰ and R³¹, Q is a direct bond, T is CR³⁴R³⁵, R³⁴ and R³⁵ are methyl.

According to a further preferred embodiment of the present invention, R²⁶ in the N-heteroaryl groups according to general formulae (XIII), (XIV) or (XV) may correspond to the following formula (XVI) 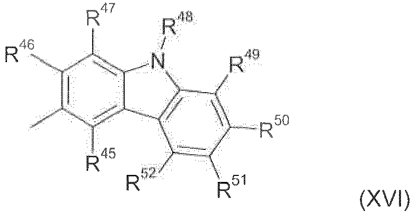
wherein R⁴⁵, R⁴⁶, R⁴⁷, R⁴⁸, R⁴⁹, R⁵⁰, R⁵¹ and R⁵² are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R⁴⁶, R⁴⁷, R⁴⁹, R⁵⁰, R⁵¹ or R⁵², if present at adjacent carbon atoms, together form at least one C₆-C₁₈aryl or C₁-C₁₈heteroaryl ring or ring system,
wherein E and D have the same meanings as mentioned above.

Particularly preferred R⁴⁵, R⁴⁶, R⁴⁷, R⁴⁹, R⁵⁰, R⁵¹ and R⁵² are independently of each other H, E, a unsubstituted C₆-C₁₈aryl group or a C₆-C₁₈aryl group substituted with at least one group E, or a C₁-C₂₅alkyl group, which is unsubstituted or substituted by at least one group E and/or interrupted by at least one group D, preferably H, phenyl, biphenyl, naphthyl, phenanthryl or dimethylfluorenyl.

R⁴⁸ is particularly preferred H, a unsubstituted C₆-C₁₈aryl group or a C₆-C₁₈aryl group substituted with at least one group E, preferably phenyl, biphenhyl or naphthyl.

Further preferred at least two of R⁴⁶, R⁴⁷, R⁴⁹, R⁵⁰, R⁵¹ or R⁵², most preferably R⁵¹ and R⁵² if present at adjacent carbon atoms, together form at least one C₆-C₁₈aryl or C₁-C₁₈heteroaryl ring or ring system, most preferably a fused phenyl ring.

According to a particularly preferred embodiment, in general formula (XVI) R⁴⁵, R⁴⁶, R⁴⁷, R⁴⁹, R⁵⁰, R⁵¹ and R⁵² are H and R⁴⁸ is phenyl.

According to a further particularly preferred embodiment, in general formula (XVI) R⁴⁵, R⁴⁶, R⁴⁷, R⁴⁹ and R⁵⁰ are H, R⁵¹ and R⁵² form a fused phenyl ring and R⁴⁸ is phenyl.

In general, m is an integer of 0 to 4, wherein m describes the number of groups M present. If m is 0, no group M is present, but the N-heteroaryl group according to general formula (XII) is directly attached to carbon atom within the skeleton of the compound of general formula (I). Preferably, m is 0, 1, 2 or 3, more preferably, m is 0 or 1.

Preferably, M is a C₆-C₄₀ arylene group which is unsubstituted, a C₁-C₂₄heteroarylene group which is unsubstituted or a C₁-C₂₅ alkylene group which unsubstituted. Particularly preferred, M is a C₆-C₁₈ arylene group which is unsubstituted, most preferably a phenylene group.

Preferably, R²⁶ is independently of each other selected from a C₆-C₄₀aryl group which is unsubstituted or substituted by at least one group E and a C₁-C₃₀heteroaryl group which is unsubstituted or substituted by at least one group E.

According to a very preferred embodiment of the present invention, m is 0, and at least two of R²⁶, if present at adjacent carbon atoms, form at least one five or six membered, substituted or unsubstituted, saturated, unsaturated, aromatic or heteroaromatic ring or ring system, without further substituents R²⁶.

According to a further very preferred embodiment of the present invention, m is 1, M is M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring or ring system, without further substituents R²⁶.

The present invention therefore preferably relates the compound according to the present invention, wherein m is 1, M is a C₆-C₄₀arylene group which is unsubstituted or substituted by at least one group E, preferably phenylene, and at least two of R²⁶, if present at adjacent carbon atoms, form a five or six membered, substituted or unsubstituted, saturated, unsaturated, aromatic or heteroaromatic ring, wherein E has the meanings as mentioned above.

According to the present invention, in compounds according to general formula (I), in particular in compounds according to general formulae (Ia) and (lb), X⁴, X⁵, X⁶, X⁷, X⁸, X⁹ and X¹⁰ can independently of each other be N, CR⁴, CR⁵, CR⁶, CR⁷, CR⁸, CR⁹ or CR¹⁰, wherein R⁴ to R¹⁰ have the meanings as mentioned above. According to a preferred embodiment of the present invention none of X⁴, X⁵, X⁶, X⁷, X⁸, X⁹ and X¹⁰ is N, i.e. X⁴, X⁵, X⁶, X⁷, X⁸, X⁹ and X¹⁰ are CR⁴, CR⁵, CR⁶, CR⁷, CR⁸, CR⁹ respectively CR¹⁰, wherein R⁴ to R¹⁰ have the meanings as mentioned above.

The present invention therefore preferably relates to compounds according to formulae (Ia1) and (Ib1) as mentioned above, wherein independently of each other R², R⁴, R⁵, R⁶, R⁷, R⁸, R⁹, R¹⁰ and R¹¹ have the same meanings as mentioned above. Most preferably, R², R⁴, R⁵, R⁶, R⁷, R⁸, R⁹, and R¹⁰ are H, wherein one of R², R⁴, R⁵, R⁶, R⁷, R⁸, R⁹, R¹⁰ in the compound of formula (I) is a N-heteroaryl group according to general formulae (XIII), (XV) or (XXI), preferably, R² in the compound of formula (I) is a N-heteroaryl group according to general formulae (XIII), (XV) or (XXI)
Particularly preferred molecules according to general formula (I) according to the present invention are shown in the following.

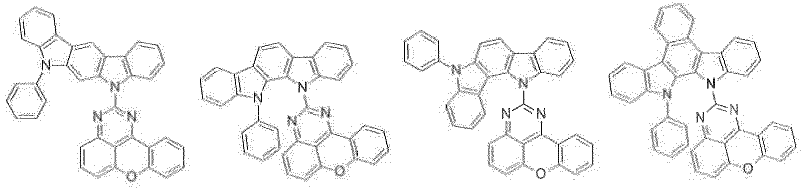

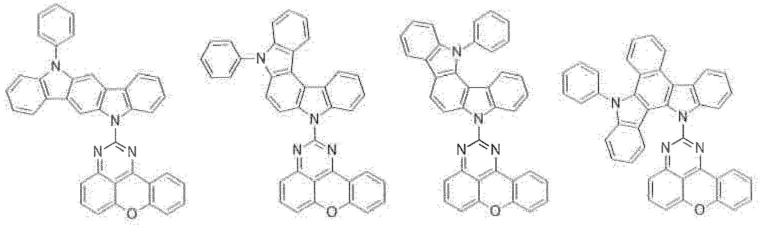

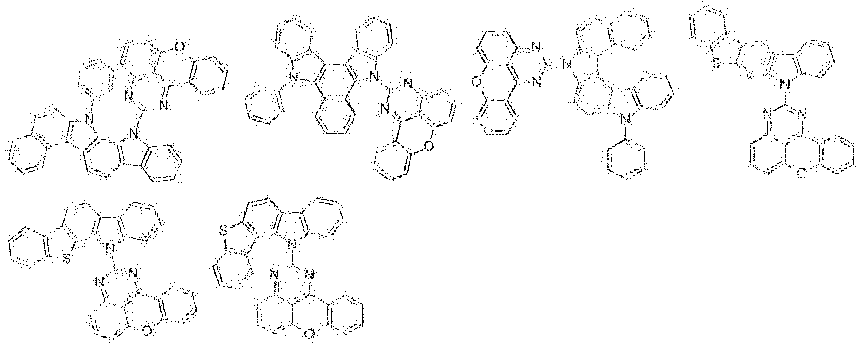

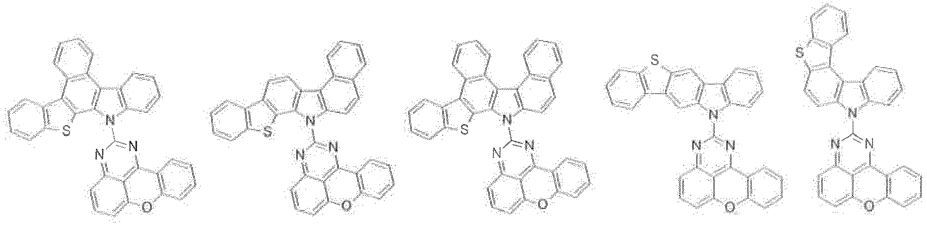

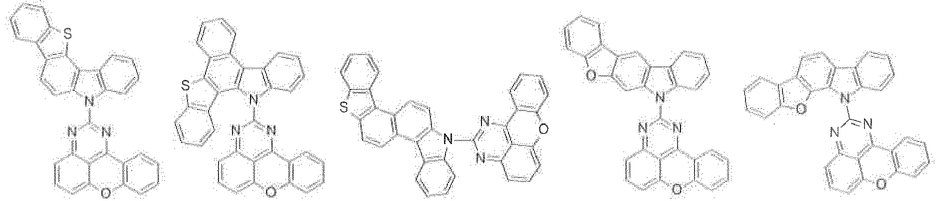

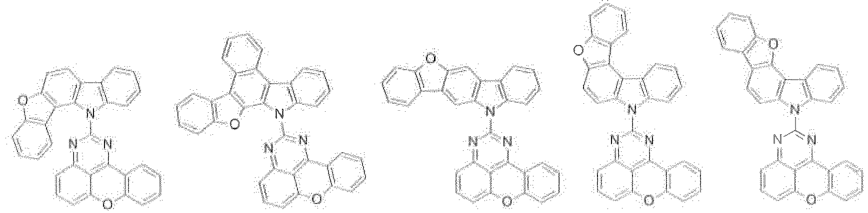

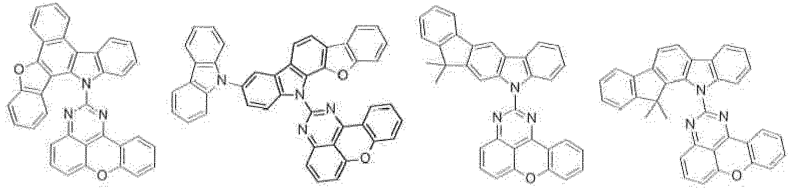

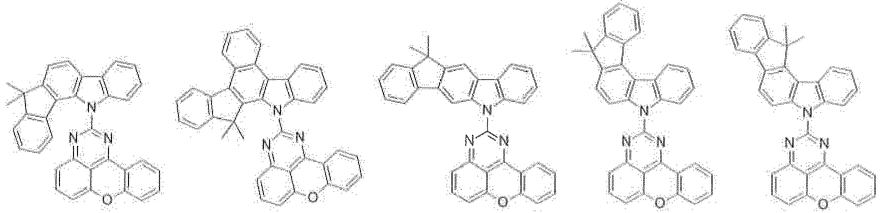

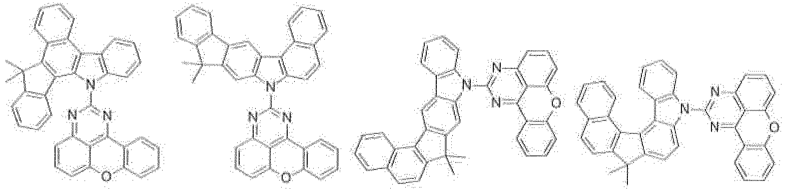

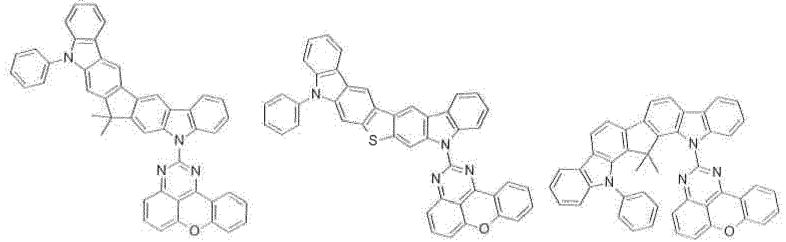

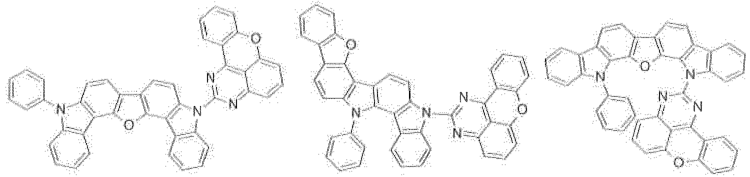

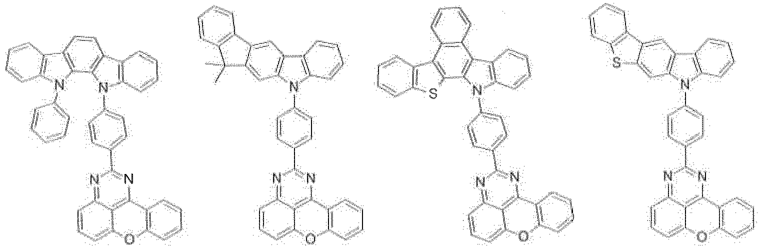

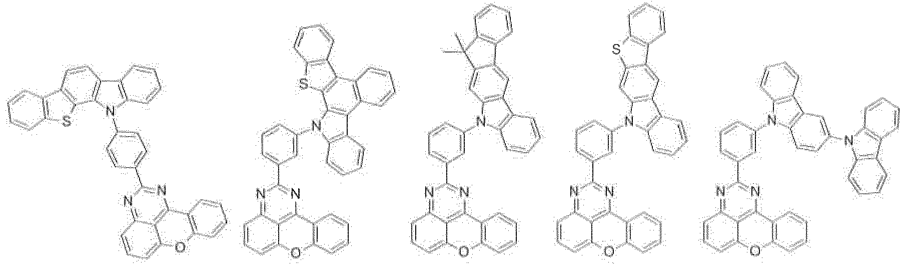

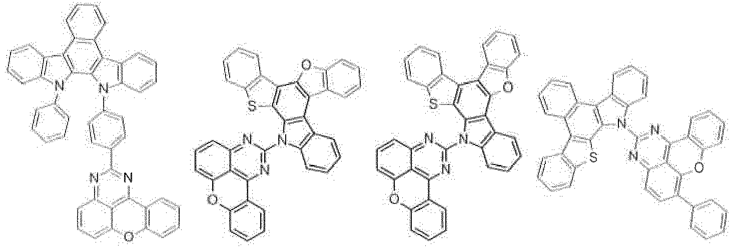

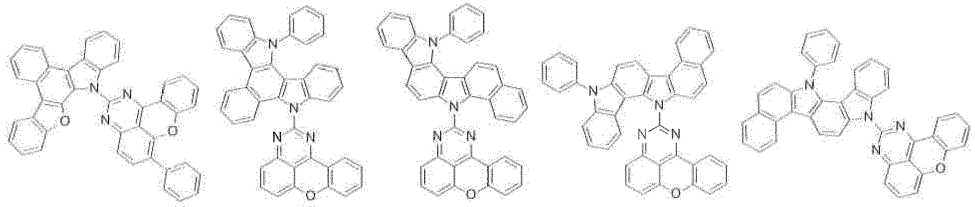

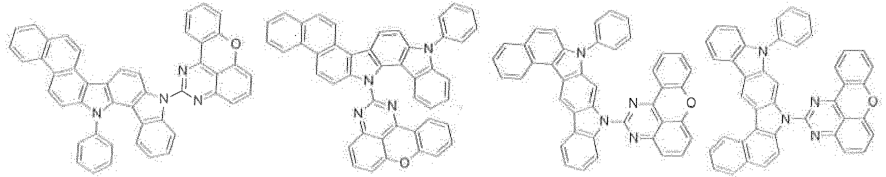

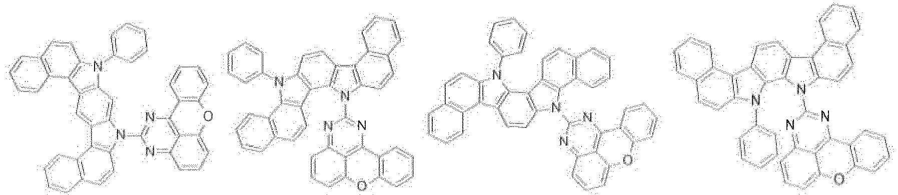

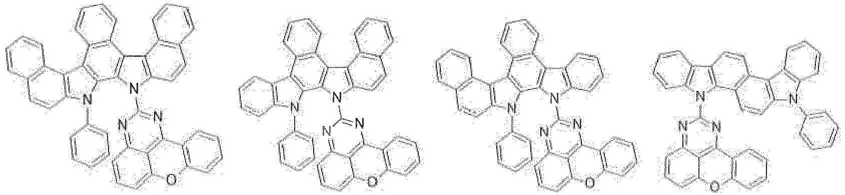

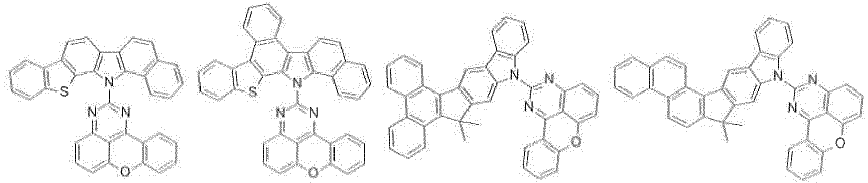

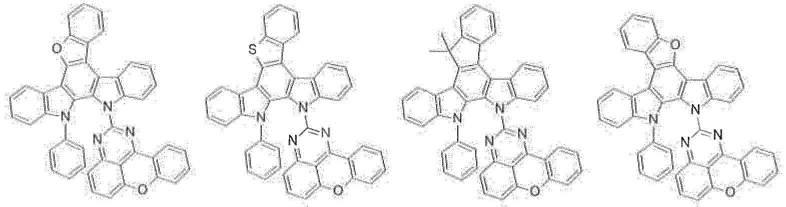

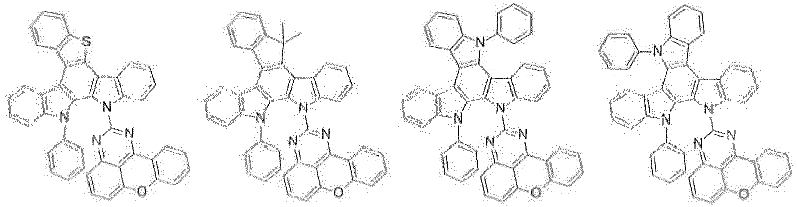

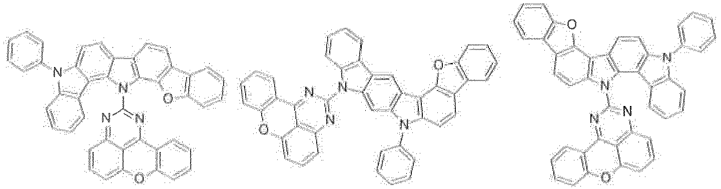

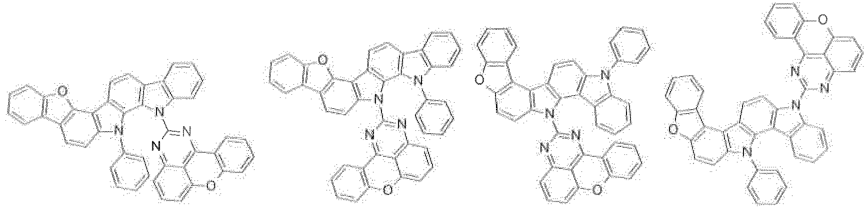

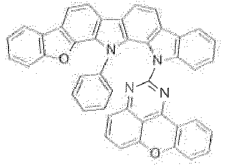

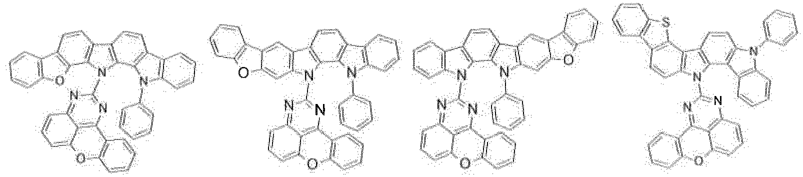

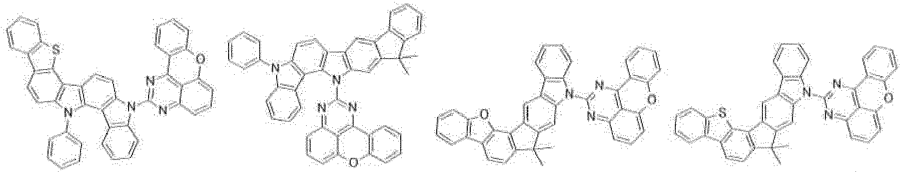

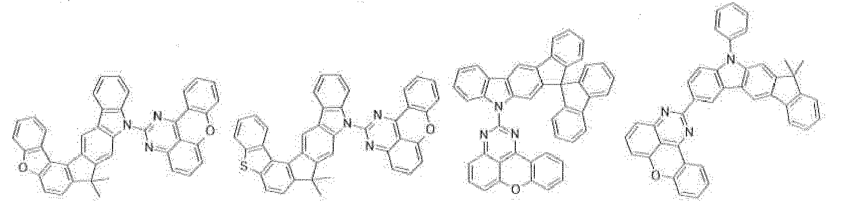

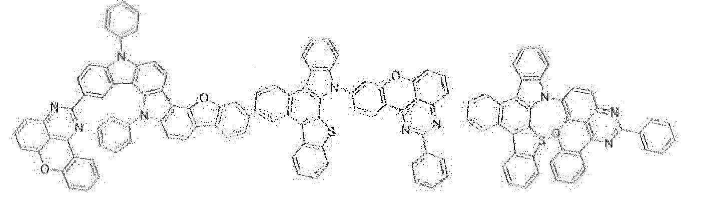

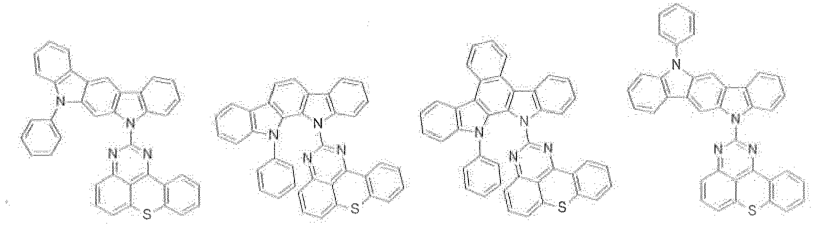

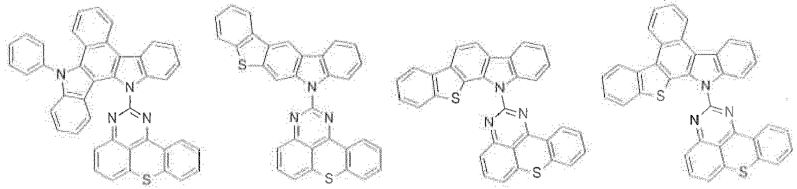

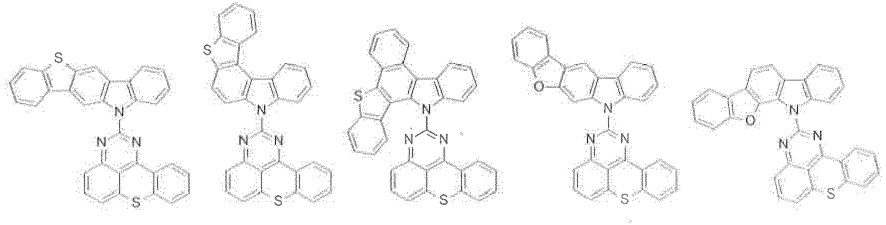

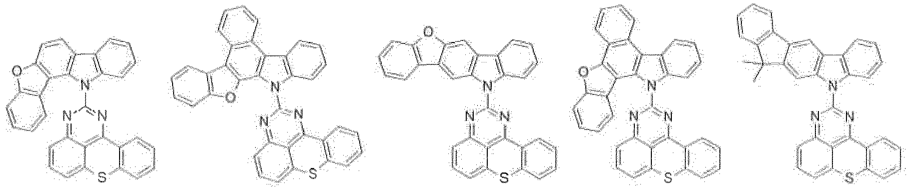

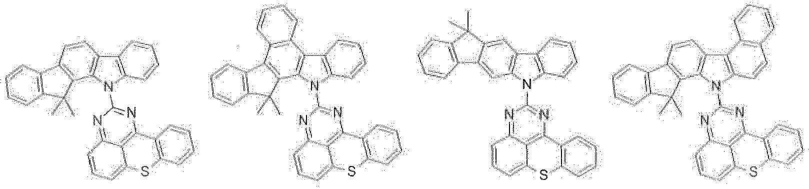

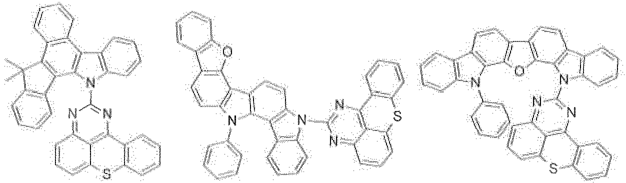

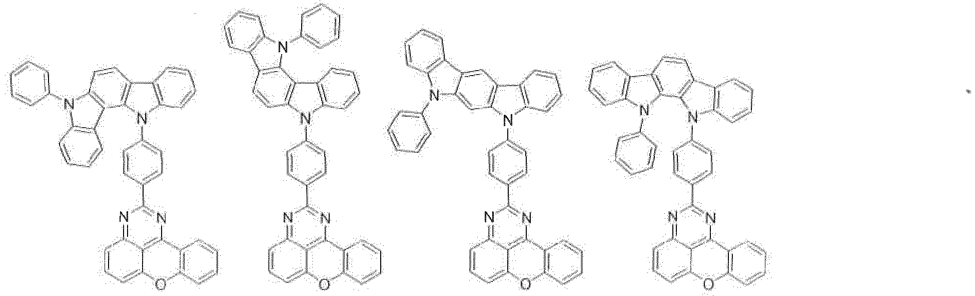

The present invention also relates to a process for the preparation of a compound according to general formula (I) as defined above, at least comprising step (A)

### (A) coupling of a compound according to general formula (V)

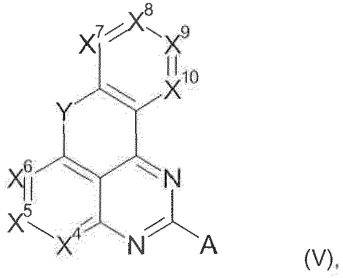

with a compound of formula R²-H to obtain a compound according to general formula (I), wherein Y, X⁴, X⁵, X⁶, X⁷, X⁸, X⁹ and X¹⁰ have the same meanings as defined above and A is a selected from Cl, Br, I, F, OSO₂CH₃, and OSO₂CF₃ or OSO₂C₆H₄CH₃.; and
R² is a N-heteroaryl group according to general formulae (XIII), (XV) or (XXI).

Step (A) of the process according to the present invention can in general be conducted by any method and under any conditions that are known to provide the desired product by a person having ordinary skill in the art. For example, step (A) of the process according to the present invention can be coupling reactions that are known to a person having ordinary skill in the art, for example reactions using palladium or copper catalysts.

The present invention therefore preferably relates to the process according to the present invention, wherein step (A) is a coupling reaction that is conducted in the presence of a palladium and/or copper catalyst.

According to one preferred embodiment, step (A) of the process according to the present invention can be conducted using the so called Buchwald-Hartwig reaction which is known to the skilled artisan and which is, for example, mentioned in Adv. Synth. Catal., 2006, 23, J. Organomet. Chem., 1999, 125, Chem. Sci., 2011, 27.

According to another preferred embodiment, step (A) of the process according to the present invention can be conducted using the so called Suzuki reaction which is known to the skilled artisan and which is, for example, mentioned in Chem. Soc. Rev., 2014, 3525, Angew. Chem. Int. Ed., 2009, 6954.

According to another preferred embodiment, step (A) of the process according to the present invention can be conducted using the so called Ulmann reaction which is known to the skilled artisan and which is, for example, mentioned in Chem. Rev., 1995, 2457, "Boronic Acids" Wiley-VCH, 2005.

In particular the coupling according to step (A) of the process according to the present invention is conducted in the presence of at least one basic compound, for example selected from the group consisting of alkali metal salts of alcohols having 1 to 6 carbon atoms, in particular sodium tert-butoxide, potassium tert-butoxide, potassium carbonate, cesium carbonate, rubidium carbonate or potassium phosphate.

The reaction is preferably conducted in at least one aprotic, organic solvent. Preferred are aromatic solvents, for example selected from the group consisting of toluene, benzene, xylene, mesitylene and mixtures thereof.

As a catalyst particularly preferably a combination of at least one Lewis acid and of at least one palladium compound is used. Particularly preferably, a combination of at least one boron comprising complex and at least one palladium salt is used, for example a combination of tert-Bu₃P-HBF₄ and Pd₂(dba)₃, wherein dba means dibenzylideneacetone. Other suitable ligands and/or palladium comprising reagents are mentioned in the above mentioned scientific papers.

The reaction is conducted at a temperature that is high enough to obtain the desired compound in high yield and high selectivity, for example at 40 to 160 °C, preferably at 60 to 140 °C, particularly preferably at 70 to 120 °C.

The reaction is conducted for a time that is long enough to obtain the desired compound in high yield and high selectivity, for example for 1 to 6 h, preferably for 2 to 4 h, particularly preferably for 3 h.

After the reaction is completed, the reaction mixture can be worked up according to methods that are known to the skilled artisan, for example extraction, filtration, recrystallization, chromatography etc.

The reaction product can be analyzed, for example, by proton- or carbon-NMR, mass spectrometry etc.

The substrates that are used in step (A) of the process according to the present invention, i.e. compounds according to general formula (V) and compounds according to general formula R²-H, wherein R² has the meanings as mentioned above, can be made by methods that are known to a person having ordinary skill in the art. The compound according to general formula R²-H, is, for example, commercially available.

A preferred reaction sequence to obtain the compound of formula (V) is shown in the following:

Step (A01): A compound according to general formula (VI) is transferred to a compound of general formula (VII). 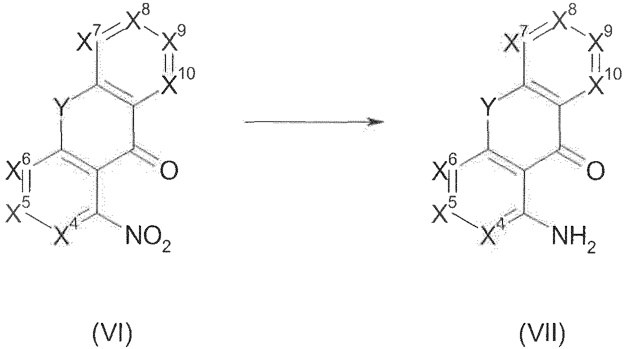 wherein Y, X⁴, X⁵, X⁶, X⁷, X⁸, X⁹ and X¹⁰ have the same meanings as defined above. Y is preferably O.

In particular the reaction according to step (A01) of the process according to the present invention is conducted in the presence of at least one reducing compound, for example selected from the group consisting of HCO₂NH₄ and mixtures thereof.

The reaction is preferably conducted in at least one organic solvent. Preferred are alcohols, for example selected from the group consisting of ethanol, isopropanol and mixtures thereof

Preferably step (A01) is conducted in the presence of a catalyst. As a preferred catalyst palladium is used. Particularly preferably, palladium on carbon is used.

The reaction is conducted at a temperature that is high enough to obtain the desired compound in high yield and high selectivity, for example at 40 to 160 °C, preferably at 60 to 120 °C, particularly preferably at reflux temperature of the solvent.

The reaction is conducted for a time that is long enough to obtain the desired compound in high yield and high selectivity, for example for 0.2 to 6 h, preferably for 0.5 to 3 h.

After the reaction is completed, the reaction mixture can be worked up according to methods that are known to the skilled artisan, for example extraction, filtration, recrystallization, chromatography etc.

Step (A02): The compound according to general formula (VII) is then transferred to a compound of general formula (VIII). 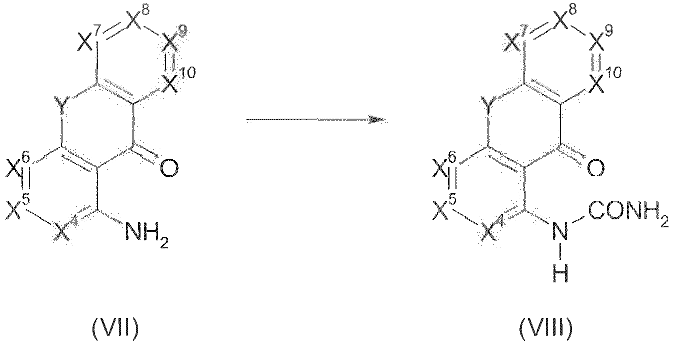 wherein Y, X⁴, X⁵, X⁶, X⁷, X⁸, X⁹ and X¹⁰ have the same meanings as defined above. Y is preferably O.

In particular the reaction according to step (A02) of the process according to the present invention is conducted in the presence of KOCN.

The reaction is preferably conducted in at least one acidic organic solvent. Preferred are carboxylic acids, for example acetic acid.

The reaction is conducted at a temperature that is high enough to obtain the desired compound in high yield and high selectivity, for example at 10 to 40 °C, preferably at room temperature, i.e. 20 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Step (A03): The compound according to general formula (VIII) is then transferred to a compound of general formula (IX). 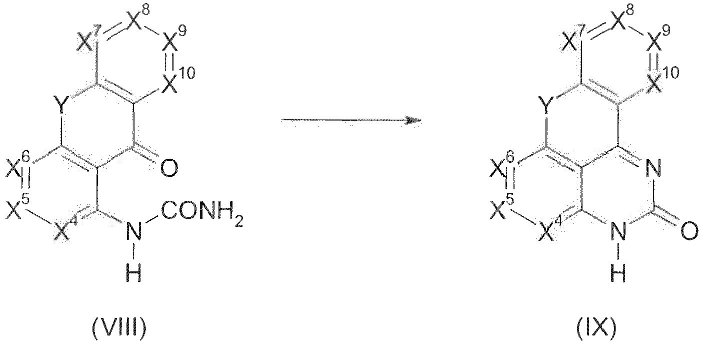 wherein Y, X⁴, X⁵, X⁶, X⁷, X⁸, X⁹ and X¹⁰ have the same meanings as defined above. Y is preferably O.

In particular the reaction according to step (A03) of the process according to the present invention is conducted in the presence of at least one basic compound selected from the group consisting of KOH, NaOH and mixtures thereof.

The reaction is preferably conducted in at least one organic solvent, for example alcohols like ethanol, isopropanol or mixtures thereof
The reaction is conducted at a temperature that is high enough to obtain the desired compound in high yield and high selectivity, for example at 50 to 150 °C, preferably at reflux temperature of the solvent.

The reaction is conducted for a time that is long enough to obtain the desired compound in high yield and high selectivity, for example for 0.1 to 2 h, preferably for 0.3 to 1 h.

After the reaction is completed, the reaction mixture can be worked up according to methods that are known to the skilled artisan, for example extraction, filtration, recrystallization, chromatography etc.

Step (A04): The compound according to general formula (IX) is then transferred to a compound of general formula (X). 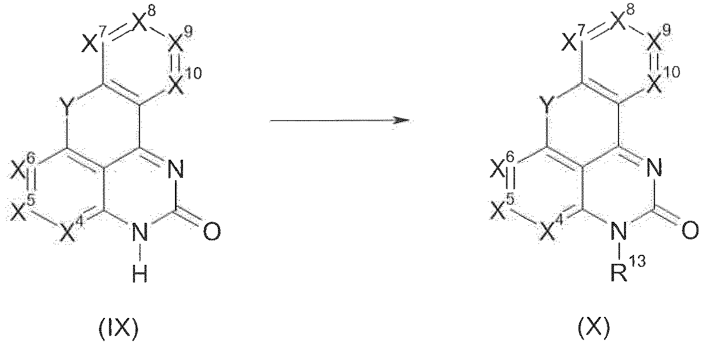 wherein Y, X⁴, X⁵, X⁶, X⁷, X⁸, X⁹ and X¹⁰ have the same meanings as defined above and R¹³ is linear or branched C₁-C₆alkylgroup, preferably methyl. Y is preferably O.

In particular the reaction according to step (A04) of the process according to the present invention is conducted in the presence of at least one strongly basic compound, for example NaH.

The reaction according to step (A04) of the process according to the present invention is further conducted in the presence of at least one compound according to formula R¹³-B, wherein R¹³ has the meanings as mentioned above and B is selected from I, Br or Cl.

The reaction is preferably conducted in at least one organic solvent, for example dimethylformamide or mixtures thereof
The reaction is conducted at a temperature that is high enough to obtain the desired compound in high yield and high selectivity, for example at -20 to 20 °C, preferably at -10 to 10 °C, most preferably at 0 °C.

The reaction is conducted for a time that is long enough to obtain the desired compound in high yield and high selectivity, for example for 0.5 to 4 h, preferably for 1 to 3 h.

After the reaction is completed, the reaction mixture can be worked up according to methods that are known to the skilled artisan, for example extraction, filtration, recrystallization, chromatography etc.

Step (A05): The compound according to general formula (X) is then transferred to a compound of general formula (V). 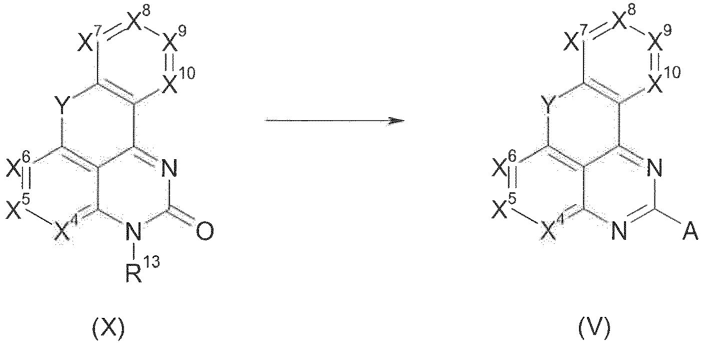 wherein Y, X⁴, X⁵, X⁶, X⁷, X⁸, X⁹, X¹⁰ and R¹³ have the same meanings as defined above. Y is preferably O.

In particular, the reaction according to step (A05) according to the present invention is conducted in the presence of at least one compound, which is able to introduce the moiety A into the molecule. According to the preferred case that A is Cl, chlorination agents are used, for example phosphorous comprising chlorination agents like POCl₃ or PCl₅ in step (A05) of the process according to the present invention
The reaction according to step (A05) is preferably conducted without any solvent, i.e. in substance.

The reaction is conducted at a temperature that is high enough to obtain the desired compound in high yield and high selectivity, for example at 40 to 160 °C, preferably at 60 to 120 °C, particularly preferably at 70 to 90 °C.

The reaction is conducted for a time that is long enough to obtain the desired compound in high yield and high selectivity, for example for 1 to 6 h, preferably for 2 to 4 h.

After the reaction is completed, the reaction mixture can be worked up according to methods that are known to the skilled artisan, for example extraction, filtration, recrystallization, chromatography etc.

Further detailed reaction conditions of this reaction scheme for obtaining the compound of formula (V) can be taken from Carlos M. Martinez et al., J. Heterocyclic Chem., 44, 1035 (2007).

Particularly preferably, the process according to the present invention for the preparation of compounds according to general formula (I) comprises step (A01), followed by step (A02), followed by step (A03), followed by step (A04), followed by step (A05), followed by step (A).

According to another preferred embodiment of the process according to the present invention, the compound according to general formula (V) can also be obtained directly from compound of general formula (IX) using reaction conditions as mentioned in respect of step (A05). The present invention therefore preferably relates to the process according to the present invention, wherein the compound according to general formula (V) can also be obtained directly from compound of general formula (IX) as shown in the following (step (A05a):

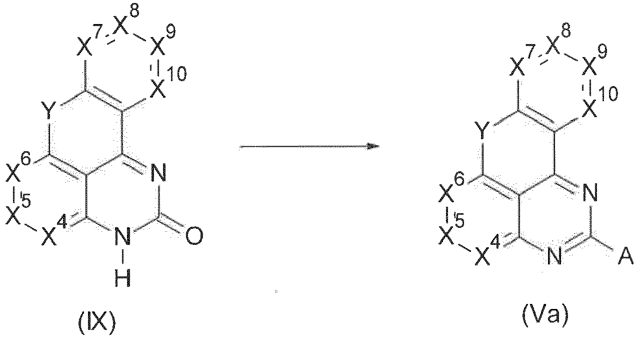

Therefore, particularly preferably, the process according to the present invention for the preparation of compounds according to general formula (I) comprises step (A01), followed by step (A02), followed by step (A03), followed by step (A05a), followed by step (A).

According to the preferred embodiment that Y is S, the process according to the present invention for the preparation of compounds according to general formula (I) comprises step (A01), followed by step (A02), followed by step (A03), followed by step (A05a), followed by step (A).

Compound (VI) as mentioned above can be prepared in chemical reactions that are known to the skilled artisan. Preferred methods for the preparation of the compound according to general formula (VI) are mentioned in Yamagami, Isao et al., Jpn. Kokai Tokkyo Koh, 2008273906, 2008, Kralj, Ana et al., ChemMedChem, 9(1), 151-168, 2014 and Okabayashi, Ichizo and Iwata, Noriko, Chemical and Pharmaceutical Bulletin, 28(9), 2831-5, 1980.

The compound according to general formula (V) as mentioned above can also be prepared by further methods that are explained in the following. For example, the compound according to general formula (V) can be prepared from a compound according to general formula (XI): 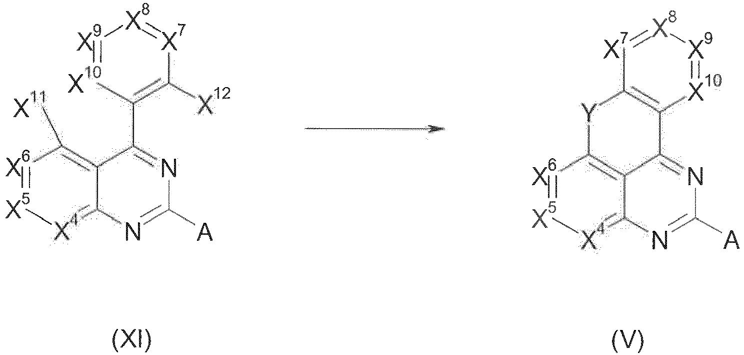 wherein Y, X⁴, X⁵, X⁶, X⁷, X⁸, X⁹, X¹⁰ and A have the same meanings as defined above, X¹¹ and X¹² are independently of each other F or OH, if Y is O, or X¹¹ and X¹² are independently of each other H or S(O)CH₃, if Y is S.

According to the preferred embodiment that Y is O, the following reaction is conducted (step (A06):

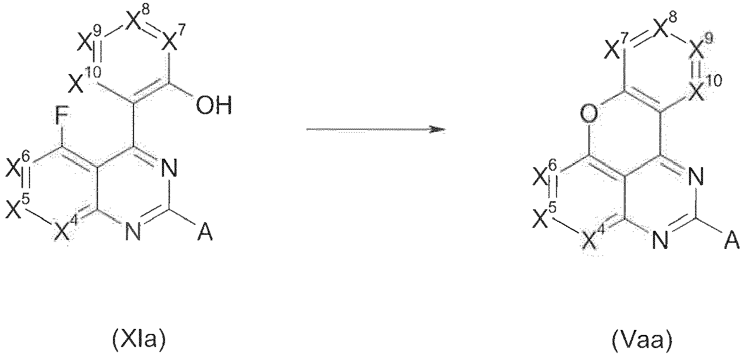

According to this preferred embodiment (step (A06)) the compound according to general formula (Xla) is transferred into the compound according to general formula (Vaa), wherein X⁴, X⁵, X⁶, X⁷, X⁸, X⁹, X¹⁰ and A have the same meanings as defined above.

The reaction is preferably conducted in the presence of at least one basic compound, for example selected from the group consisting of K₂CO₃, NaH and mixtures thereof. Further reaction conditions like temperature, solvent, reaction time etc. are known to the person having ordinary skill in the art.

The compound according to general formula (Xla) can be obtained by any method that is known to the skilled artisan. Preferably the compound according to general formula (Xla) is obtained according to the method described in Kang Hyun-Ju et al., WO 2014088290 or Welsh Dean M et al., Jpn. Kokai Tokkyo Koh, 2014183315, 2014.

According to the preferred embodiment that Y is S, the following reaction is conducted (step (A07):

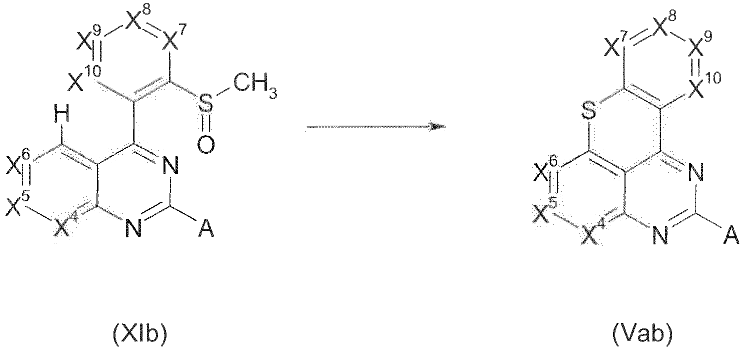

According to this preferred embodiment (step (A07)) the compound according to general formula (Xlb) is transferred into the compound according to general formula (Vab), wherein X⁴, X⁵, X⁶, X⁷, X⁸, X⁹, X¹⁰ and A have the same meanings as defined above.

The reaction is preferably conducted in the presence of at least one acidic compound, for example selected from the group consisting of (CF₃SO₂)₂, CF₃SO₂OH, and mixtures thereof.

Further reaction conditions like temperature, solvent, reaction time etc. are known to the person having ordinary skill in the art.

### Ir complexes of formula (XX)

The composition according to the present invention comprises beside at least one compound of formula (I) at least one Ir complex represented by the following formula (XX)

IrLg₁Lg₂Lg₃ (XX)

wherein
Lg₁, Lg₂ and Lg₃ are selected from the following groups A-1, A-2 and A-3 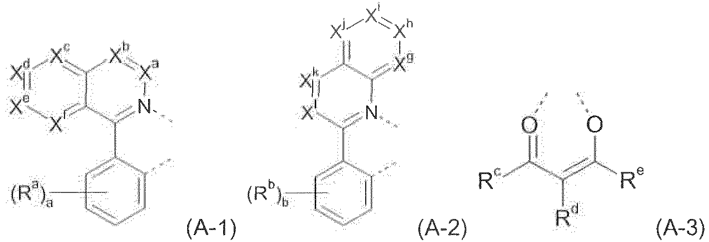
wherein one or two of Lg₁, Lg₂ and Lg₃, preferably one, are a group A-3, and one or two of Lg₁, Lg₂ and Lg₃, preferably two, are selected from the group consisting of A-1 and A-2,
wherein
X^{a} is CR^{a'} or N;
X^{b} is CR^{b'} or N;
X^{c} is CR^{c'} or N;
X^{d} is CR^{d'} or N;
X^{e} is CR^{e'} or N;
X^{f} is CR^{f} or N;
X^{g} is CR^{g} or N;
X^{h} is CR^{h} or N;
Xⁱ is CRⁱ or N;
X^{j} is CR^{j} or N;
X^{k} is CR^{k} or N;and
X^{l} is CR^{l} or N;
wherein at least one of X^{a}, X^{b}, X^{c}, X^{d}, X^{e} and X^{f} is CR^{a'}, CR^{b'}, CR^{c'}, CR^{d'}, CR^{e'} respectively CR^{f}, and at least one of X^{g}, X^{h}, Xⁱ, X^{j}, X^{k} and X^{l} is CR^{g}, CR^{h}, CRⁱ, CR^{j}, CR^{k} respectively CR^{l};
R^{a'}, R^{b'}, R^{c'}, R^{d'}, R^{e'}, R^{f}, R^{g}, R^{h}, Rⁱ, R^{j}, R^{k} and R^{l} each independently represent hydrogen, deuterium, a halogen, a cyano group, a substituted or unsubstituted C₁-C₂₅alkyl group, a substituted or unsubstituted C₁-C₂₅fluoroalkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group;
wherein at least one of R^{a'}, R^{b'}, R^{c'}, R^{d'}, R^{e'} and R^{f}, and at least one of R^{g}, R^{h}, Rⁱ, R^{j}, R^{k} and R^{l} is a substituted or unsubstituted C₁-C₂₅alkyl group, a substituted or unsubstituted C₁-C₂₅fluoroalkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably a substituted or unsubstituted C₁-C₂₅alkyl group;
R^{a} to R^{e} each independently represent hydrogen, deuterium, a halogen, a cyano group, a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably, R^{a} to R^{e} each independently represent hydrogen, deuterium, a substituted or unsubstituted C₁-C₁₀alkyl group, a substituted or unsubstituted C₃-C₁₀cycloalkyl group, a substituted or unsubstituted C₆-C₂₀ary group, or a substituted or unsubstituted 5- to 20-membered heteroaryl group;
a and b each independently represent an integer of 0 to 5; and
each of R^{a} and R^{b} is the same or different;
wherein the dotted lines are bonding sites.

Preferably, 0, 1, 2 or 3, more preferably 0 or 1 of X^{a}, X^{b}, X^{c}, X^{d}, X^{e} and X^{f} are N and the remaining of X^{a}, X^{b}, X^{c}, X^{d}, X^{e} and X^{f} are CR^{a'}, CR^{b'}, CR^{c'}, CR^{d'}, CR^{e'} respectively CR^{f}.

Preferably, R^{a'}, R^{b'}, R^{c'}, R^{d'}, R^{e'}, R^{f}, R^{g}, R^{h}, Rⁱ, R^{j}, R^{k} and R^{l} each independently represent hydrogen, deuterium, CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl, most preferably hydrogen, wherein at least one R^{a'}, R^{b'}, R^{c'}, R^{d'}, R^{e'} and R^{f}, and at least one of R^{g}, R^{h}, Rⁱ, R^{j}, R^{k} and R^{l} is preferably CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl.

Preferably, R^{a} to R^{e} each independently represent hydrogen, deuterium, a substituted or unsubstituted C₁-C₈alkyl group, a substituted or unsubstituted C₃-C₆cycloalkyl group, a substituted or unsubstituted C₆-C₁₄aryl group, or a substituted or unsubstituted 5- to 13-membered heteroaryl group, more preferably hydrogen methyl, CH(CH₃)₂, CH(CH₂CH₃)₂ or CH₂CH(CH₃)₂.

Preferably, a and b each independently are 0, 1, 2 or 3, more preferably 0, 1 or 2.

Preferably, the compound of formula (XX) is represented by the following formulae (XX-1) or (XX-2): 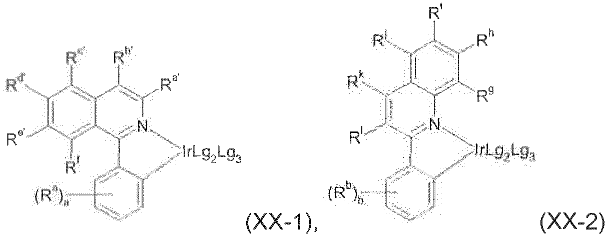
wherein Lg₂ and Lg₃ are a group A-1, A-2 or A-3, wherein in formula (XX-1), at least one of Lg₂ and Lg₃ is a group A-3, preferably, one of Lg₂ and Lg₃ is a group A-3 and the other is a group A-1; and in formula (XX-2), at least one of Lg₂ and Lg₃ is a group A-3, preferably, one of Lg₂ and Lg₃ is a group A-3 and the other is a group A-2;
and the groups A-1, A-2 or A-3 and residues R^{a'}, R^{b'}, R^{c'}, R^{d'}, R^{e'}, R^{f}, R^{g}, R^{h}, Rⁱ, R^{j}, R^{k}, R^{l}, and R^{a} to R^{e} and indices a and b are defined above.

More preferably, the compound of formula (XX) is represented by one of the following formulae (XX-3) and (XX-4): 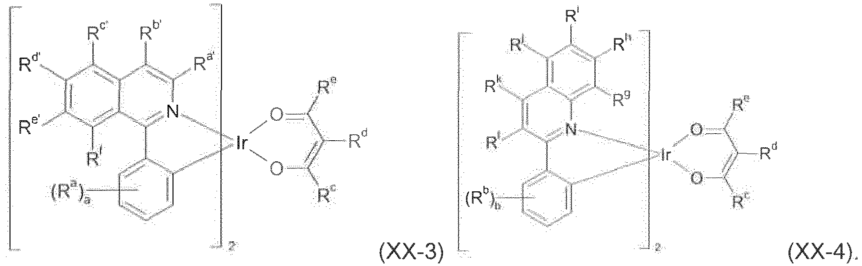 wherein the residues R^{a'}, R^{b'}, R^{c'}, R^{d'}, R^{e'}, R^{f}, R^{g}, R^{h}, Rⁱ, R^{j}, R^{k}, R^{l}, and R^{a} to R^{e} and indices a and b are defined above.

Most preferably, the compounds of formulae (XX-3) and (XX-4) are represented by the following formulae (XX-5) and (XX-6): 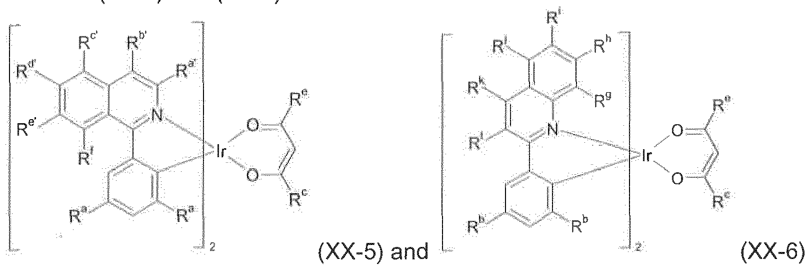
wherein the residues R^{a'}, R^{b'}, R^{c'}, R^{d'}, R^{e'}, R^{f}, R^{g}, R^{h}, Rⁱ, R^{j}, R^{k}, R^{l} and indices a and b are defined above, and
R^{a} and R^{b} each independently represent a substituted or unsubstituted C₁-C₂₅alkyl group, methyl, CH(CH₃)₂, CH(CH₂CH₃)₂ or CH₂CH(CH₃)₂.

Further most preferably, the compounds of formulae (XX-5) and (XX-6) are represented by the following formulae (XX-7) and (XX-8): 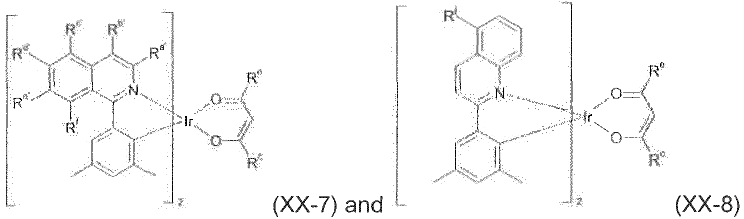 wherein
one or two of R^{a'}, R^{b'}, R^{c'}, R^{d'}, R^{e'} and R^{f} each independently represent CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl;
and the remaining of R^{a'}, R^{b'}, R^{c'}, R^{d'}, R^{e'} and R^{f} are hydrogen;
R^{j} is CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl;
and
R^{c} and R^{e} are independently of each other methyl, CH(CH₃)₂, CH(CH₂CH₃)₂ or CH₂CH(CH₃)₂.

The Ir complexes of formula (XX) are commercially available and/or prepared as known in the art.

Examples for most preferred compounds of formula (XX) are mentioned below.

### Compositions according to the present invention

The composition of the present invention comprises
i) at least one Ir complex represented by formula (XX), and
ii) at least one compound represented by the general formula (I).

Suitable and preferred compounds of formulae (XX) and (I) are mentioned above.

Preferably, the composition according to the present invention comprises:
i) from 0.1 to 70% by weight, preferably 1 to 30% by weight, of at least one Ir complex represented by formula (XX), and
ii) 30 to 99.9% by weight, preferably 70 to 99% by weight, of at least one compound represented by the general formula (I),
where the sum total of the Ir complex represented by formula (XX) and of the compound represented by the general formula (I) adds up to 100% by weight.

The composition according to the present invention is particularly suitable as composition for the light-emitting layer in OLED. The structure of OLEDs is known in the art. Suitable structures are described below. The at least one Ir complex of formula (XX) is usually employed as emitter material (dopant) in the light emitting layer and the at least one compound of formula (I) is usually employed as matrix material (host).

Preferably, the composition comprises:
i) At least one Ir complex represented by the formula 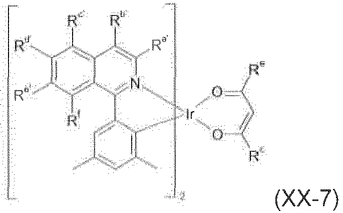 or 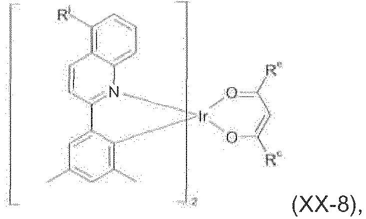 and
ii) at least one compound represented by one of the formulae (XIIIa1), (XIIIa2), (XIIIa3), (XIIIa4), (XIIIa5), (XIIIa6), (XIIIa7), (XIIIa8), (XIIIb1), (XIIIb2), (XIIIb3), (XIIIb4), (XIIIb5), (XIIIb6)), (XIIIb7), (XIIIb8), (XIVb1), (XIVb2), (XIVb3), (XVa1), (XVa2), (XVa3), (XVa4), (XVa5), (XVa6), (XVa7), (XVa8), (XVb1), (XVb2), (XVb3), (XVb4), (XVb5), (XVb6), (XVb7) or (XVb8).

Suitable amounts of components i) and ii) are mentioned above.

### Compositions according to the present invention in organic electronics applications

In the following the use of the compositions according to the present invention as mentioned above in organic electronic applications will be explained.

It has been found that the compositions according to the present invention are particularly suitable for use in applications in which charge carrier conductivity is required, especially for use in organic electronics applications, more preferably for use in organic light-emitting diodes (OLEDs).

The compositions according to the present invention being particularly suitable in OLEDs for use in a light-emitting layer, wherein the compound of formula (I) is preferably used as host, as a single host or as a host in combination with one or more further hosts, and the Ir complex of formula (XX) is preferably used as a dopant, as a single dopant or as a dopant in combination with one or more further dopants.

The present invention therefore relates to an electronic device, preferably an organic electroluminescent device, more preferably an organic light emitting diode (OLED), comprising least one dopant material of formula (XX) as described in the present application and at least one host material of formula (I) as described in the present application.

The present invention also relates to an electronic device, preferably an organic electroluminescent device, more preferably an organic light emitting diode (OLED), comprising a composition according to the present invention.

The present invention therefore preferably relates to the electronic device according to the present invention, preferably an organic electroluminescence device, more preferably an organic light emitting diode (OLED), comprising a cathode, an anode, and a plurality of organic thin film layers provided between the cathode and the anode, the plurality of organic thin film layers comprising at least one emitting layer comprising least one dopant material of formula (XX) as described in the present application and at least one host material of formula (I) as described in the present application.

The present invention therefore preferably relates to the electronic device according to the present invention, preferably an organic electroluminescence device, more preferably an organic light emitting diode (OLED), comprising a cathode, an anode, and a plurality of organic thin film layers provided between the cathode and the anode, the plurality of organic thin film layers comprising at least one emitting layer comprising the composition according to the present invention.

The present invention further relates to an electronic equipment comprising the organic electroluminescence device according to the present invention.

The present invention also relates to an emitting layer, preferably present in an electronic device, more preferably in an electroluminescence device, particularly preferably in an organic light emitting diode (OLED), comprising least one dopant material of formula (XX) as described in the present application and at least one host material of formula (I) as described in the present application.

The present invention also relates to an emitting layer, preferably present in an electronic device, more preferably in an electroluminescence device, particularly preferably in an organic light emitting diode (OLED), comprising the composition according to the present invention.

The present invention therefore preferably relates to the use of the compound of formula (XX) as described in the present application as dopant material and the compound of formula (I) as described in the present application as host material, respectively of the composition according to the present invention as defined above in an electronic device, preferably in an electroluminescence device, particularly preferably in an organic light emitting diode (OLED), preferably in an emitting layer.

In the case of use of the compound of formula (XX) as described in the present application as dopant material and the compound of formula (I) as described in the present application as host material, respectively of the compositions according to the present invention in OLEDs, OLEDs with a good overall performance, especially with good efficiencies which can be operated especially at a low use and operating voltage are obtained.

According to the present application, the terms matrix and host are used interchangeable.

Suitable structures of organic electronic devices, especially organic light-emitting diodes (OLED), are known to those skilled in the art and are specified below.

For example, the electronic device, preferably an organic electroluminescence device, more preferably an organic light emitting diode (OLED), according to the present invention comprises a cathode, an anode, and a plurality of organic thin film layers provided between the cathode and the anode, the organic thin film layers comprising an emitting layer comprising the compound of formula (XX) as described in the present application as dopant material and the compound of formula (I) as described in the present application as host material, respectively the composition according to the present invention .

Preferably, the present invention provides an organic light-emitting diode (OLED) comprising an anode and a cathode and a light-emitting layer arranged between the anode and the cathode, and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the compound of formula (XX) as described in the present application as dopant material and the compound of formula (I) as described in the present application as host material, respectively the composition according to the present invention is present in the light-emitting layer. The present application further relates to a light-emitting layer, preferably present in an electronic device, more preferably in an electroluminescence device, particularly preferably in an organic light emitting diode (OLED), comprising the compound of formula (XX) as described in the present application as dopant material and the compound of formula (I) as described in the present application as host material, respectively the composition according to the present invention. Examples of preferred compounds according to general formula (I) and compounds according to the general formula (XX) as well as preferred compositions are shown above.

Most preferably, the electronic device according to the present invention is an organic light emitting diode (OLED).

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron transport layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS.

### Hole transport layer (c):

According to a preferred embodiment the OLED according to the present invention comprises at least one compound according to general formula (I) or their preferred embodiments as a charge transporting material, preferably as a hole transporting layer. In addition to the compounds according to general formula (I) or without these compounds either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 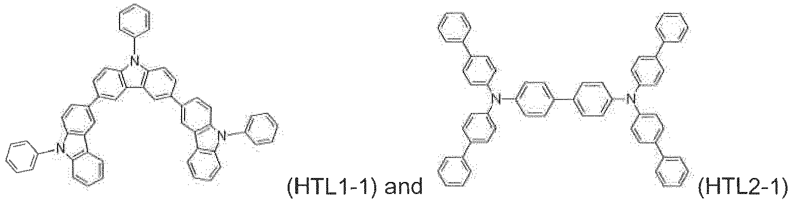 constitute the hole transport layer.
Customarily used hole-transporting molecules are selected from the group consisting of 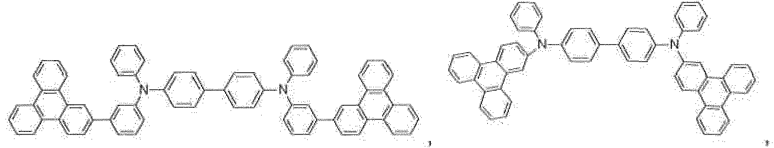 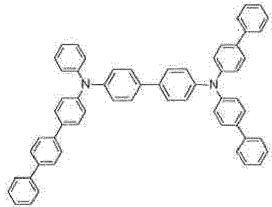 (4-phenyl-N-(4-phenylphenyl)-N-[4-[4-(N-[4-(4-phenyl-phenyl)phenyl]anilino)phenyl]phenyl]aniline), 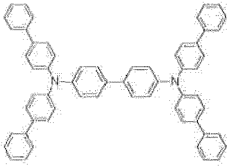 (4-phenyl-N-(4-phenylphenyl)-N-[4-[4-(4-phenyl-N-(4-phenylphenyl)anilino)phenyl]phenyl]aniline), 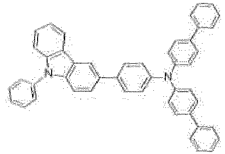 (4-phenyl-N-[4-(9-phenylcarbazol-3-yl)phenyl]-N-(4-phenylphenyl)aniline), 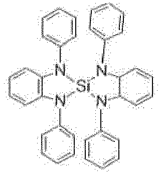 (1,1',3,3'-tetraphenylspiro[1,3,2-benzodiazasilole-2,2'-3a,7a-dihydro-1,3,2-benzodiazasilole]), 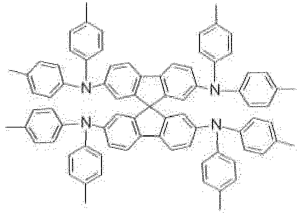 (N2,N2,N2',N2',N7,N7,N7',N7'-octakis(p-tolyl)-9,9'-spirobi[fluorene)-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole injecting layer are a porphyrin compound, an aromatic tertiary amine compound, or a styrylamine compound. Particularly preferable examples include an aromatic tertiary amine compound such as hexacyanohexaazatriphenylene (HAT).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂, MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254.

### Exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application.

According to a preferred embodiment of the present invention, at least one compound of general formula (I) is present in the exciton blocking layer of the OLED according to the present invention.

### Emitting layer (e)

The light emitting layer is an organic layer having a light emitting function and is formed from one or more layers, wherein one of the layers comprises a host material (first host material), optionally a second host material, and the light emitting material. According to the present invention, the light-emitting layer comprises the compound of formula (XX) as described in the present application as dopant material and the compound of formula (I) as described in the present application as host material, respectively the composition according to the present invention. The compounds of formulae (XX) and (I) and the composition of the present invention are described above.

When the light emitting layer is composed of two or more layers, the light emitting layer or layers other than that mentioned above contains or contain a host material and a dopant material when a doping system is employed. The major function of the host material is to promote the recombination of electrons and holes and confine excitons in the light emitting layer. The dopant material causes the excitons generated by recombination to emit light efficiently.

In case of a phosphorescent device, the major function of the host material is to confine the excitons generated on the dopant in the light emitting layer.

The light emitting layer may be made into a double dopant layer, in which two or more kinds of dopant materials having high quantum yield are used in combination and each dopant material emits light with its own color. For example, to obtain a yellow emission, a light emitting layer formed by co-depositing a host, a red-emitting dopant and a green-emitting dopant is used.

In a laminate of two or more light emitting layers, electrons and holes are accumulated in the interface between the light emitting layers, and therefore, the recombination region is localized in the interface between the light emitting layers, to improve the quantum efficiency.

The light emitting layer may be different in the hole injection ability and the electron injection ability, and also in the hole transporting ability and the electron transporting ability each being expressed by mobility.

The light emitting layer is formed, for example, by a known method, such as a vapor deposition method, a spin coating method, and LB method. Alternatively, the light emitting layer may be formed by making a solution of a binder, such as resin, and the material for the light emitting layer in a solvent into a thin film by a method such as spin coating.

The light emitting layer is preferably a molecular deposit film. The molecular deposit film is a thin film formed by depositing a vaporized material or a film formed by solidifying a material in the state of solution or liquid. The molecular deposit film can be distinguished from a thin film formed by LB method (molecular build-up film) by the differences in the assembly structures and higher order structures and the functional difference due to the structural differences.

The light-emitting layer (e) according to the present invention comprises at least one emitter material of formula (XX). Further emitter which may be additionally employed for example in the case that more than one light emitting layer is present in the OLED, are fluorescence or phosphorescence emitter, suitable emitter materials being known to those skilled in the art.

The emission wavelength of the phosphorescent dopant used in the light emitting layer is not particularly limited. In a preferred embodiment, at least one of the phosphorescent dopants used in the light emitting layer has the peak of emission wavelength of in general 430 nm or longer and 780 nm or shorter, preferably 490 nm or longer and 700 nm or shorter and more preferably 490 nm or longer and 650 nm or shorter. Most preferred are green emitter materials (490 to 570 nm). In another preferred embodiment, red emitter materials (570 to 680 nm) are preferred.

The phosphorescent dopant (phosphorescent emitter material) is a compound of formula (XX), which usually emits light by releasing the energy of excited triplet state.
The compounds according to general formula (I) are used as the matrix (=host material) in the light-emitting layer.

Suitable metal complexes of formula (XX) for use in the inventive OLEDs, preferably as emitter material, are, for example,
Further suitable red emitters are shown in WO 2008/109824. Preferred red emitters according to this document are the following compounds:

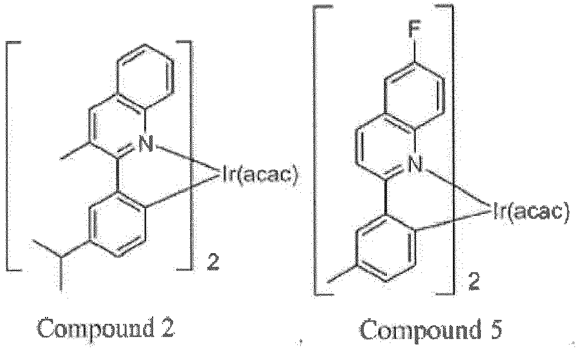

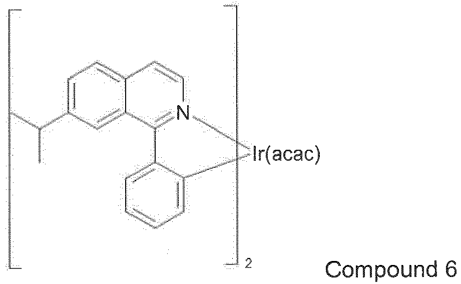

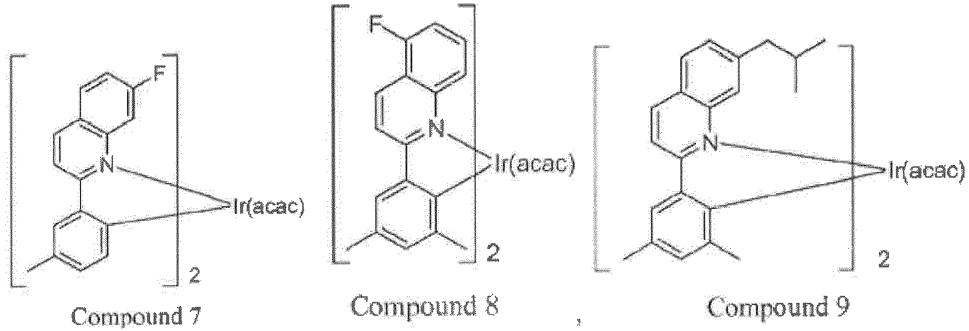

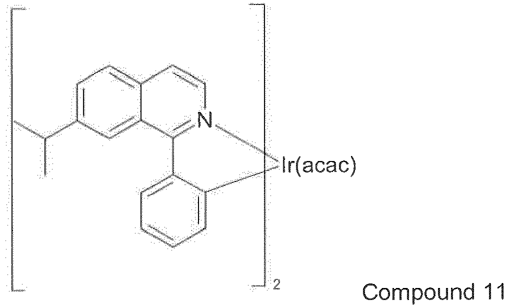

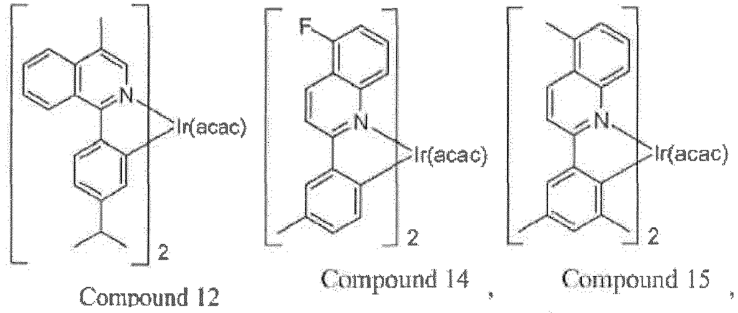

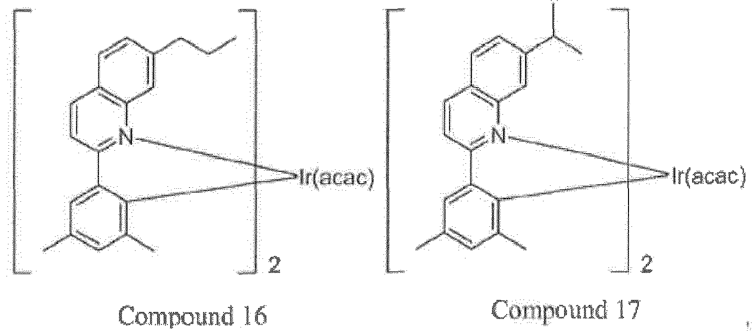

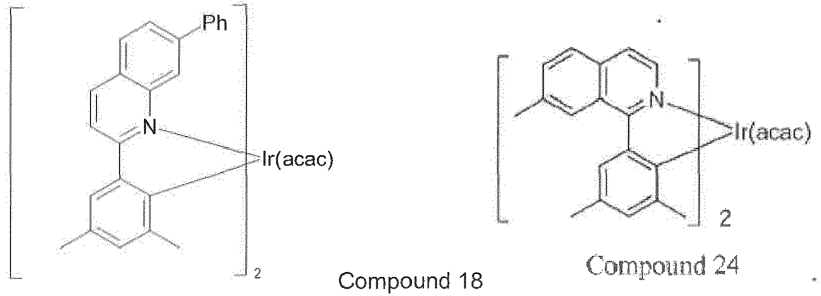

The emitter materials (dopants), preferably the phosphorescent emitter materials, may be used alone or in combination of two or more.

Further red emitters that may be used in the OLEDs according to the present invention are disclosed in US 2013/0299795 and are shown in the following:

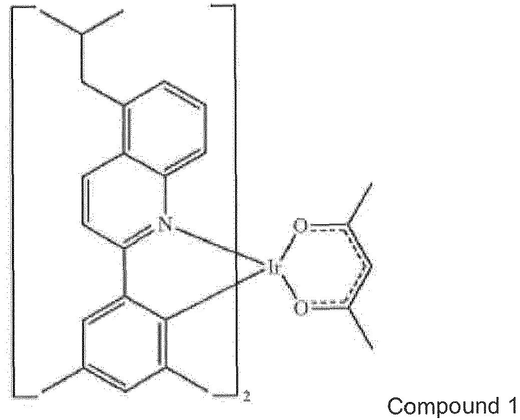

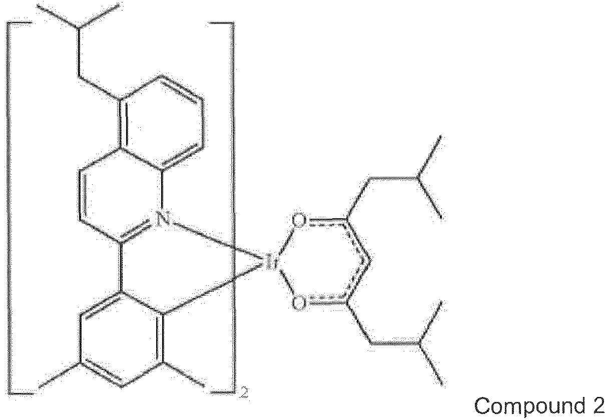

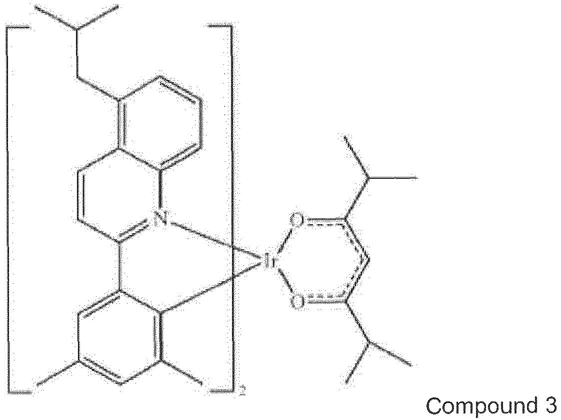

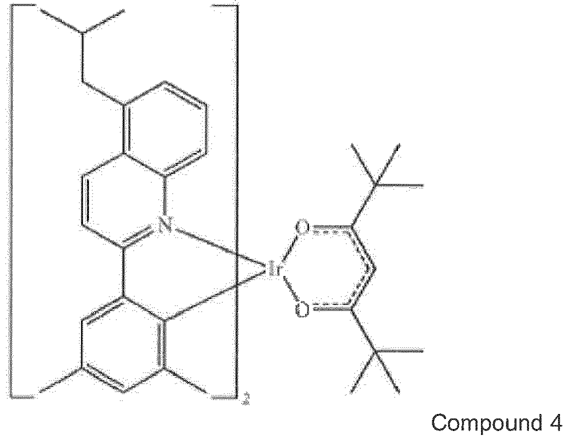

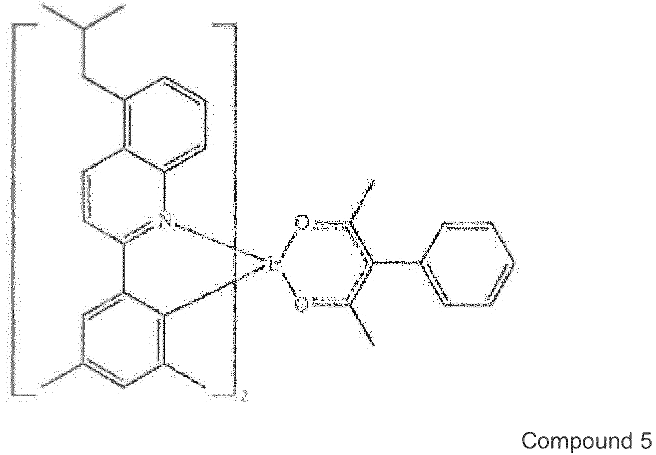

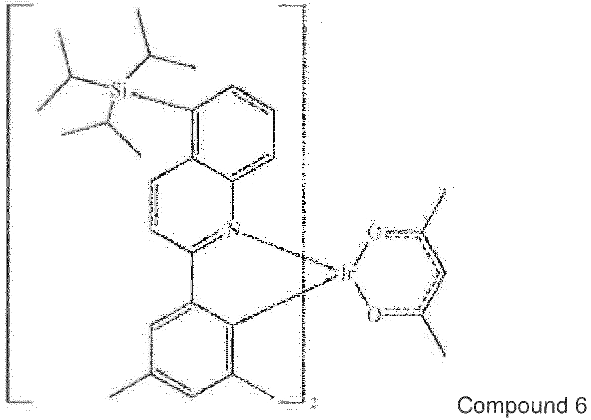

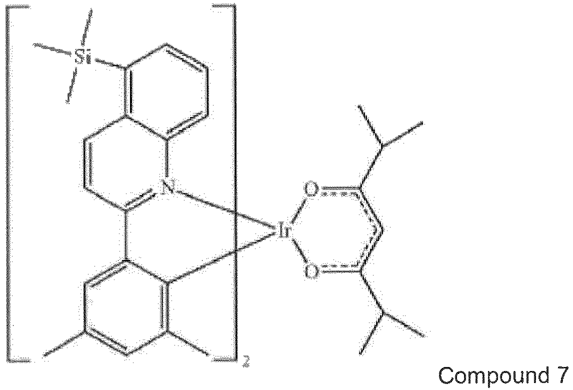

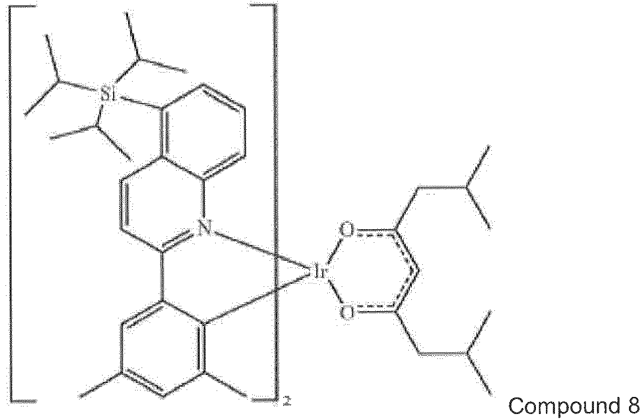

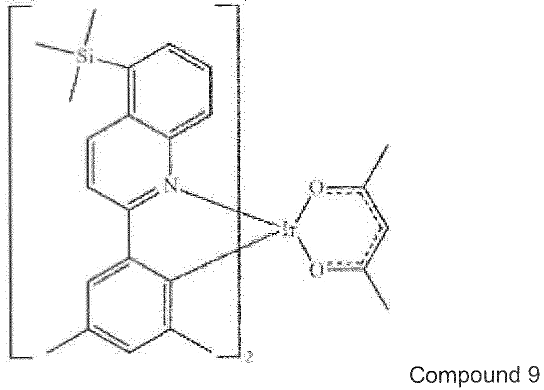

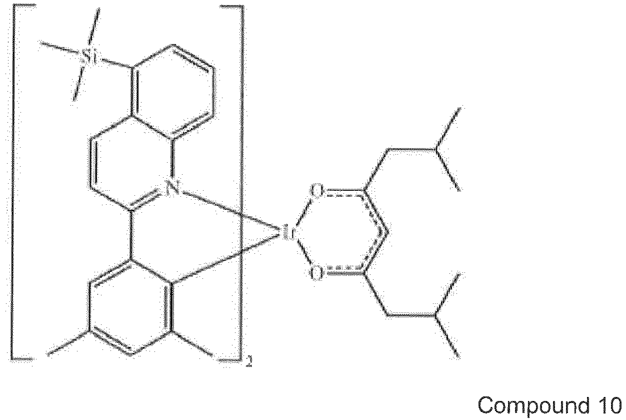

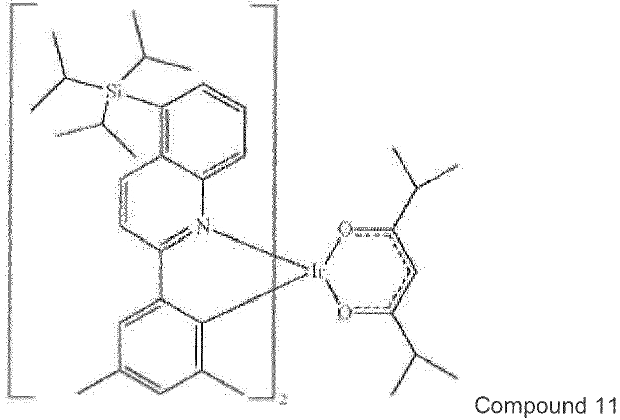

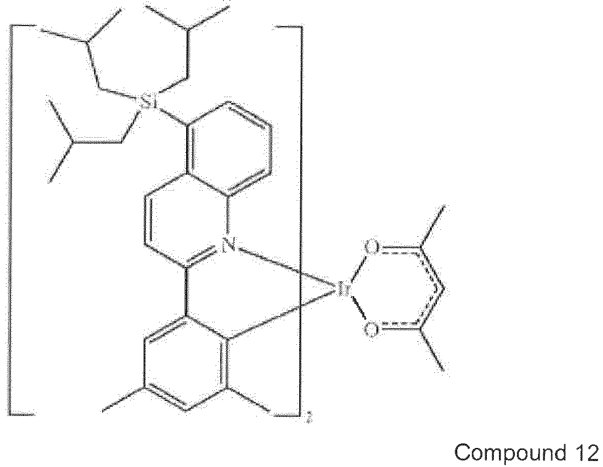

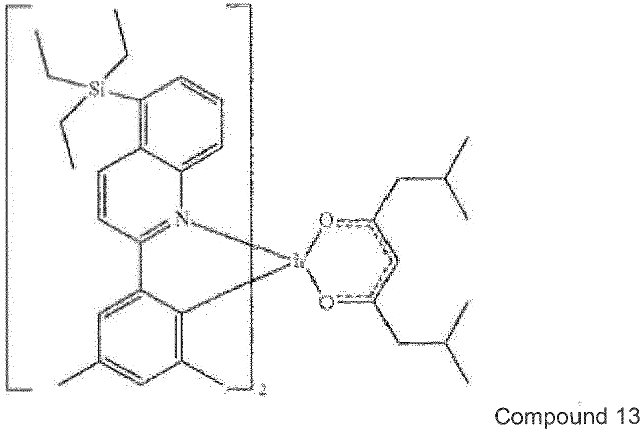

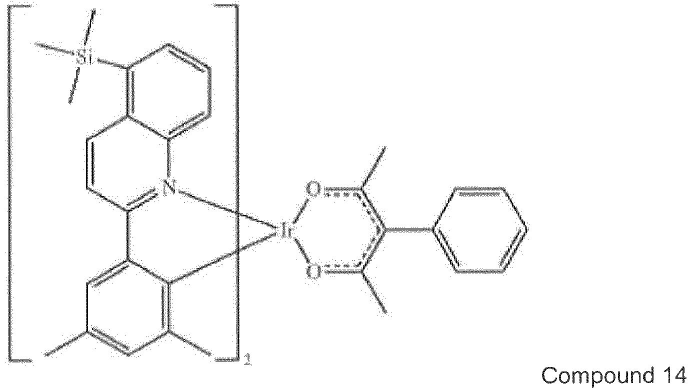

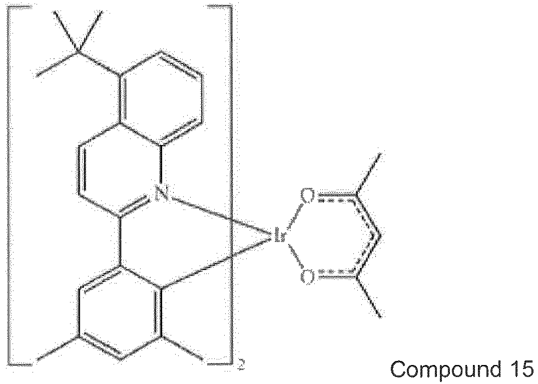

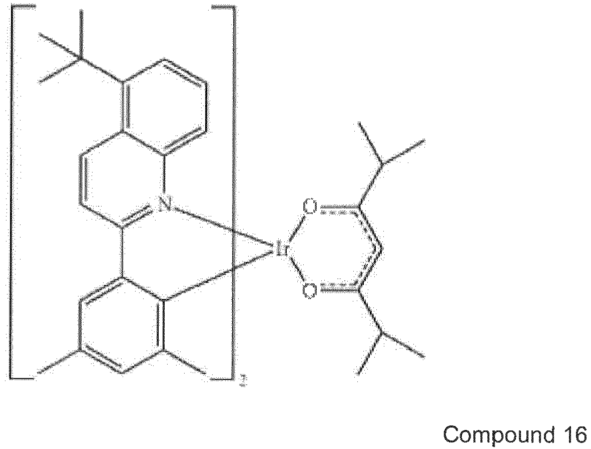

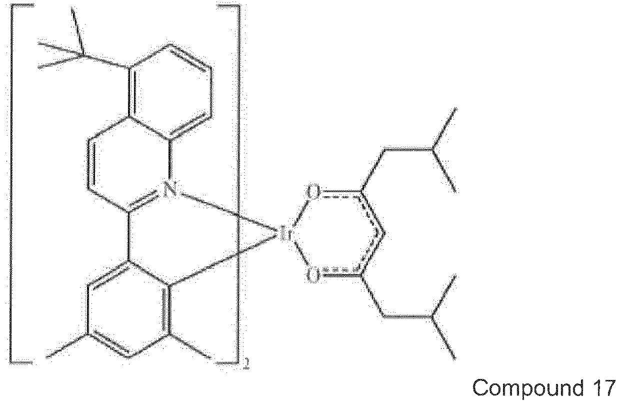

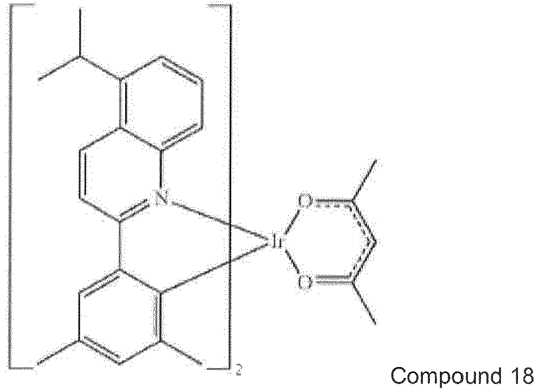

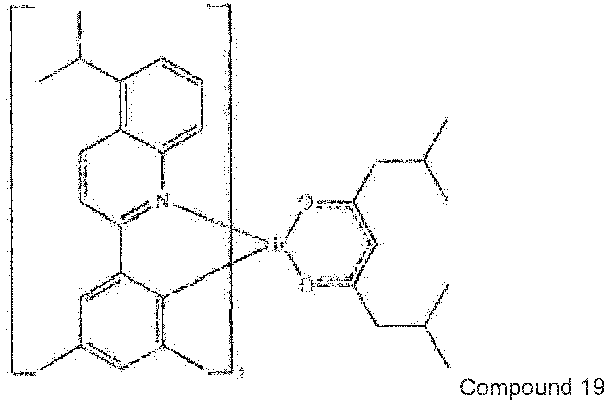

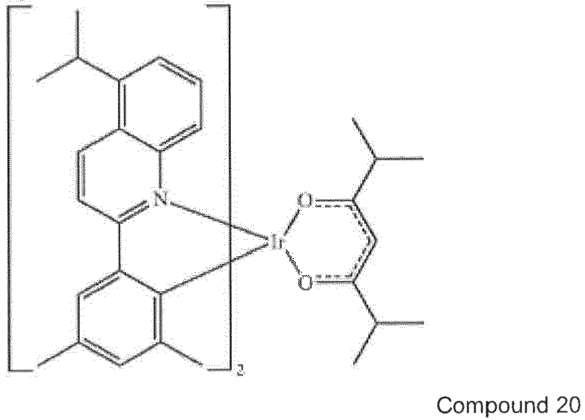

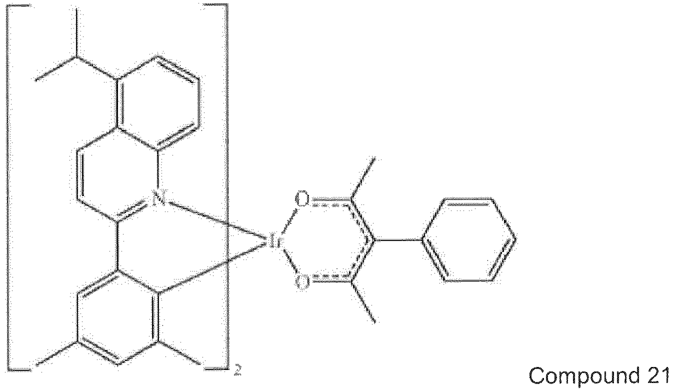

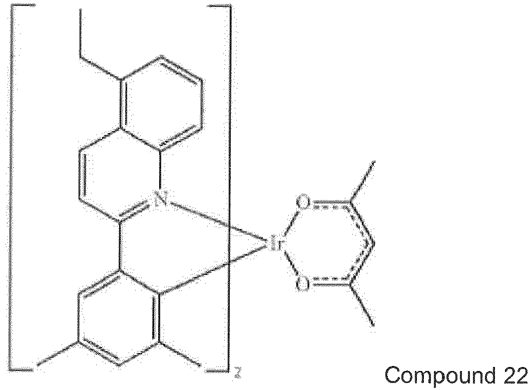

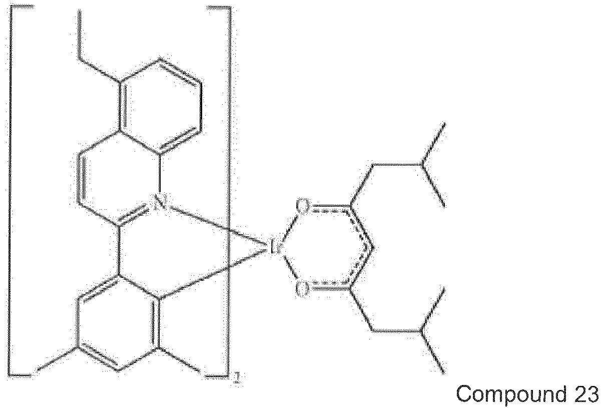

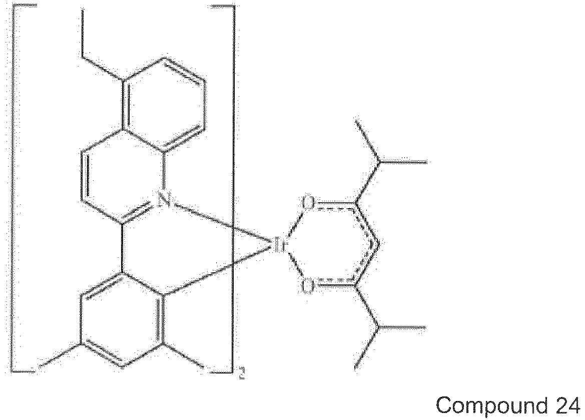

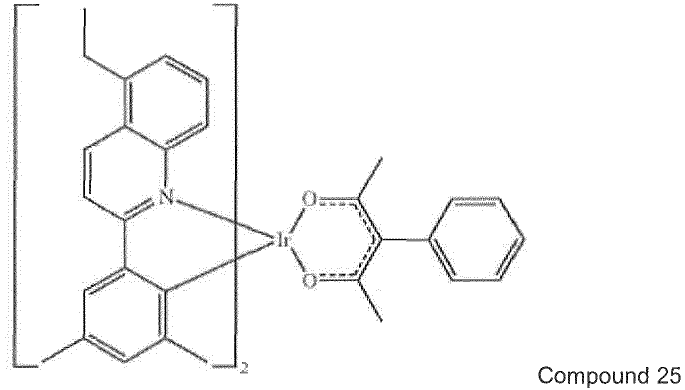

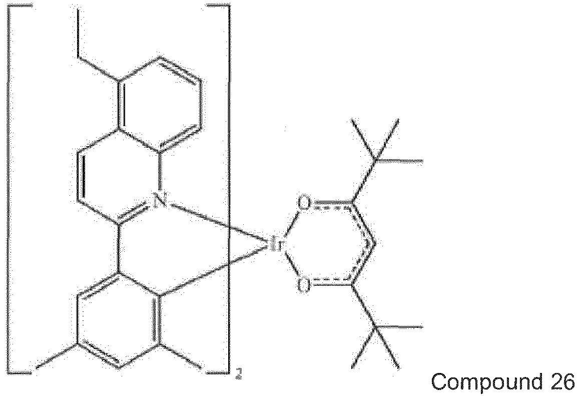

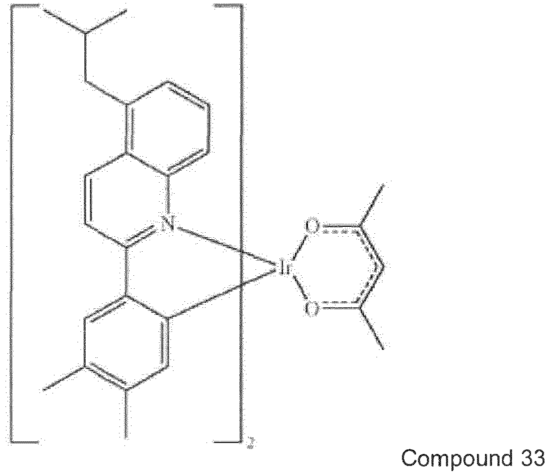

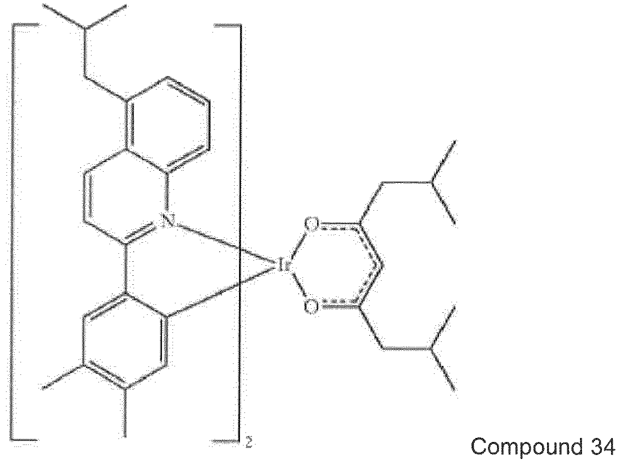

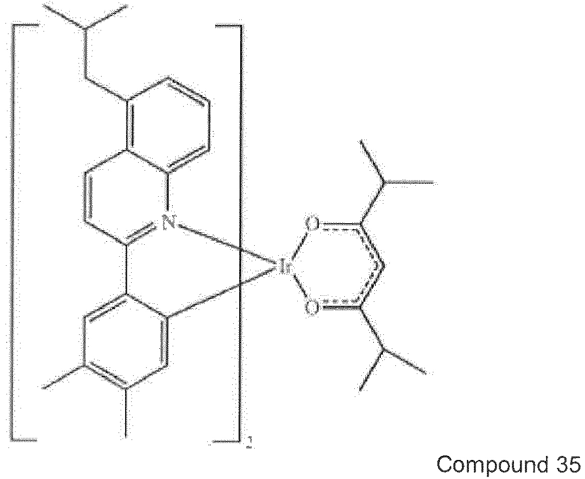

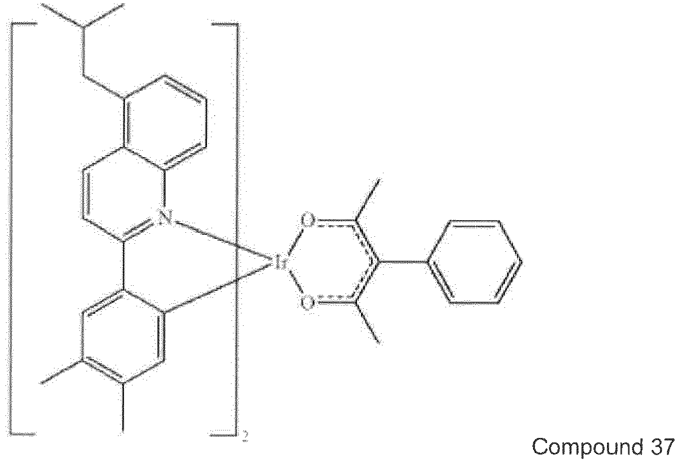

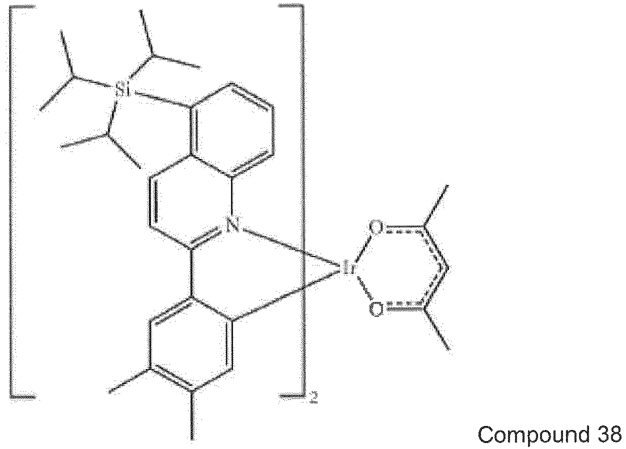

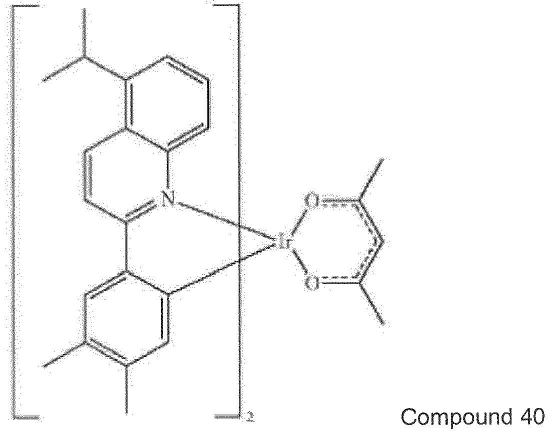

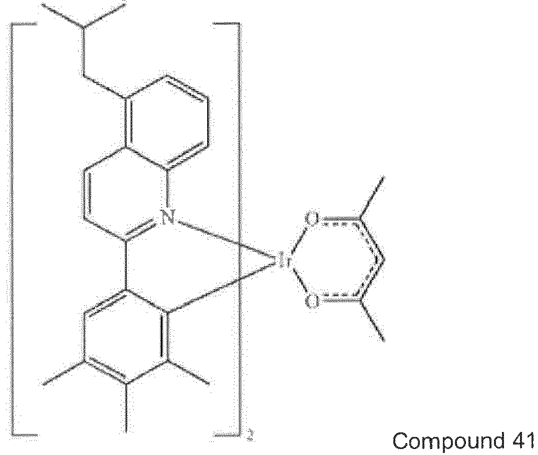

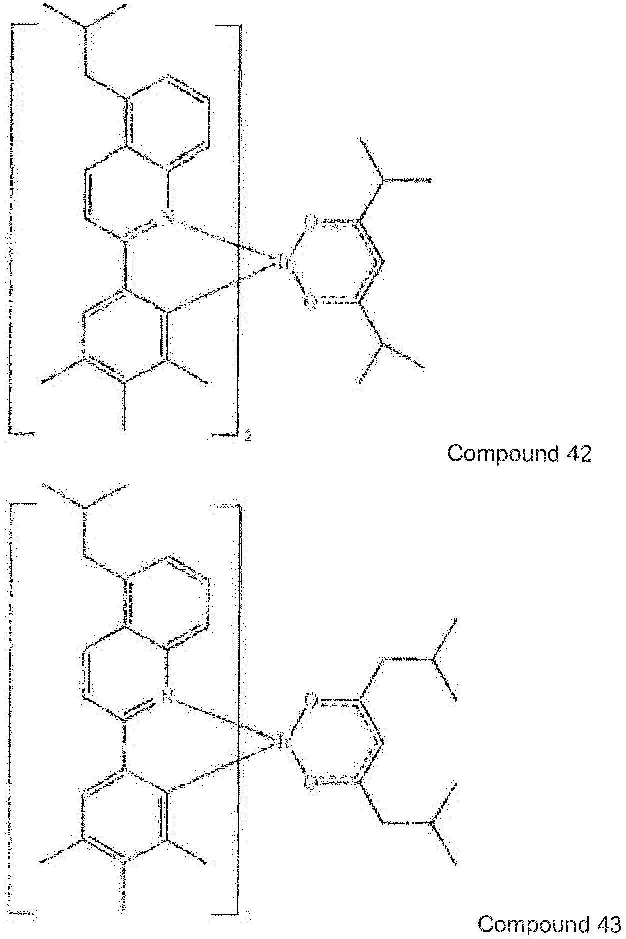

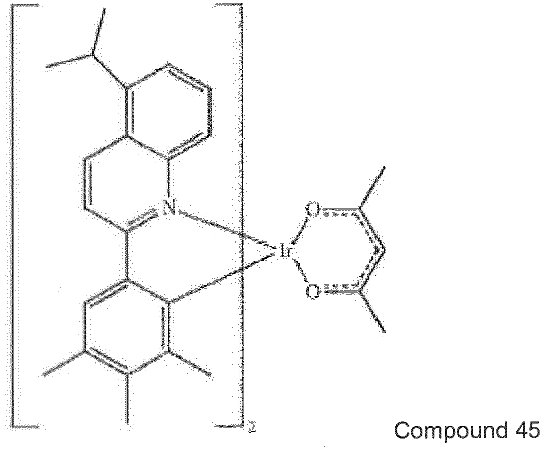

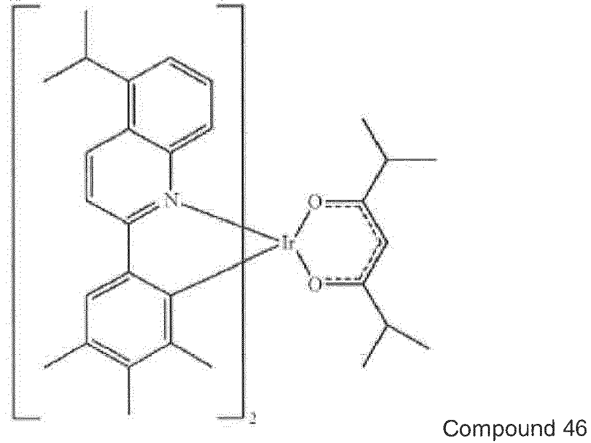

Further red emitters that may be used in the OLEDs according to the present invention are disclosed in US 2013/0146848 and are shown in the following: 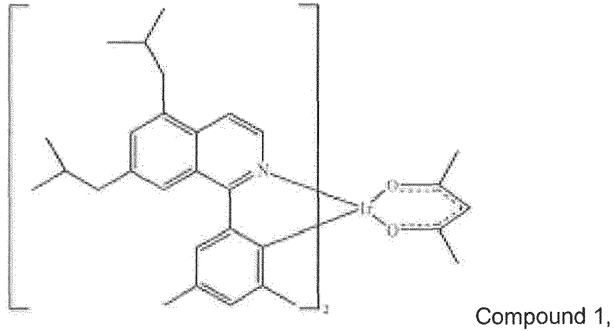 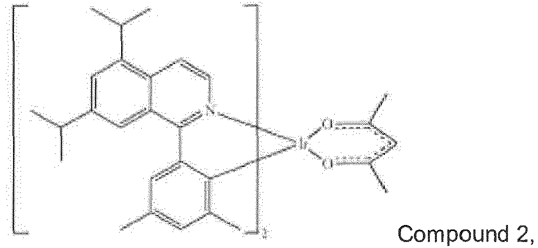 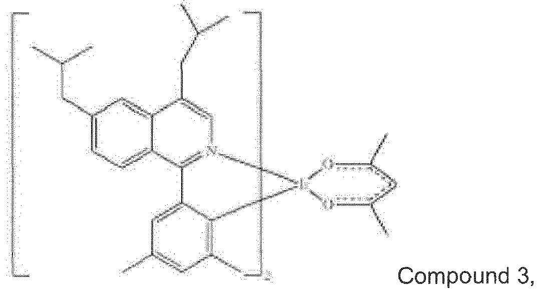 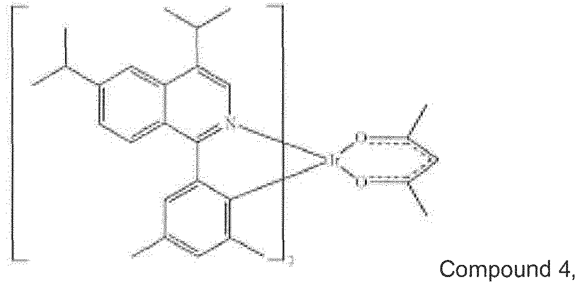 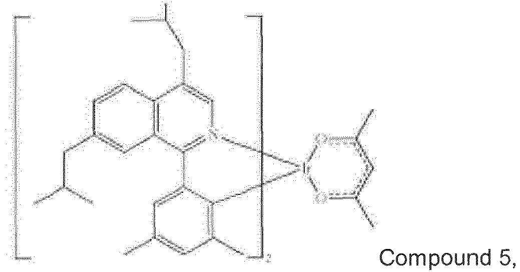 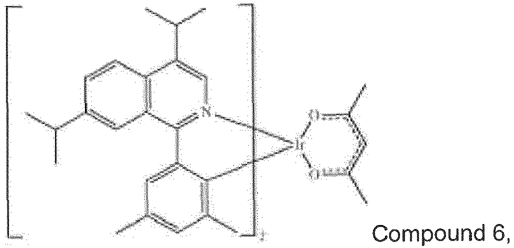 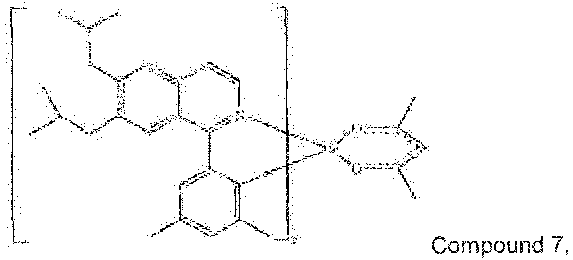 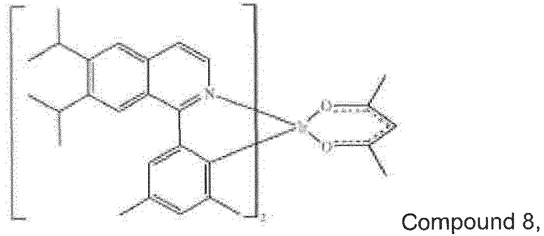 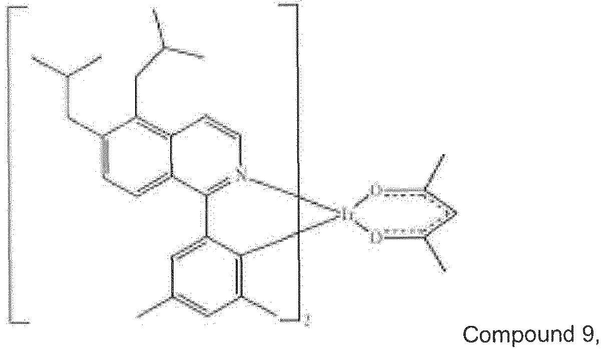 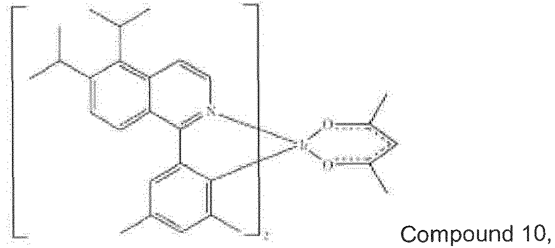 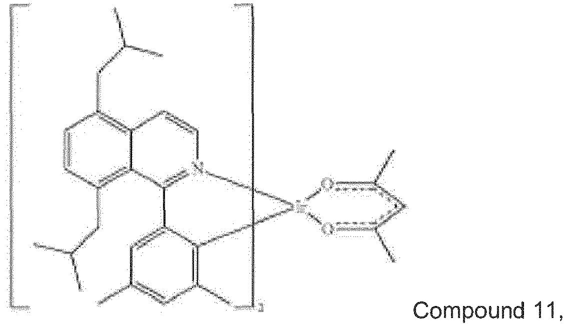 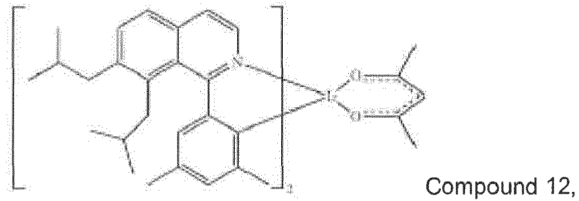 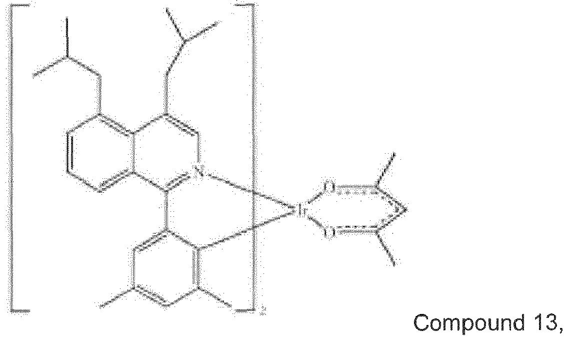 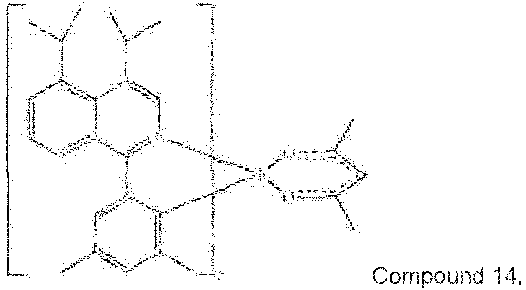 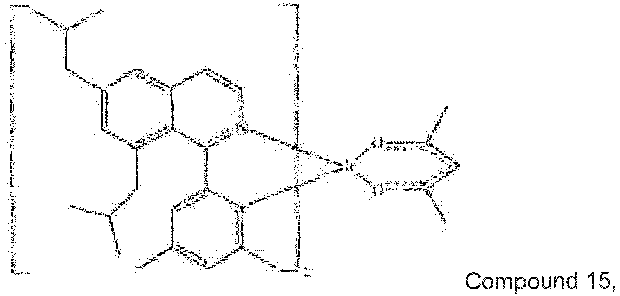 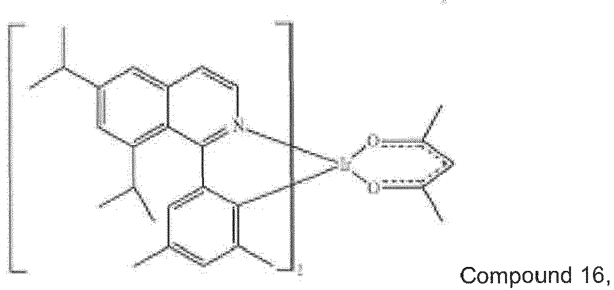 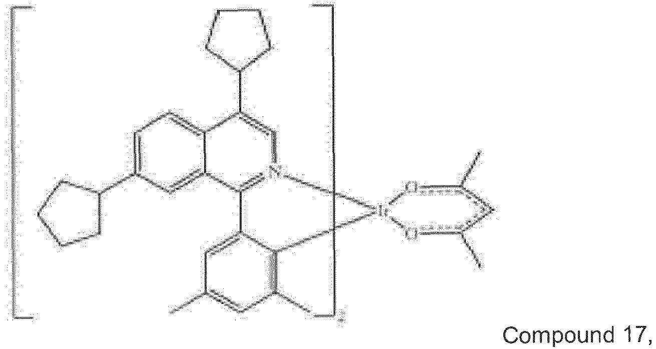 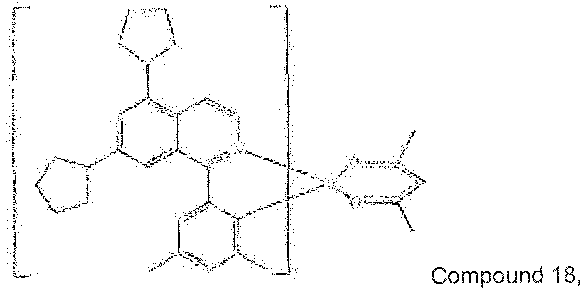 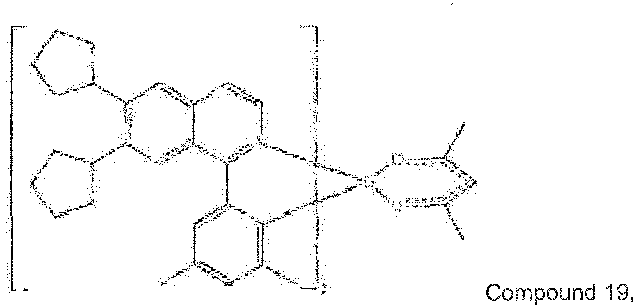 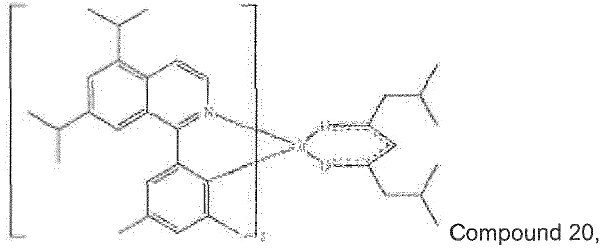 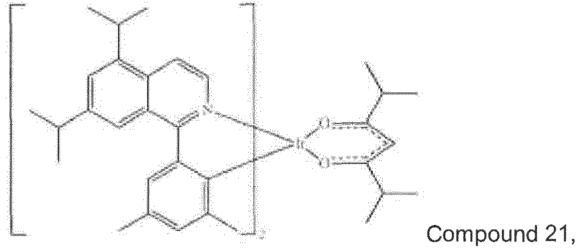 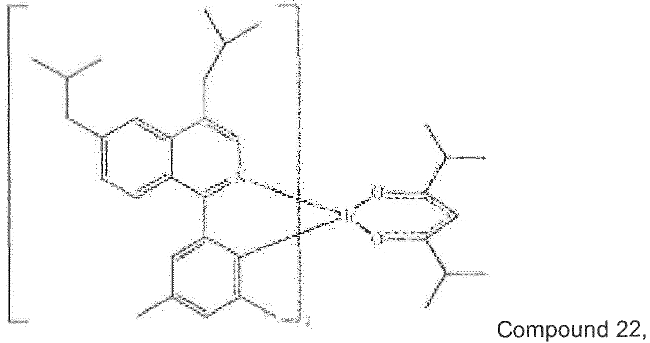 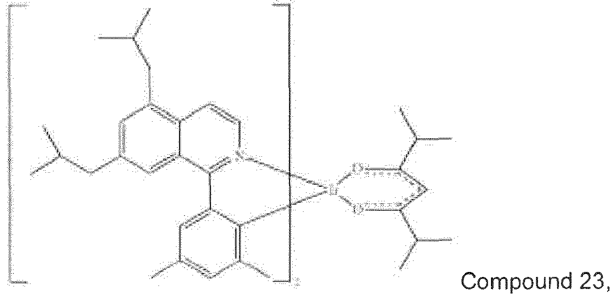 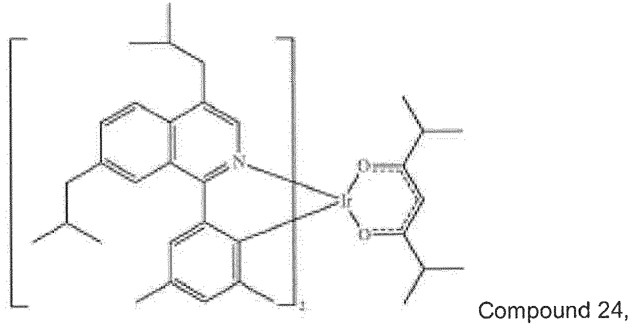 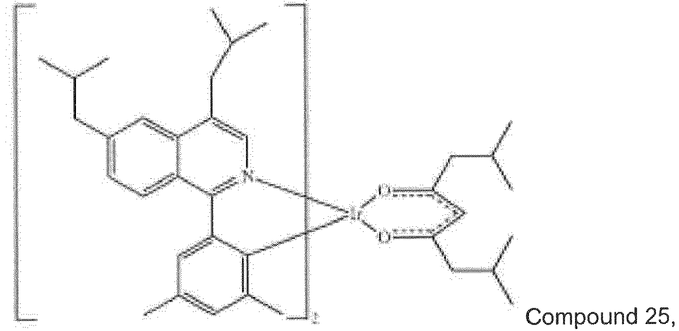 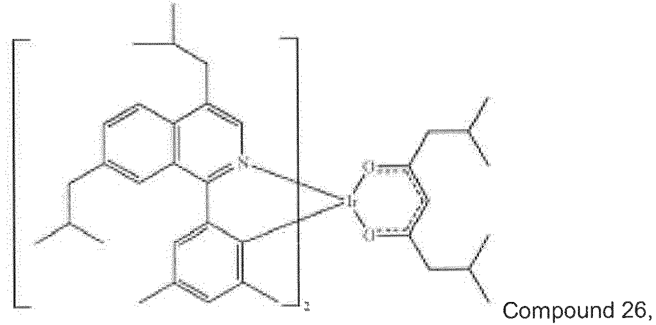 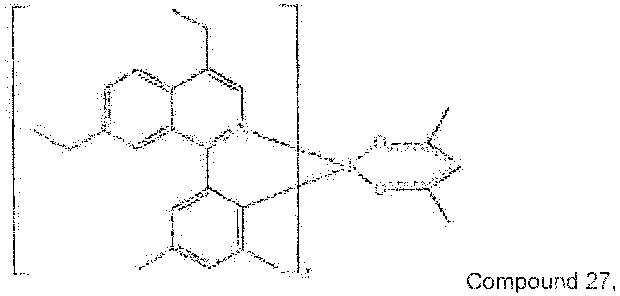 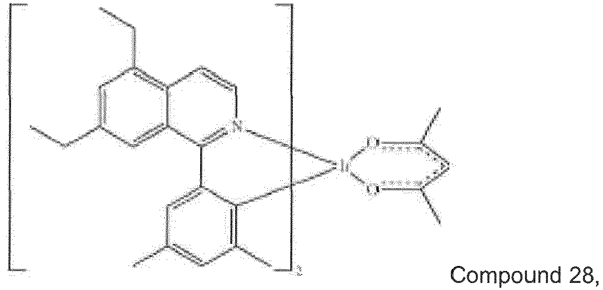 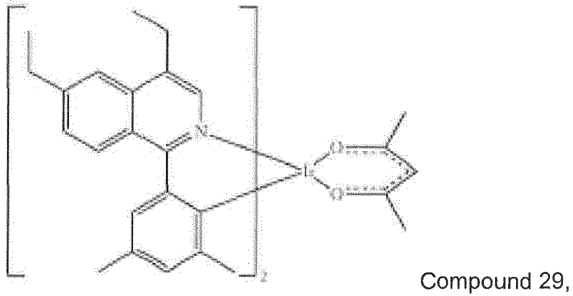 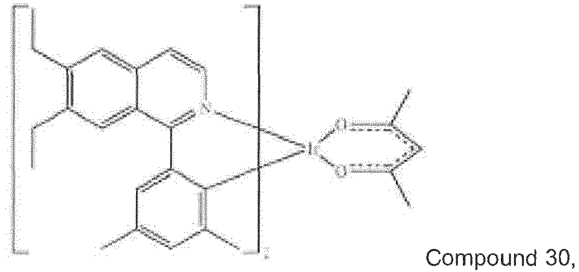 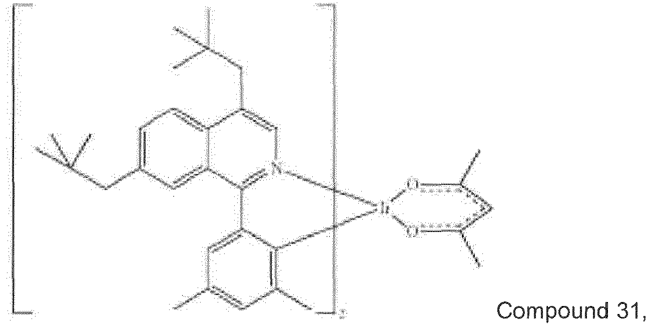 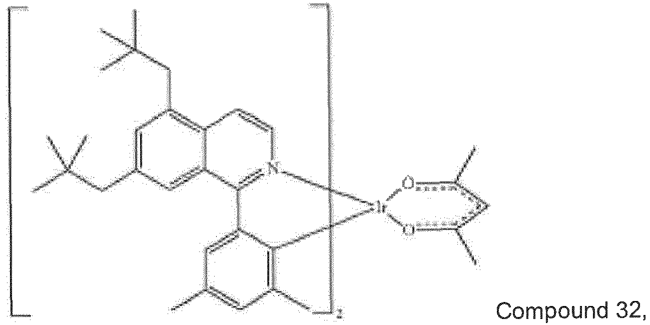 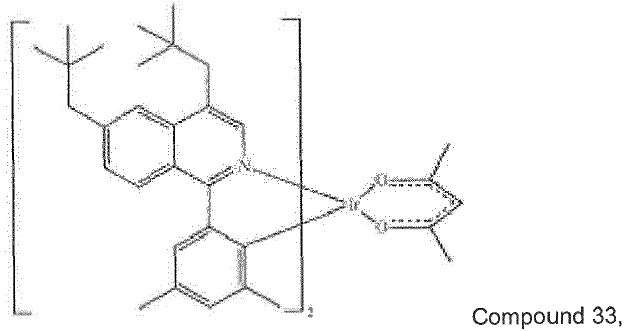 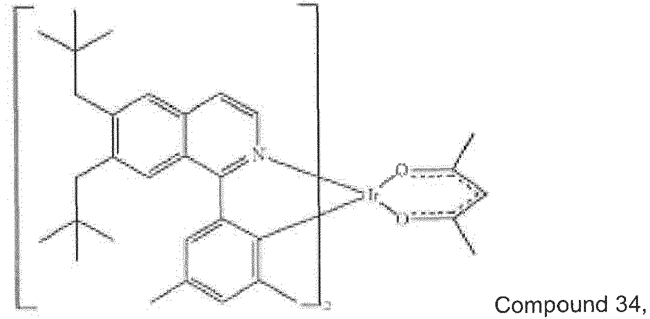 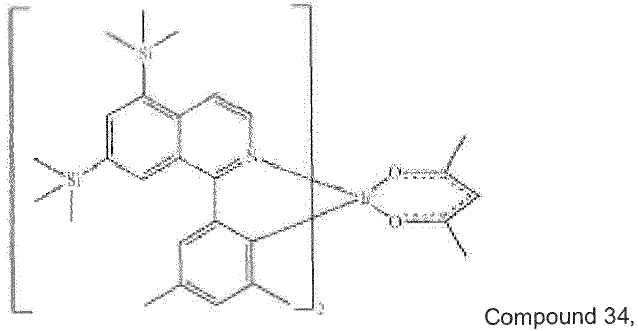 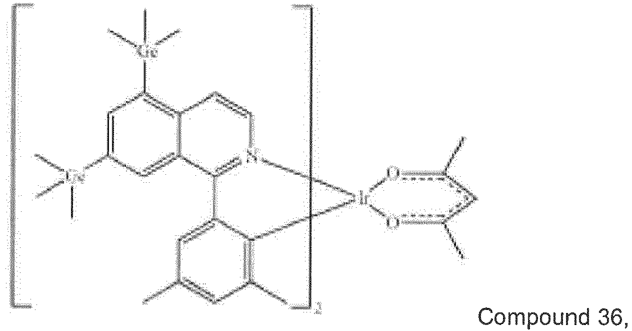 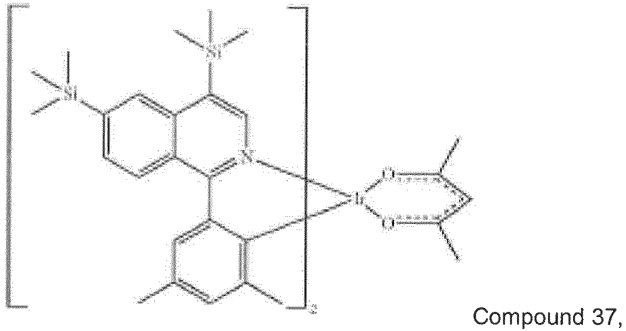 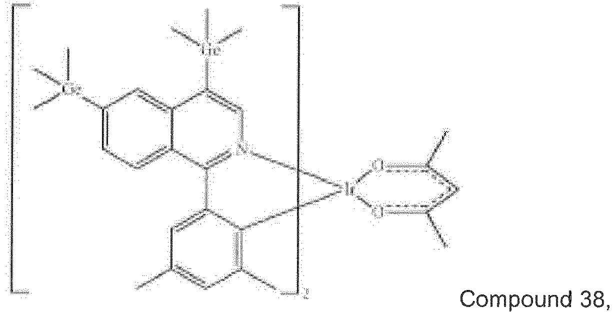 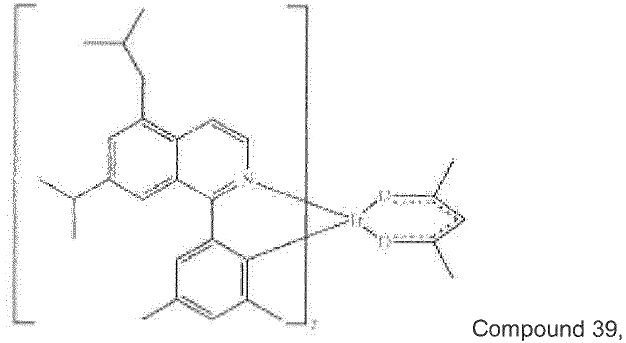 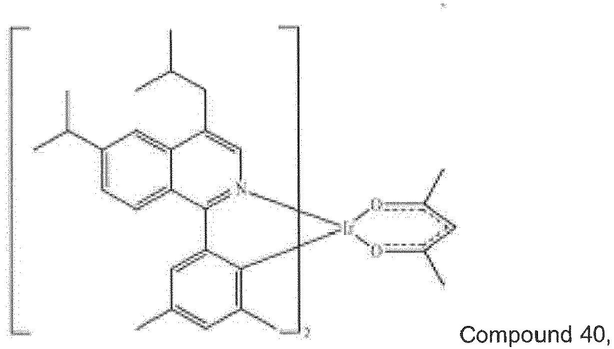 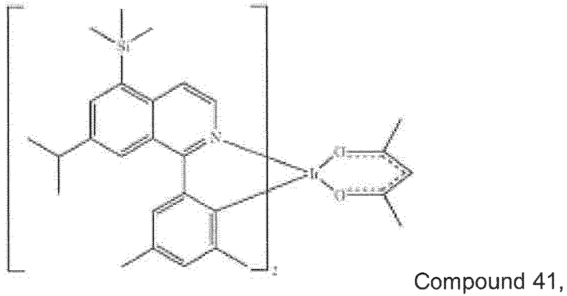 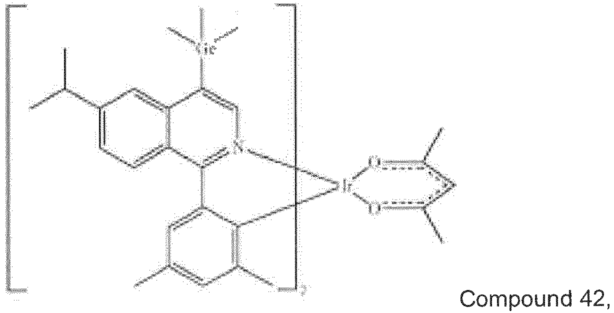 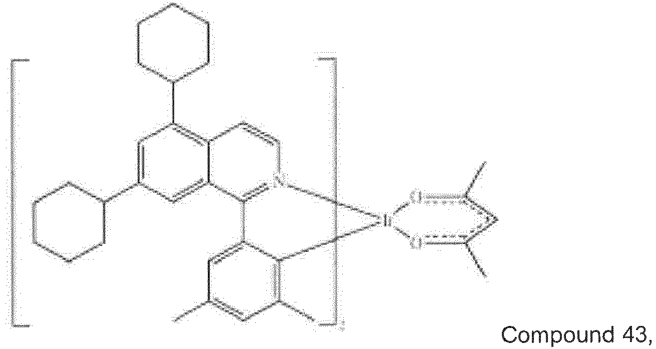 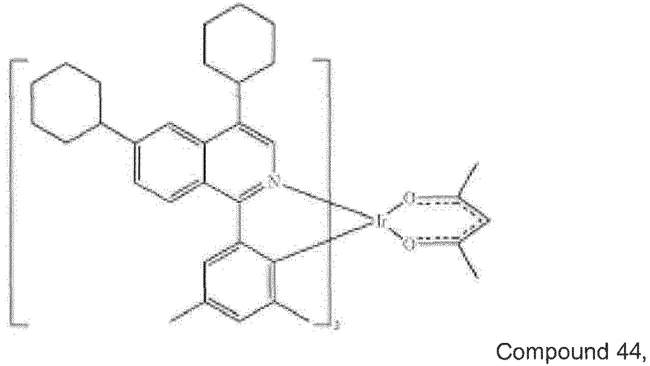 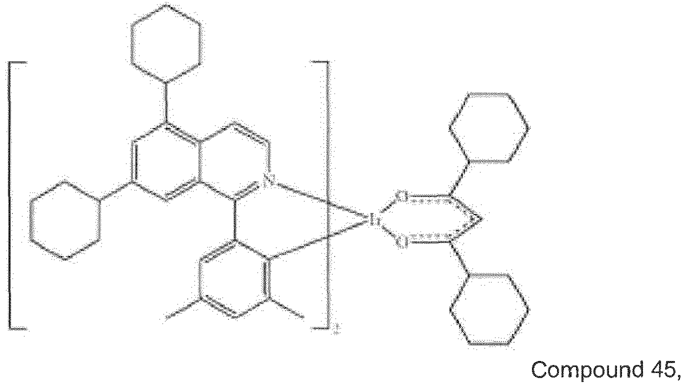 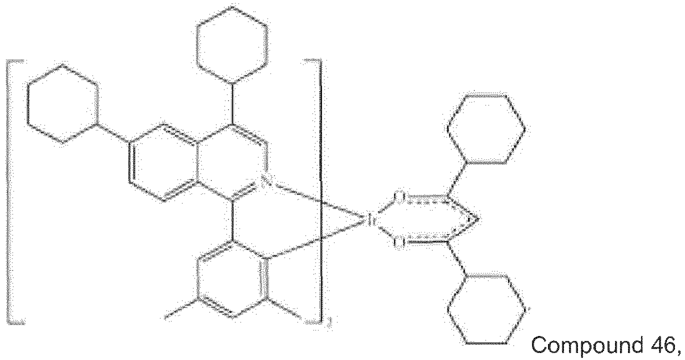 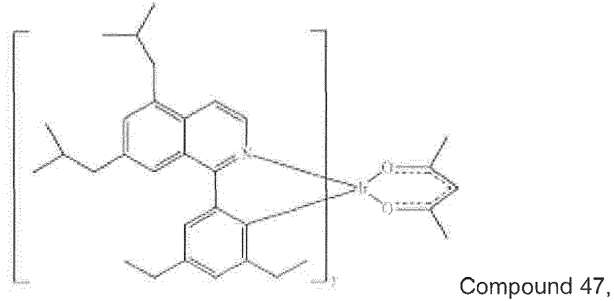 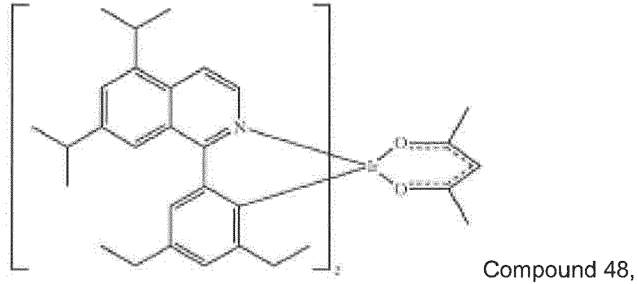 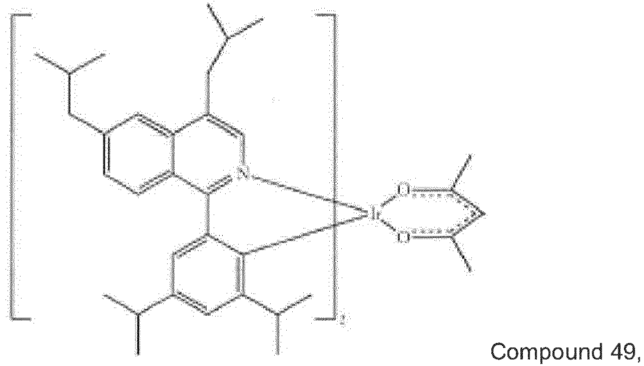 and

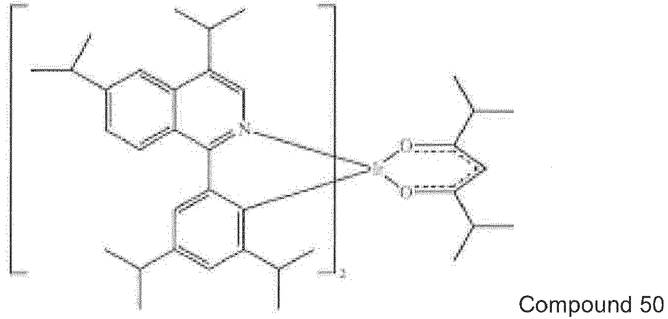

Further suitable red emitters are shown in US 2015/0001472. Preferred red emitters according to this document are the following compounds: 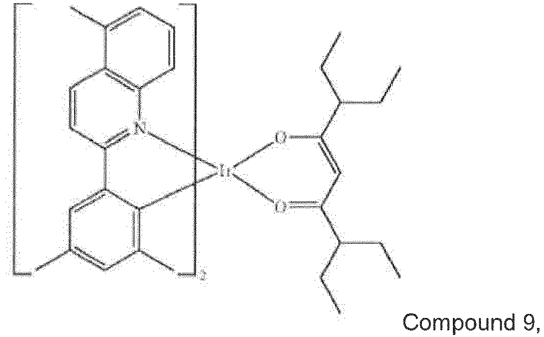 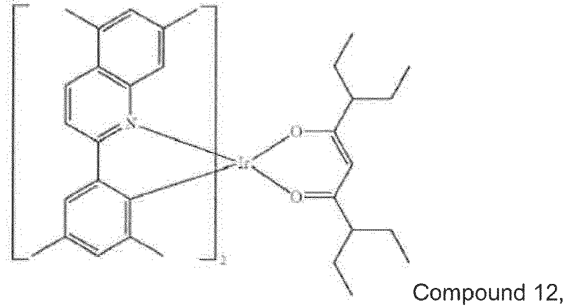 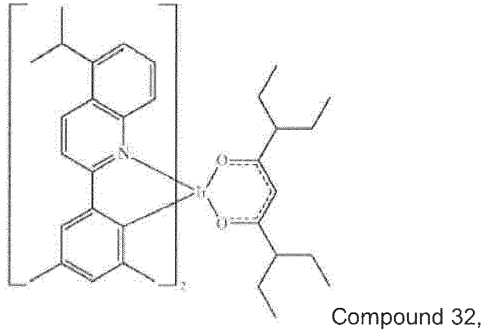 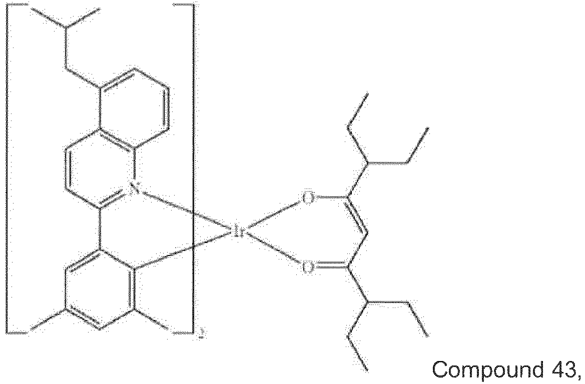 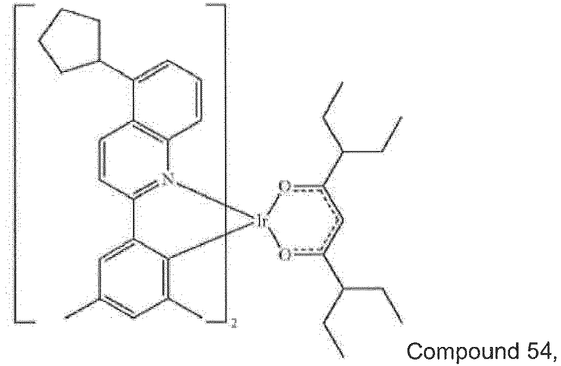 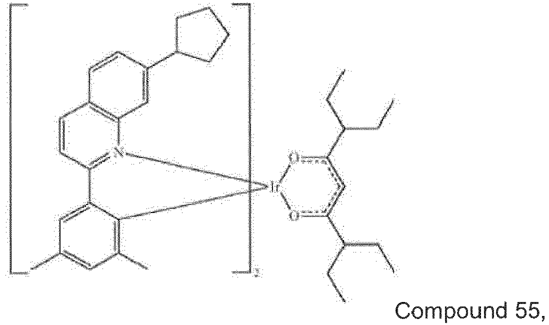 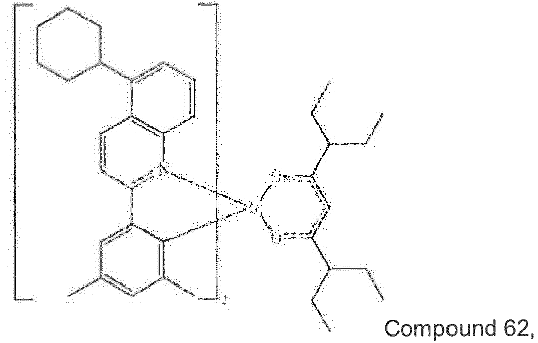 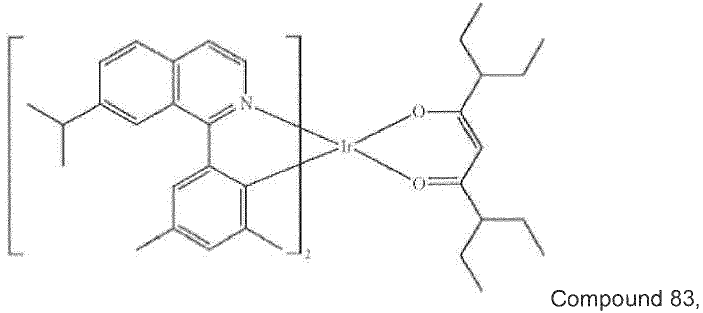 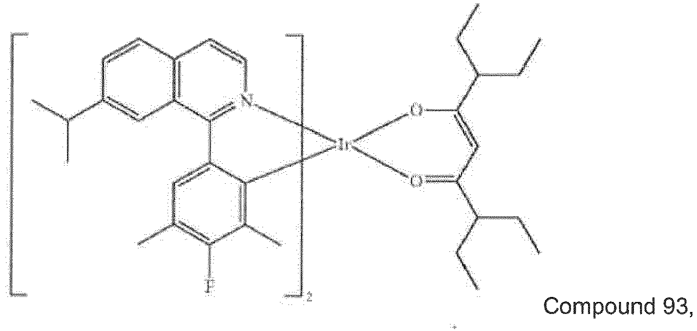 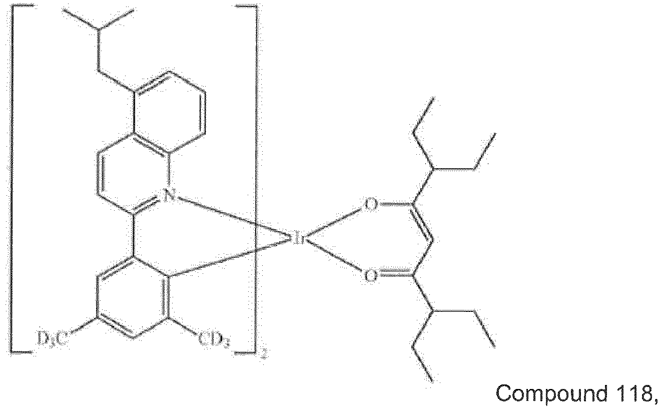 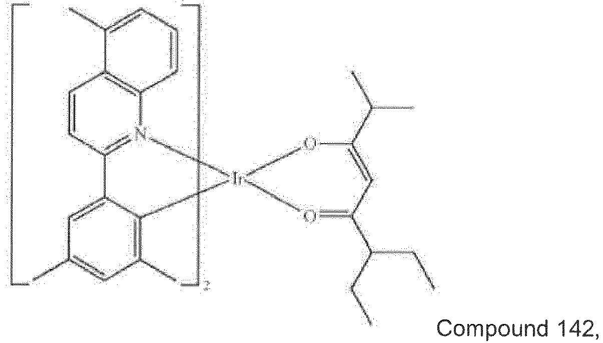 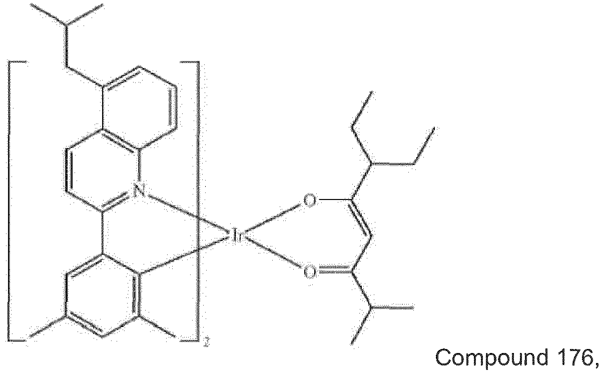 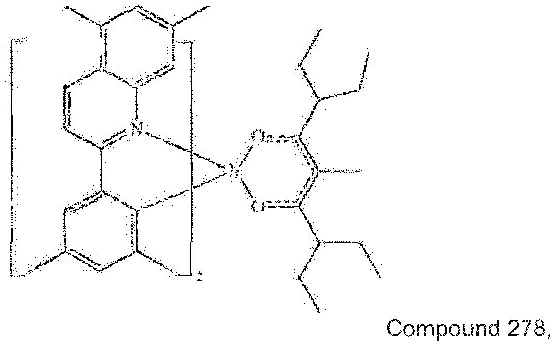 and

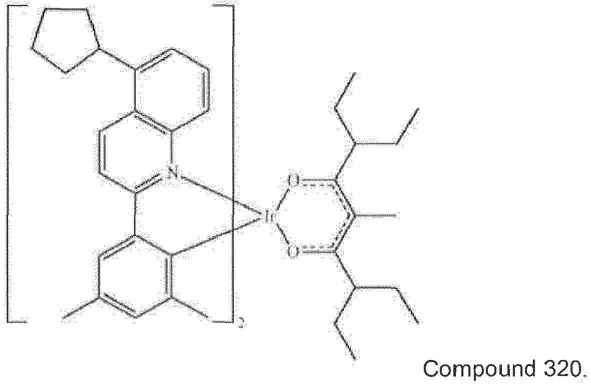

Further suitable red emitters are shown in US 2016/104848. Preferred red emitters according to this document are the following compounds: 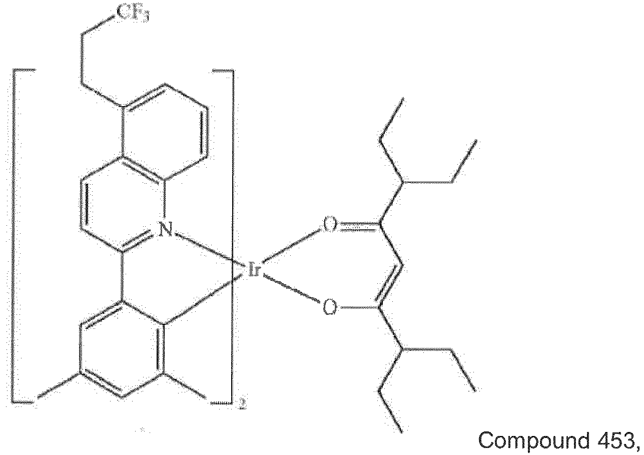 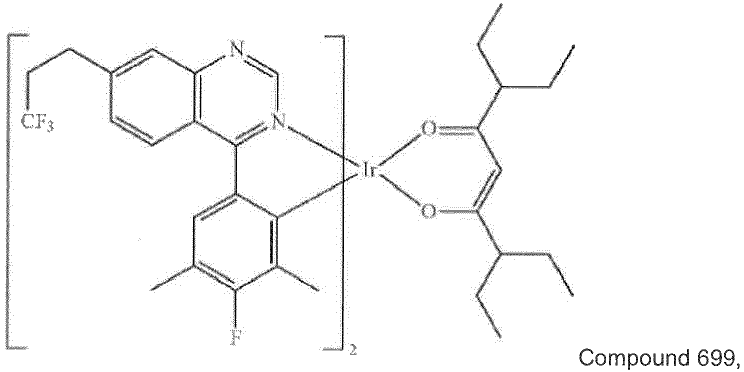 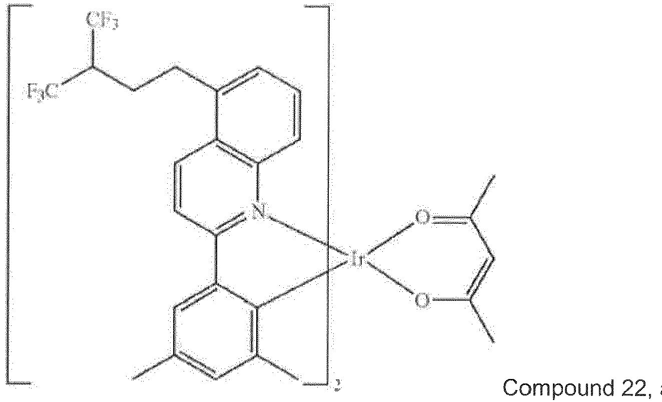 and

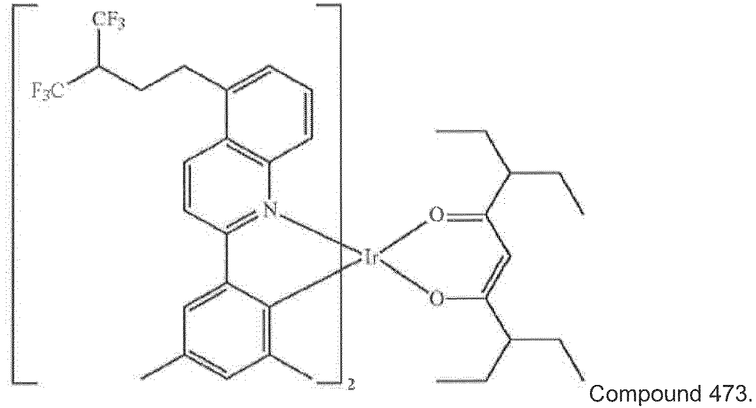

### Host (matrix) materials

The composition according to the present invention present in the light-emitting layer respectively the light-emitting layer comprising the compound of formula (XX) as described in the present application as dopant material and the compound of formula (I) as described in the present application as host material may comprise further components in addition to the at least one emitter material (XX) and the at least one compound of formula (I) according to the present invention. For example, a fluroescent dye may be present in the composition according to the present invention respectively in the light-emitting layer in order to alter the emission color of the emitter material. In addition a further matrix material can be used. This further matrix material may be a polymer, for example poly(N-vinylcarbazole) or polysilane. The further matrix material may, however, be a small molecule, for example 4,4'-N,N'-dicarbazolebiphenyl (CDP=CBP) or tertiary aromatic amines, for example TCTA.

The compound of formula (I) is preferably employed as phosphorescent host material. The phosphorescent host is a compound which confines the triplet energy of the phosphorescent dopant efficiently in the light emitting layer to cause the phosphorescent dopant to emit light efficiently.

In a preferred embodiment, the light-emitting layer is formed of the composition according to the present invention respectively of the compound of formula (XX) as described in the present application as dopant material and the compound of formula (I) as described in the present application as host material. According to a preferred embodiment, the electronic device according to the present invention, preferably the OLED according to the present invention, comprises the compound of formula (XX) as described in the present application as dopant material and the compound of formula (I) as described in the present application as host material, respectively the composition according to the present invention.

According to one embodiment, the light-emitting layer comprises the compound of formula (XX) as described in the present application and the compound of formula (I) as described in the present application, respectively the composition of the present invention comprising at least one emitter material (XX) and a compound according to general formula (I), wherein the compound of formula (I) is a single host material. Examples of preferred compounds of general formula (I) useful as single host material are shown above.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

According to one embodiment of the present invention, at least one compound according to general formula (I) may be present in the hole/exciton blocking layer.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAlq), phenothiazine *S,S*-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds **(SH-1), (SH-2), (SH-3), SH-4, SH-5, SH-6, (SH-7), (SH-8), (SH-9), (SH-10)** and **(SH-11)** may be used as hole/exciton blocking materials.

### Electron transport layer (g):

Electron transport layer may include a material capable of transporting electrons. Electron transport layer may be intrinsic (undoped), or doped. Doping may be used to enhance conductivity.

The compound according to general formula (I) according to the present invention is also suitable as electron transport material, either alone or in combination with one or more of the electron transport materials mentioned below. The compound according to general formula (I) according to the present invention is preferably suitable as electron transport material, if a blue fluorescent emitter is present in the emitting layer.

Further suitable electron-transporting materials for layer (g) of the inventive OLEDs, which may be used in combination with the compound of general formula (I) according to the present invention or in absence of the compound of general formula (I) according to the present invention as electron transport material, comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

Further suitable electron transport materials, which may be used in combination with the compound of general formula (I) according to the present invention or in absence of the compound of general formula (I) according to the present invention as electron transport material, are mentioned in Abhishek P. Kulkarni, Christopher J. Tonzola, Amit Babel, and Samson A. Jenekhe, Chem. Mater. 2004, 16, 4556-4573; G. Hughes, M. R. Bryce, J. Mater. Chem. 2005, 15, 94-107 and Yasuhiko Shirota and Hiroshi Kageyama, Chem. Rev. 2007, 107, 953-1010 *(ETM, HTM).*

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (XVI) below, preferably a compound of the formula (XVIa) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (formula XVII). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (XVII) 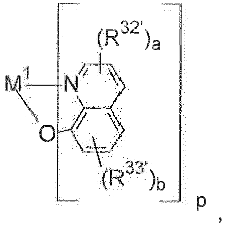 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (XVII) is 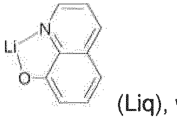 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (XVI), 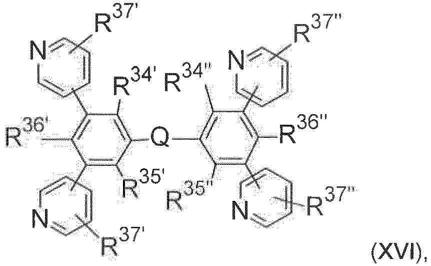 in which
R^{34"}, R^{35"}, R^{36"}, R^{37"}, R^{34'}, R^{35'}, R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-; E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which R^{38'}and R^{39'} are each independently H, C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₆-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula (XVI) are compounds of the formula (XVIa) 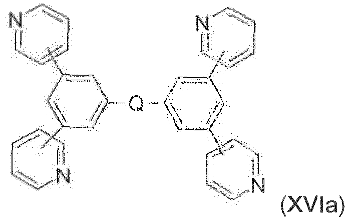
in which Q is: 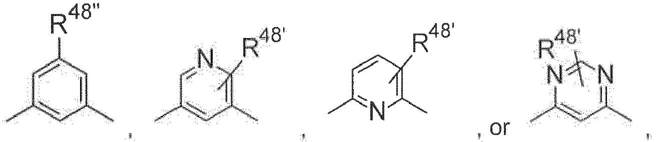
R^{48'} is H or C₁-C₁₈-alkyl and
R^{48"} is H, C₁-C₁₈-alkyl or

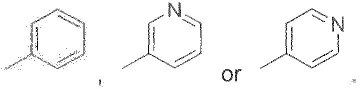

Particular preference is given to a compound of the formula

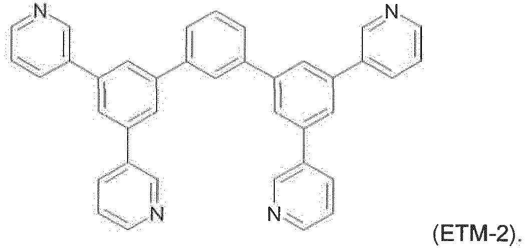

In a further, very particularly preferred embodiment, the electron-transport layer comprises a compound Liq and a compound **ETM-2.**

In a preferred embodiment, the electron-transport layer comprises at least one compound of the formula **(XVII)** in an amount of 99 to 1% by weight, preferably 75 to 25% by weight, more preferably about 50% by weight, and at least one compound of the formula **(XVI)** in an amount of 1 to 99% by weight, preferably 25 to 75% by weight, more preferably about 50% by weight, where the amount of the compounds of the formulae **(XVII)** and the amount of the compounds of the formulae **(XVI)** adds up to a total of 100% by weight.

The preparation of the compounds of the formula (XVI) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1** to **A-36** and **B-1** to **B-22** described in WO2011/157790 are preferred, wherein dibenzofuran compound 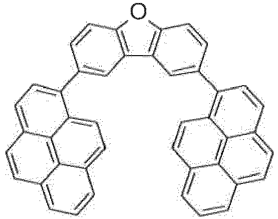 **(A-10;** = **ETM-1)** is most preferred.

In a preferred embodiment, the electron-transport layer comprises Liq in an amount of 99 to 1% by weight, preferably 75 to 25% by weight, more preferably about 50% by weight, and at least one dibenzofuran compound in an amount of 1 to 99% by weight, preferably 25 to 75% by weight, more preferably about 50% by weight, where the amount of Liq and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 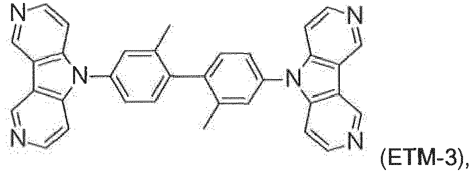 US2012/0261654 , such as, for example, a compound of formula 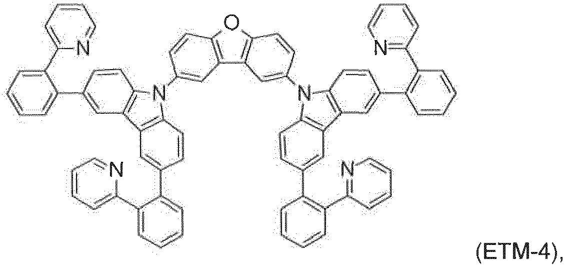 and WO2012/115034, such as for example, such as, for example, a compound of formula

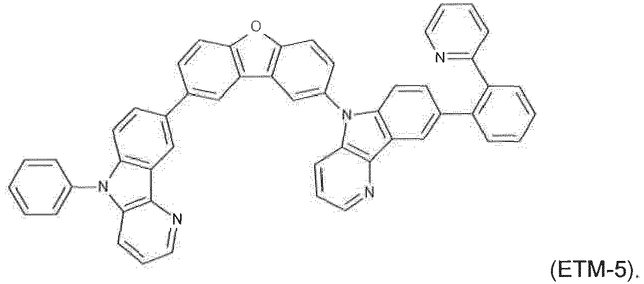

Further preferred embodiments of the electron injection layer of the OLED according to the present invention are mentioned in US 2013306955.

For example, the electron transporting material that may be present in the electron transporting layer of the OLED according to the present invention is an electron transporting material represented by formula (1):

A1(-L1 -L2-L3-L4-Ar1)m (1)

wherein:
each of L1, L2, L3, and L4 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar1 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A1 represents an m-valent residue of a ring-containing compound represented by formula (2); and
m represents an integer of 1 or more: 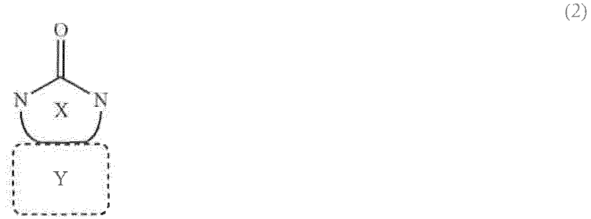
wherein:
ring X is a substituted or unsubstituted, saturated or unsaturated 5- to 8-membered ring having a ring nitrogen atom and a ring carbon atom;
the ring X may be fused to one or more rings Y; and
the ring Y represents a substituted or unsubstituted hydrocarbon ring or a substituted or unsubstituted heteroring;
The ring Y preferably represents a substituted or unsubstituted non-fused aromatic hydrocarbon ring having 6 to 30 ring carbon atoms, a substituted or unsubstituted fused aromatic hydrocarbon ring having 10 to 30 ring carbon atoms, a substituted or unsubstituted non-fused heteroring having 5 to 30 ring atoms, or a substituted or unsubstituted fused heteroring having 10 to 30 ring atoms.

The electron transporting material of the invention is preferably represented by formula (1-1) or (1-2) :

A11(-L11-L21-L31-L41-Ar11)p (1-1)

wherein;
each of L11, L21, L31, and L41 independently represents a single bond, a substituted or unsubstituted alkylene group having 1 to 50 carbon atoms, a substituted or unsubstituted alkenylene group having 1 to 50 carbon atoms, a substituted or unsubstituted alkynylene group having 1 to 50 carbon atoms, a substituted or unsubstituted cycloalkylene group having 3 to 50 ring carbon atoms, a substituted or unsubstituted arylene group having 6 to 30 ring carbon atoms, or a substituted or unsubstituted heteroarylene group having 5 to 30 ring atoms;
Ar11 represents a substituted or unsubstituted alkyl group having 1 to 50 carbon atoms, a substituted or unsubstituted cycloalkyl group having 3 to 50 ring carbon atoms, a substituted or unsubstituted aryl group having 6 to 30 ring carbon atoms, or a substituted or unsubstituted heteroaryl group having 5 to 30 ring atoms;
A11 represents a p-valent residue of a ring-containing compound represented by formula (2-1); and
p represents an integer of 1 or more: 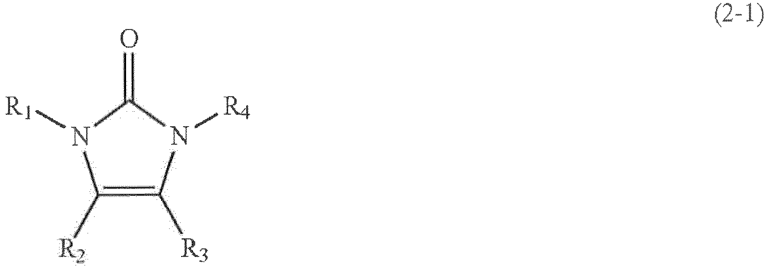
wherein;
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, a boryl group, a phosphino group, an amino group, a halogen atom, a cyano group, a nitro group, a hydroxyl group, a carboxyl group, or a valence bonded to L11; or a pair of R1 and R2, R2 and R3, or R3 and R4 are bonded to each other to form a ring Y represented by a substituted or unsubstituted hydrocarbon ring or a substituted or unsubstituted heteroring.

A11 of formula (1-1) preferably represents a p-valent residue of a compound represented by formula (2-1-1), (2-1-2), or (2-1-3): 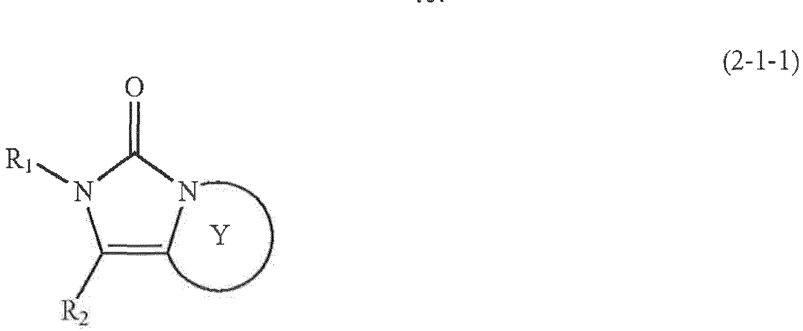 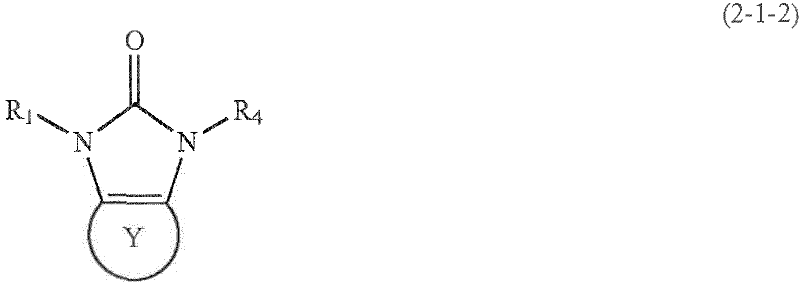 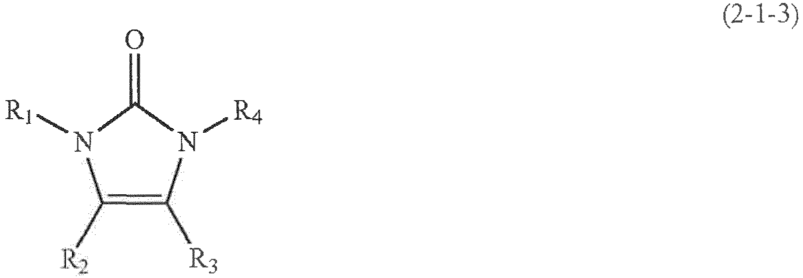 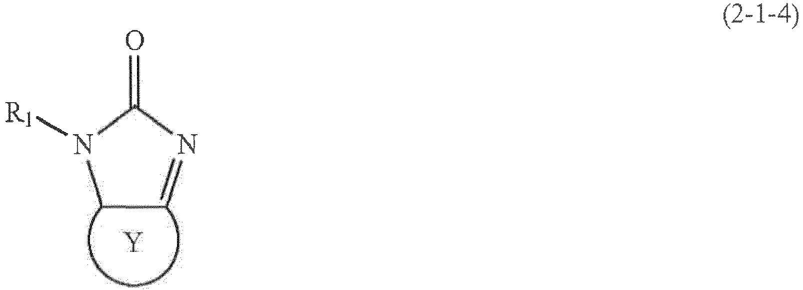 wherein:
each of R1 to R4 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted arylthio group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, an amino group substituted by a substituted or unsubstituted alkyl group having 1 to 50 carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; and
Y represents the ring Y.

Further, A11 preferably represents a p-valent residue of a compound represented by formula (2-1-2-1): 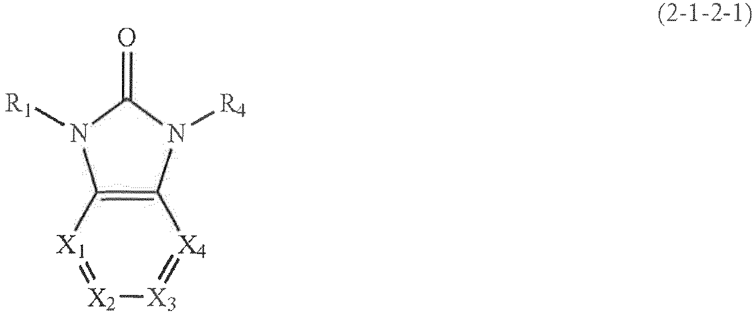 wherein:
each of X1 to X4 independently represents CR5 or N;
each of R1, R4, and R5 independently represents a hydrogen atom, a substituted or unsubstituted alkyl group having 1 to 50 carbon atoms, a substituted or unsubstituted alkoxy group having 1 to 50 carbon atoms, a substituted or unsubstituted alkoxycarbonyl group having 1 to 50 carbon atoms, a substituted or unsubstituted cycloalkyl group having 3 to 50 ring carbon atoms, a substituted or unsubstituted aryl group having 6 to 30 ring carbon atoms, a substituted or unsubstituted aralkyl group having 6 to 50 ring carbon atoms, a substituted or unsubstituted aryloxy group having 6 to 50 ring carbon atoms, a substituted or unsubstituted heteroaryl group having 5 to 30 ring atoms, a substituted or unsubstituted alkylsilyl group having 1 to 50 carbon atoms, a substituted or unsubstituted arylsilyl group having 6 to 50 ring carbon atoms, a substituted or unsubstituted haloalkyl group having 1 to 50 carbon atoms, a substituted or unsubstituted alkenyl group having 1 to 50 carbon atoms, a substituted or unsubstituted alkynyl group having 1 to 50 carbon atoms, a substituted or unsubstituted alkylamino group having 1 to 50 carbon atoms, a substituted or unsubstituted arylamino group having 6 to 30 ring carbon atoms, a substituted or unsubstituted heteroarylamino group having 5 to 30 ring atoms, a substituted or unsubstituted acylamino group having 2 to 50 carbon atoms, a substituted or unsubstituted heteroaryloxy group having 5 to 30 ring atoms, a substituted or unsubstituted acyl group having 2 to 50 carbon atoms, a substituted or unsubstituted aryloxycarbonyl group having 6 to 30 ring carbon atoms, a substituted or unsubstituted alkylcarbonyl group having 1 to 50 carbon atoms, a mercapto group, a substituted or unsubstituted alkylthio group having 1 to 50 carbon atoms, a substituted or unsubstituted arylthio group having 6 to 30 ring carbon atoms, a sulfonyl group, an boryl group, a phosphino group, an amino group, a halogen atom, a cyano group, a nitro group, a hydroxyl group, a carboxyl group, or a valence bonded to L11; or R1, R4, and R5 are each bonded to each other to form a ring which forms a part of the ring Y.

In particular, the electron transporting layer of the OLED according to the present invention, between the light emitting layer and the cathode, preferably comprises at least one compound of the general formula (I).

In a preferred embodiment, the electron transporting layer comprising at least one compound of the general fomrula (I) further comprises a reducing dopant.

Examples of the reducing dopant include a donating metal, a donating metal compound, and a donating metal complex. The reducing dopant may be used alone or in combination of two or more.

The reducing dopant referred to herein is an electron-donating material. The electron-donating material is a material which generates radical anions by the interaction with a coexisting organic material in the electron transporting layer or an organic material in a layer adjacent to the electron transporting layer, or a material having an electron-donating radical.

The donating metal is a metal having a work function of 3.8 eV or less, preferably an alkali metal, an alkaline earth metal, or a rare earth metal, and more preferably Cs, Li, Na, Sr, K, Mg, Ca, Ba, Yb, Eu, or Ce.

The donating metal compound is a compound comprising the above donating metal, preferably a compound comprising an alkali metal, an alkaline earth metal, or a rare earth metal, and more preferably a halide, an oxide, a carbonate, or a borate of these metals, for example, a compound represented by MOₓ (M: donating metal, x: 0.5 to 1.5), MFₓ (x: 1 to 3), or M(CO₃)ₓ (x: 0.5 to 1.5).

The donating metal complex is a complex comprising the above donating metal, preferably an organic metal complex of an alkali metal, an alkaline earth metal or a rare earth metal, and more preferably an organic metal complex represented by formula (I):

MQₙ (I)

wherein M is a donating metal, Q is a ligand, preferably a carboxylic acid derivative, a diketone derivative, or a quinoline derivative, and n is an integer of 1 to 4.

Examples of the donating metal complex include watermill-shaped tungsten compounds described in JP 2005-72012A and phthalocyanine compounds having an alkali metal or an alkaline earth metal as the central metal, which are described in JP 11-345687A.

The reducing dopant is preferably at least one selected from the group consisting of an alkali metal, an alkaline earth metal, a rare earth metal, an alkali metal oxide, an alkali metal halide, an alkaline earth metal oxide, an alkaline earth metal halide, a rare earth metal oxide, a rare earth metal halide, an organic complex having an alkali metal, an organic complex having an alkaline earth metal, and an organic complex having a rare earth metal, and more preferably a 8-quinolinol complex of an alkali metal.

Examples of the alkali metal includes:
Li (lithium, work function: 2.93 eV),
Na (sodium, work function: 2.36 eV),
K (potassium, work function: 2.3 eV),
Rb (rubidium, work function: 2.16 eV), and
Cs (cesium, work function: 1.95 eV).

The values of work functions are based on Handbook of Chemistry (Pure Chemistry II, 1984, p. 493, edited by The Chemical Society of Japan). The same applies hereafter
Preferred examples of the alkaline earth metals are:
Ca (calcium, work function: 2.9 eV),
Mg (magnesium, work function: 3.66 eV),
Ba (barium, work function: 2.52 eV), and
Sr (strontium, work function: 2.0 to 2.5 eV).

The work function of strontium is based of Physics of Semiconductor Device (N.Y., Wiley, 1969, p. 366).

Preferred examples of the rare earth metal are:
Yb (ytterbium, work function: 2.6 eV),
Eu (europium, work function: 2.5 eV),
Gd (gadolinium, work function: 3.1 eV), and
Er (erbium, work function: 2.5 eV).

Examples of the alkali metal oxide include Li₂O, LiO, and NaO. The alkaline earth metal oxide is preferably CaO, BaO, SrO, BeO, or MgO.

Examples of the alkali metal halide include a fluoride, for example, LiF, NaF, CsF, and KF and a chloride, for example, LiCI, KCI, and NaCl.

The alkaline earth metal halide is preferably a fluoride, such as CaF₂, BaF₂, SrF₂, MgF₂, and BeF₂ and a halide other than fluoride.

An OLED wherein at least one compound according to general formula (I) used in the electron transporting layer is particularly preferred because the driving voltage is reduced while increasing the efficiency.

The content of the at least one compound according to general formula (I) in the electron transporting layer is preferably 50% by mass or more and more preferably 60% by mass or more.

The electron transporting layer facilitates the injection of electrons into the light emitting layer and transports the electrons to the light emitting zone, and has a large electron mobility and an electron affinity generally as large as 2.5 eV or more. The electron transporting layer is preferably formed from a material capable of transporting electrons to the light emitting layer at a lower strength of electric field, preferably having an electron mobility of, for example, at least 10⁻⁶ cm²/V·s under an electric field of 10⁴ to 10⁶ V/cm.

The material for forming the electron injecting/transporting layer in combination with the compound according to general formula (I) is not particularly limited as long as having the preferred properties mentioned above and may be selected from those commonly used as the electron transporting material in the field of photoconductive materials and those known as the materials for the electron injecting/transporting layer of organic EL devices.

In the present invention, an electron injecting layer including an insulating material or a semiconductor may be disposed between the cathode and the organic layer. By such an electron injecting layer, the leak of electric current is effectively prevented to improve the electron injecting ability. Preferred examples of the insulating material include at least one metal compound selected from the group consisting of an alkali metal chalcogenide, an alkaline earth metal chalcogenide, an alkali metal halide, and an alkaline earth metal halide. An electron injecting layer including the above alkali metal chalcogenide is preferred because the electron injecting property is further improved. Preferred alkali metal chalcogenides include Li₂O, K₂O, Na₂S, Na₂Se, and Na₂O; preferred alkaline earth metal chalcogenides include CaO, BaO, SrO, BeO, BaS, and CaSe; preferred alkali metal halides include LiF, NaF, KF, LiCI, KCI, and NaCl; and preferred alkaline earth metal halides include fluoride such as CaF₂, BaF₂, SrF₂, MgF₂, and BeF₂ and halides other than fluoride.

Examples of the semiconductor for the electron transporting layer include an oxide, a nitride and an oxynitride of at least one element selected from Ba, Ca, Sr, Yb, Al, Ga, In, Li, Na, Cd, Mg, Si, Ta, Sb, and Zn, which are used singly or in combination of two or more. It is preferred that the inorganic compound constituting the electron transporting layer forms a microcrystalline or amorphous insulating thin film. When constituted of the insulating thin film described above, the electron injecting layer is made more uniform to reduce the pixel defect such as dark spots. Examples of such a inorganic compound include the alkali metal chalcogenide, the alkaline earth metal chalcogenide, the alkali metal halide and the alkaline earth metal halide which are described above.

### Electron injection layer (h):

The electron injection layer may be any layer that improves the injection of electrons into an adjacent organic layer.

Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds according to general formula (I) in at least one layer of the OLED, preferably in the light-emitting layer, preferably as a host material, a charge transporting material, particularly preferably as a host material and hole or electron transporting material, makes it possible to obtain OLEDs with high efficiency and with low use and operating voltage. Frequently, the OLEDs obtained by the use of the compounds according to general formula (I) additionally have high lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Compounds synthesized

### Compound 1

### Intermediate 1-1

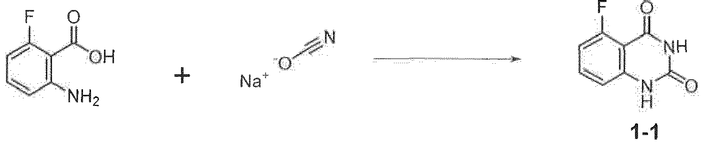

2-Amino-6-fluorobenzoic acid (35 g, 225.6 mmol) was dissolved in 885 ml of water and 26 mL of acetic acid. The mixture was stirred at 35 °C for 15 min. After that, sodium cyanate (36.67 g, 564 mmol) dissolved in 442 mL of water was added dropwise to the suspension, and the mixture was stirred at 35 °C for 30 min. Then, sodium hydroxide (180.49 g, 4.51 mol) was slowly added to the reaction mixture, the mixture is cooled at room temperature. After 374 mL of hydrogen chloride was added there, the precipitate was collected by filtration and washed with water. The solid was dried in a vacuum oven to yield 37.19 (92%) of 1-1 as white solid.
LC-MS: 179 [M+H]

### Intermediate 1-2

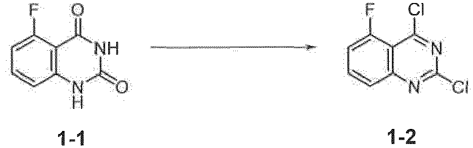

1-1 (2.6 g, 14.43 mmol) was suspended in 29 mL of toluene and heated to 50 °C. phosphoryl chloride (9.88 mL, 108.25 mmol) was added dropwise, and then DBU (4.31 mL, 28.87 mmol) was added dropwise. The mixture was stirred vigorously at 120 °C for overnight. After the reaction mixture was cooled at room temperature, it was added dropwise to ice-water. The aqueous layer was extracted with ethyl acetate (AcOEt). After it was washed with brine and dried with Na₂SO₄, it was concentrated to give a solid. The crude product was purified by column chromatography on silica gel eluting with toluene to yield 3.41 g (80.8%) of 1-2 as a white powder.
LC-MS: 217 [M+H]

### Intermediate 1-3

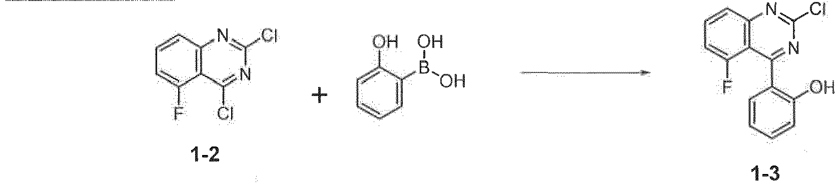

1-2 (2.17 g, 10.0 mmol) and 2-hydroxybenzene boronic acid (1.38 g, 10.0 mmol) were dissolved in 10 mL of THF. To the solution was added potassium fluoride (1.74 g, 30.0 mmol) dissolved in 5 mL of water, and the mixture was evacuated and purged with Argon gas. Then, ^{t}Bu₃P-HBF₄ (290 mg, 1.00 mmol) and Pd(OAc)₂ (225 mg, 1.00 mmol) were added to the mixture, and the mixture was stirred at room temperature for 1.5 h. The reaction mixture was dried with MgSO₄, filtrated over Celite and washed with ethyl acetate. The crude was purified by column chromatography on silica gel eluting with a mixed solvent of heptane and AcOEt to yield 2.27 g (96%) of 1-3 as a slightly yellow solid.
LC-MS : 275 [M+H]

### Intermediate 1-4

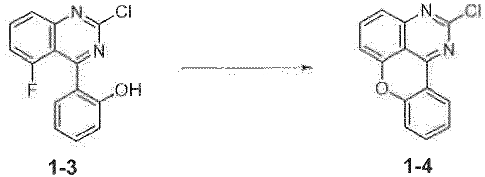

1-3 (3.02 g, 11 mmol) was dissolved in 55 mL of DMF and potassium carbonate (1.67 g, 12.1 mmol) was added. The mixture was stirred at room temperature for 5 min. The reaction mixture was diluted with 100 mL of water, and the solid was collected by filtration and washed with water. It was dried in vacuum oven to yield 2.60 g of 1-4 as a yellow powder.
LC-MS: 255 [M+1]

### Compound 1

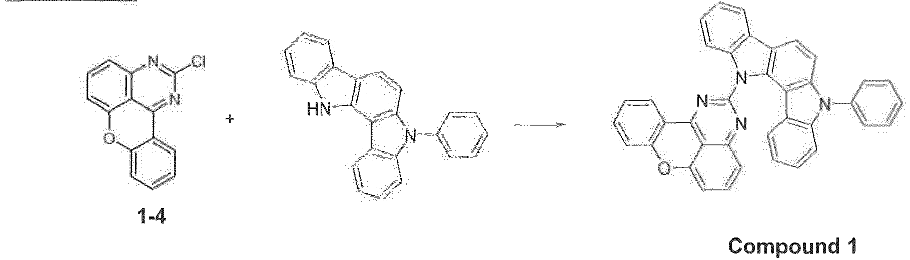

1-4 (1.02 g, 4.00 mmol), 5,12-dihydro-5-phenylindolo[3,2-a]carbabole (1.21 g, 3.63 mmol) which prepared according to the protocol mentioned in a patent application (US2015243893), and sodium *tert*-butoxide (489 mg, 5.09 mmol) were added to 24 mL of Toluene. The mixture was evacuated and purged with argon gas three times. Then, ^{t}Bu₃P-HBF₄ (93 mg, 0.32 mmol) and Pd₂(dba)₃ (67 mg, 0.07 mmol) were added to the mixture, and the reaction mixture was stirred at 100°C for 15 h. The precipitate was collected by filtration, and washed with toluene, ethanol (EtOH), and water. It was purified by chromatography eluting with heptane and toluene to yield 1.46 g (73%) of Compound 1 as a yellow powder.
LC-MS: 551 [M+H]

### Compound 2

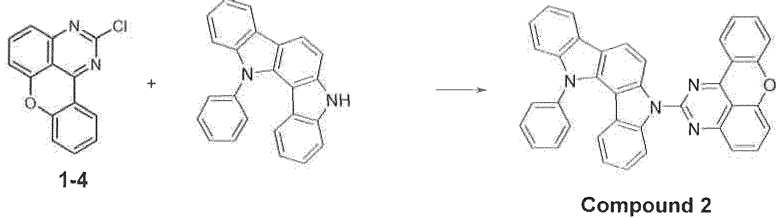

1-4 (1.375 g, 5.40 mmol), 5,12-Dihydro-12-phenylindolo[3,2-a]carbazole (1.50 g, 4.5 mmol) which prepared according to the protocol mentioned in a patent application (WO2016099204), and sodium *tert*-butoxide (1.30 mg, 13.5 mmol) were added to 30 mL of toluene. The mixture was evacuated and purged with argon gas three times. Then, xantphos (156 mg, 0.27 mmol) and Pd₂(dba)₃ (124 mg, 0.14 mmol) were added to the mixture, and the reaction mixture was stirred at 110°C for 5 h. The precipitate was collected by filtration, and washed with toluene, EtOH, and water. It was purified by chromatography eluting with heptane and toluene to yield 1.85 g (75%) of Compound 2 as a yellow powder.
LC-MS: 551 [M+H]

### Compound 3

### Intermediate 3-1

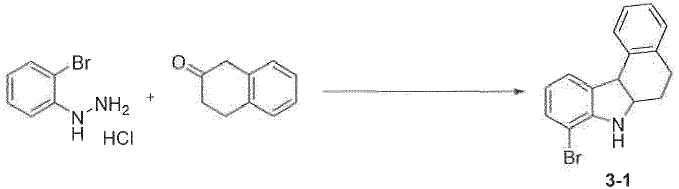

(2-Bromophenyl)hydrazine HCl salt (7.48 g, 40.0 mmol) was suspended in water (20 mL) and HCI (21 mL, 4 M in dioxane) in a 100 mL three-necked round-bottom flask. The flask was fitted with a reflux condenser and heated to 85 °C, where 2-tetralone (5.55 ml, 42.0 mmol) was added dropwise over 10 min. The reaction was refluxed for 16 h. Then, the reaction was allowed to cool to room temperature and neutralized with 30% aqueous NaOH solution. The layers were transferred to a 250 mL separation funnel, separated and the aqueous layer was extracted with AcOEt. The combined organic layers were dried with Na₂SO₄, filtered and concentrated. The residue was purified via flash chromatography with AcOEt and heptane to yield 6.37 g (54%) of 3-1 as an off-white solid.
LC-MS:296 [M-H]

### Intermediate 3-2

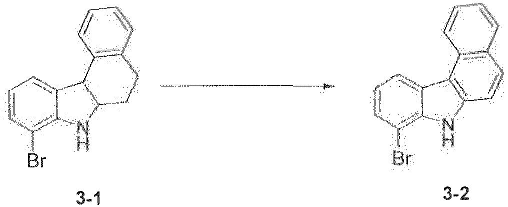

To a 250 mL three-necked round-bottom flask were added 3-1 (6.3 g, 21.1 mmol), *p*-chloranil (5.45 g, 22.2 mmol) and degassed xylenes (75 ml). The reaction was fitted with a reflux condenser, placed under Ar and heated to 140 °C for 16 h. Then, the reaction was allowed to cool to room temperature and the solid was filtered off. The resulting filtrate was concentrated, and then purified via flash chromatography with a mixed solvent of AcOEt and heptane to yield 5.9 g (95%) of 3-2 as a gray solid.
LC-MS: 294 [M-H]

### Intermediate 3-3

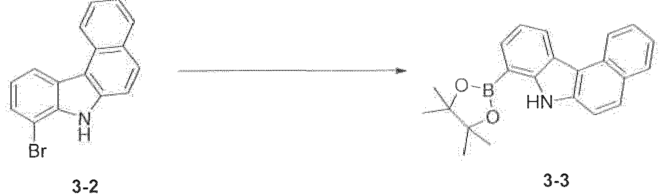

To a dried 250 mL three-necked round-bottom flask were added 3-2 (12 g, 40.5 mmol), bis(pinacolato)diboron (13.38 g, 52.7 mmol), potassium acetate (7.95 g, 81 mmol) and Pd(dppf)Cl₂ (1.20 g, 1.621 mmol). The reaction was evacuated and backfilled with Ar, then degassed dioxane (101 ml) was added. The reaction was gently refluxed at 100 °C for 15 h. Then, the reaction was allowed to cool down to room temperature, then filtered over a pad of silica/celite. The filtrate was concentrated, and then purified via flash chromatography with AcOEt and heptane to yield 11 g (79%) of 3-3 as an off-white solid. 3-3 was used for the next reaction without further purification.

### Intermediate 3-4

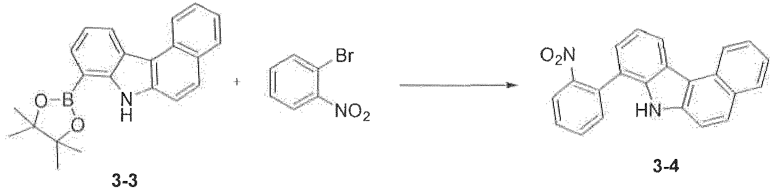

To a 250 mL three-necked round-bottom flask were added 3-3 (10.91 g, 31.8 mmol), 1-bromo-2-nitrobenzene (7.71 g, 38.1 mmol), NaOH (3.81 g, 95 mmol) and Pd(PPh₃)₄ (1.102 g, 0.954 mmol). The flask was purged with N₂ for 5 min, and then THF (70 ml) and water (35 ml) were added. The reaction was fitted with a reflux condenser and gently refluxed for 16 h. Then, the reaction was allowed to cool down to room temperature and transferred to a 500 mL separation funnel. The *aqueous* layer was extracted with AcOEt. The combined organic layers were dried with Na₂SO₄, filtered and concentrated. The residue was purified via flash chromatography with AcOEt and heptane to yield 9.36 g (87%) of 3-4 as an off-white solid.
LC-MS: 337 [M-H]

### Intermediate 3-5

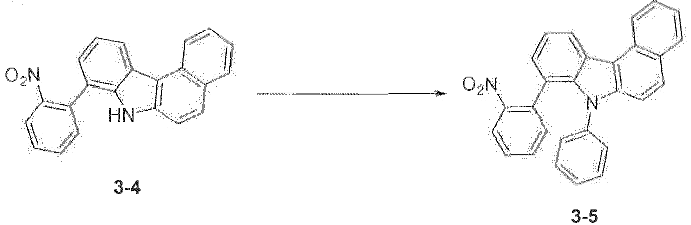

To a dried 250 mL three-necked round-bottom flask were added 3-4 (9 g, 26.6 mmol), potassium carbonate (11.03 g, 80 mmol) and copper (1.69 g, 26.6 mmol). The flask was fitted with a reflux condenser and placed under N₂. Nitrobenzene (27 ml) and iodobenzene (7.5 mL, 66.9 mmol) were added and the reaction was heated to 190 °C for three days. Then, the reaction was allowed to cool down to room temperature, diluted with AcOEt and transferred to a 500 mL separation funnel. The organic layer was thoroughly washed with a 1% solution of 2-aminopropanol in water. The organic layer was dried with Na₂SO₄, filtered and concentrated. The resulting solid was dried, and then recrystallized from toluene. After filtration, 6.93 g (63%) of 3-5 was obtained as a yellow solid.
LC-MS: 415 [M+H]

### Intermediate 3-6

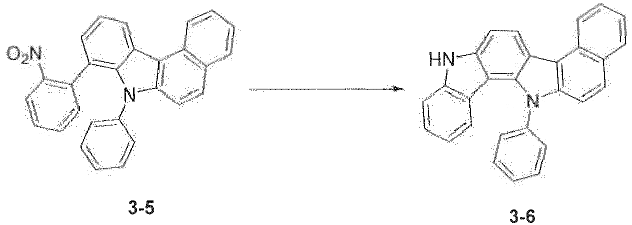

To a dried 100 mL three-necked round-bottom flask were added **3-5** (6.9 g, 16.65 mmol) and triphenylphosphine (21.83 g, 83 mmol). The reaction was placed under N₂, and heated at 200 °C for 16 h. Then, the reaction was allowed to cool down to room temperature, diluted with heptane and filtered. The resulting solid was washed several times with refluxing MeOH, and then further purified by a hot filtration with toluene and chlorobenzene. 5 g (79%) of 3-6 was obtained as a gray solid.
LC-MS: 383 [M+H]

### Compound 3

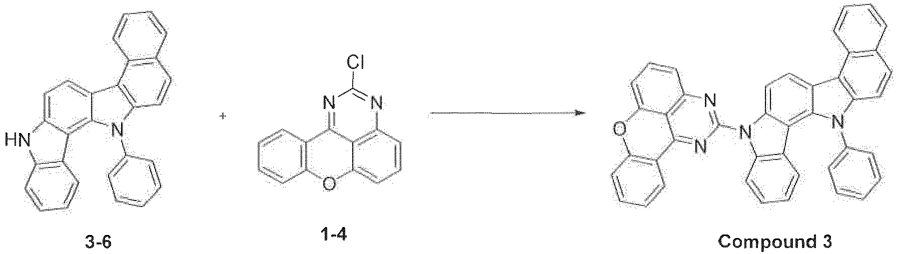

To a dried 100 mL three-necked round-bottom flask were added 3-6 (1.912 g, 5 mmol), potassium carbonate (1.382 g, 10.00 mmol) and 1-4 (1.27 g, 5.00 mmol) and placed under N₂ for 5 min. Degassed DMF (15 ml) was added and the reaction was heated at 120 °C for 21 h. Then, the reaction was allowed to cool down to room temperature and filtered, rinsing with toluene, water and EtOH. The crude yellow solid was recrystallized from chlorobenzene, and then purified via flash chromatography with CHCl₃ and heptane to give 1.96 g (65%) of **Compound 3** as a yellow solid.
LC-MS: 601[M+H]

### Compound 4

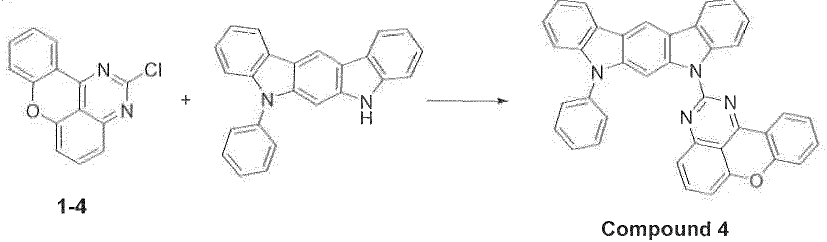

**1-4** (1.23 g, 4.84 mmol), 5,7-Dihydro-5-phenylindolo[2,3-b]carbazole (1.34 g, 4.04 mmol) which prepared according to the protocol mentioned in a patent application (WO2015099484), and sodium *tert*-butoxide (543 mg, 5.65 mmol) were added to 30 mL of Toluene. The mixture was evacuated and purged with argon gas three times. Then, ^{t}Bu₃P-HBF₄ (94 mg, 0.32 mmol) and Pd₂(dba)₃ (74 mg, 0.08 mmol) were added to the mixture, and the reaction mixture was stirred at 80°C for 15 h. The precipitate was collected by filtration, and washed with toluene, EtOH, and water. The product was extracted in Soxhlet extractor with a mixed solvent of THF, chloroform, and EtOH. The solid formed in the solvent was collected by filtration to yield 1.78 g (81%) of **compound 4** as a yellow powder.
LC-MS: 551 [M+H]

### Compound 5

### Intermediate 5-1

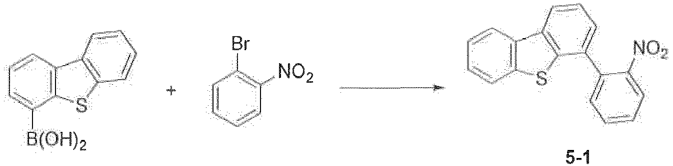

4-Dibenzothiopheneboronic acid (12.9 g, 56.6 mmol), 2-nitrobromobenzene (12.6 g, 62.2 mmol), Pd(PPh)3 (1.3 g, 1.13 mmol), and 2M Na₂CO₃ aqueous solution (85 mL) were entered to dimetoxymethane (260 mL). The mixture was refluxed for 24 h. Then, the reaction mixture was cooled to room temperature. After addition of 500 mL of toluene to the reaction mixture, the aqueous layer was extracted with toluene. The organic layer was dried with Na₂SO₄, filtered and concentrated. The crude product was purified by column chromatography eluting with toluene. Then, it was further purified by recrystallization with toluene to yield 13.7 g (80%) of 5-1 as a yellow solid.
LC-MS: 306 [M+H]

### Intermediate 5-2

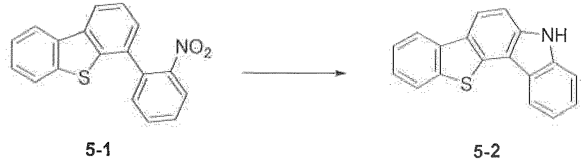

5-1 (13.7 g, 44.9 mmol) and triphenylphosphine (29.4 g, 112 mmol) were entered to 200 mL of *o*-dichlorobenzene. The mixture was stirred at 185 °C for 20 h. After the solvent was removed by distillation, methanol was added there to solidify the product. The solid was washed with hot toluene to yield 5.5 g (45%) of **5-2** as a white solid.
LC-MS: 274 [M+H]

### Compound 5

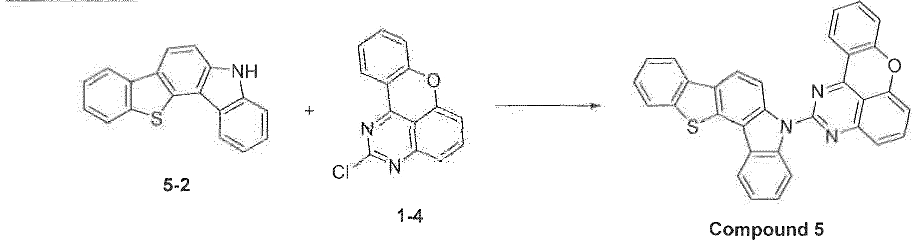

**1-4** (1.36 g, 5.34 mmol), **5-2** (1.39 g, 5.08 mmol), and sodium *tert-*butoxide (489 mg, 5.09 mmol) were added to 24 mL of Toluene. The mixture was evacuated and purged with argon gas three times. Then, ^{t}Bu₃P-HBF₄ (118 mg, 0.41 mmol) and Pd₂(dba)₃ (93 mg, 0.10 mmol) were added to the mixture, and the reaction mixture was stirred at 110°C for 15 h. The precipitate was collected by filtration, and washed with toluene, EtOH, and water. It was purified by chromatography eluting with heptane and toluene to yield 2.28 g (91%) of Compound 5 as a yellow powder.
LC-MS: 551 [M+H]

### Compound 6

### Intermediate 6-1

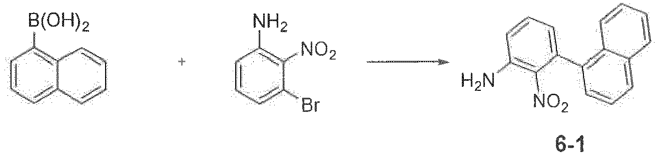

3-Bromo-2-nitroaniline (50.00 g, 230.0 mmol), naphthalene-1-ylboronic acid (44.00 g, 256.0 mmol) and potassium phosphate (98.00 g, 461.0 mmol) were dissolved in 400 mL of tetrahydrofuran and 100 mL of water under nitrogen atmosphere. PB_{U3}-HBF₄ (1.34 g, 4.6 mmol) and Pd₂(dba)₃ (2.11 g, 2.3 mmol) were added and the mixture was refluxed for 3 hours. After cooling to room temperature, the two phases were separated, the aqueous phase was extracted with 30 mL of toluene and the combined organic phases were evaporated. The residue was dissolved in AcOEt and filtered over a silica plug. The filtrate was triturated with heptane until a yellow product precipitates. The mixture was filtered to yield 57 g (92%) of 6-1 as a yellow solid.
LC-MS: 265 [M+H]

### Intermediate 6-2

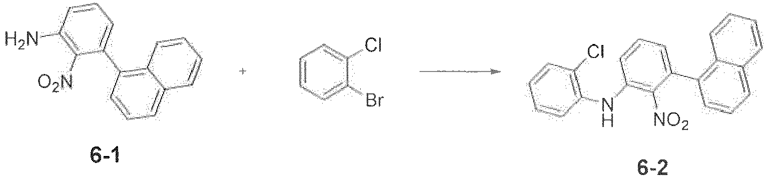

6-1 (15.00 g, 56.8 mmol), 1-bromo-2-chlorobenzene (11.41 g, 59.6 mmol) and cesium carbonate (37.00 g, 114.0 mmol) were suspended in 190 mL of toluene. Nitrogen was bubbled through for 15 minutes. Rac-BINAP (1.06 g, 1.7 mmol) and Pd₂(dba)₃ (1.04 g, 1.1 mmol) were added and the reaction mixture was refluxed for 15 hours. After cooling to room temperature the reaction mixture was filtered over a silica plug, the filtrate was evaporated and the residue was chromatographed with heptane and CH₂Cl₂ as eluent to yield 18.95 g (86%) of 6-2 as a solid.
LC-MS: 375 [M+H]

### Intermediate 6-3

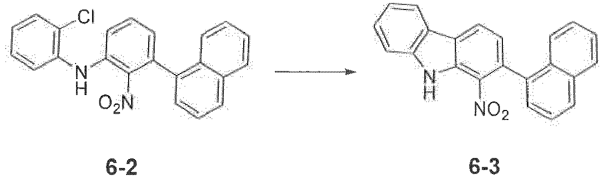

**6-2** (13.00 g, 34.7 mmol), palladium(II)acetate (78 mg, 0.3 mmol), 1,3-Bis(2,6-diisopropylphenyl)imidazolium chloride (0.15 g, 0.3 mmol) and potassium carbonate (9.59 g, 69.4 mmol) were purged with nitrogen and suspended in 170 mL of DMA. Then nitrogen was bubbled through for 15 minutes and the reaction mixture was refluxed for 3 hours. After cooling to room temperature the solvent was removed in vacuo, the residue was suspended in CH₂Cl₂ and concentrated HCI was added. The phases were separated and the organic phase was filtered over a silica plug. The solvent was evaporated and the residue recrystallized in a mixture of CH₂Cl₂ and heptane to yield 7.5 g (64%) of 6-3 as an orange solid.
LC-MS: 336 [M-H]

### Intermediate 6-4

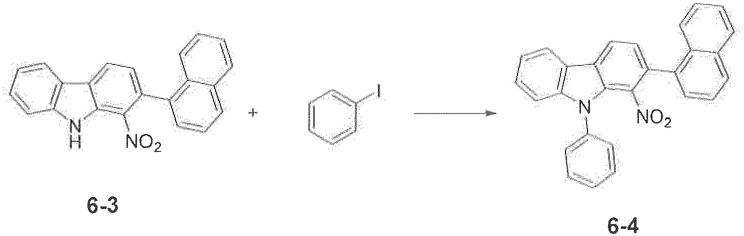

**6-3** (4.83 g, 14.3 mmol), iodobenzene (11.67 g, 57.1 mmol), copper (0.45 g, 7.1 mmol) and potassium carbonate (5.92 g, 42.8 mmol) were suspended in 40 mL of nitrobenzene and nitrogen was bubbled through for 20 minutes. The reaction mixture was stirred at 210°C for 87 hours, then cooled to room temperature and the solvent was evaporated in vacuo. The residue was dissolved in a mixture CH₂Cl₂ and heptane, and filtered over a silica plug. The yellow fraction was collected and concentrated until a yellow solid precipitates. The yellow solid was recovered by filtration to yield 4.86 g (81%) of **6-4** as a solid.
LC-MS: 415 [M+H]

### Intermediate 6-5

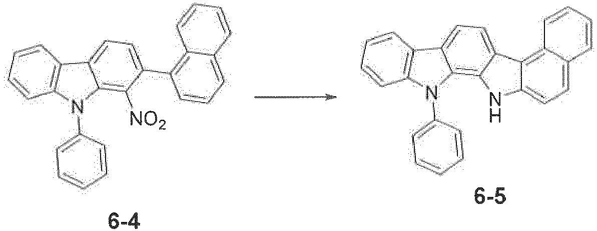

**6-4** (4.40 g, 10.6 mmol) and triphenyl phosphine (13.92 g, 53.1 mmol) were mixed and heated to 200°C. The solution was stirred at 200 °C for 48 hours, then cooled to room temperature. The brown solid was chromatographed with heptane and CH₂Cl₂ as eluent to yield 2.88 g (71%) of **6-5** as a white solid.
LC-MS: 383 [M+H]

### Compound 6

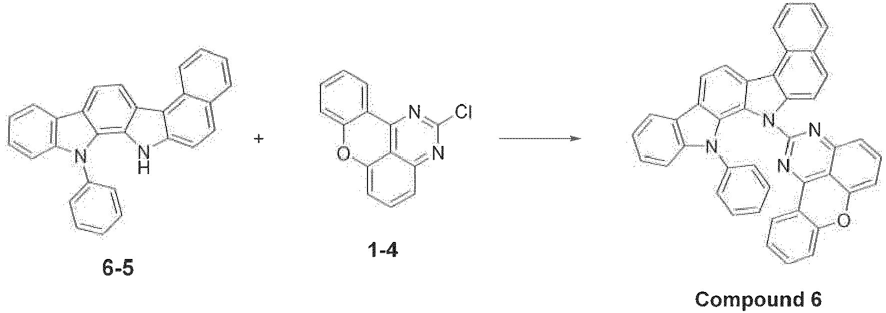

**1-4** (2.85 g, 11.2 mmol), **6-5** (2.85 g, 7.5 mmol), Xantphos (431 mg, 0.75 mmol), Pd₂(dba)₃ (341 mg, 0.37 mmol) and sodium tert-butoxide (0.80 g, 8.3 mmol) were suspended in 100 mL of toluene and nitrogen was bubbled through for 15 minutes. The reaction mixture was stirred at 100°C for 22 hours. The solvent was removed and the residue was dry deposited on silica and chromatographed with heptane and CH₂Cl₂ as eluent to yield 1.80 g (40%) of **Compound 6.**
LC-MS: 601 [M+H]

### Application Example 1

A glass substrate with 120 nm-thick indium-tin-oxide (ITO) transparent electrode (manufactured by Geomatec Co., Ltd.) used as an anode was first cleaned with isopropanol in an ultrasonic bath for 10 min. To eliminate any possible organic residues, the substrate was exposed to an ultraviolet light and ozone for further 30 min. This treatment also improves the hole injection properties of the ITO. The cleaned substrate was mounted on a substrate holder and loaded into a vacuum chamber. Thereafter, the organic materials specified below were applied by vapor deposition to the ITO substrate at a rate of approx. 0.2-1 Å/sec at about 10-6 -10-8 mbar. As the first layer, 5 nm-thick of electron accepting compound A was vapor-deposited. Then 220 nm-thick of aromatic amine compound B was applied as a hole transporting layer. Then, a mixture of 2% by weight of an emitter compound (Compound C), 98% by weight of a host (compound 1) was applied to form a 40 nm-thick phosphorescent-emitting layer. On the emitting layer, a mixture of 50% by weight of an electron transporting compound, Compound D, 50% by weight of Liq (8-Hydroxyquinolate lithium) was applied to form a 30 nm-thick electron transport layer. Finally, 1 nm-thick Liq was deposited as an electron injection layer and 80 nm-thick Al was then deposited as a cathode to complete the device. The device was sealed with a glass lid and a getter in an inert nitrogen atmosphere with less than 1 ppm of water and oxygen.

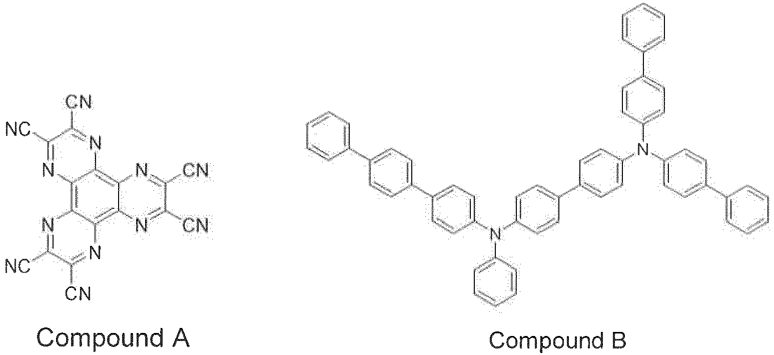

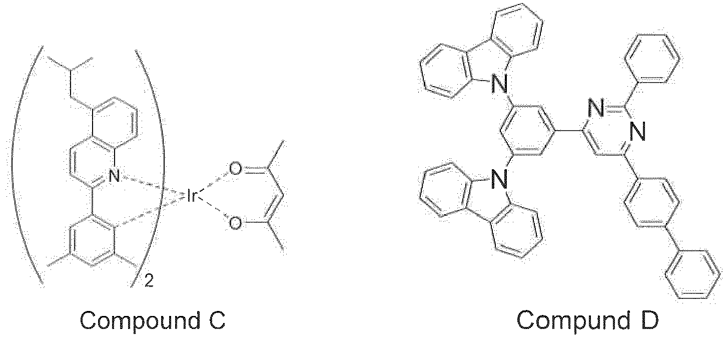

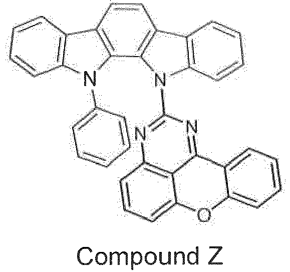

Compound Z is prepared as described in PCT/IB2016/057889 (compound 9).

### Application Example 2

Application Example 2 was repeated except that the emitter (compound C) was replaced by comparative emitter (Comparative A). The device results are shown in Table 1.

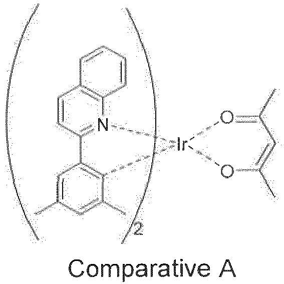

### OLED characterization

To characterize the OLED, electroluminescence spectra were recorded at various currents and voltages. In addition, the current-voltage characteristic was measured in combination with the luminance to determine luminous efficiency and external quantum efficiency (EQE). Driving voltage U, EQE and Commission Internationale de I'Eclairage (CIE) coordinate are given at 10mA/cm2 except otherwise stated.

**Table 1**

| Appl. Ex. | Host | Emitter | U (V) | EQE (%) | CIE x, y |
|---|---|---|---|---|---|
| Appl. Ex. 1 | Compound Z | Compound C | 4.72 | 10.3 | 0.66, 0.34 |
| Appl. Ex. 2 | Compound Z | Comparative A | 4.83 | 7.4 | 0.66, 0.34 |

The results are shown in Table 1. Combination with a specific emitter (Compound C) shows a lower driving voltage and higher EQE than the comparative compound (Comparative A).

### Application Examples 3 to 8

Application Example 1 was repeated except that the host (Compound 6) was replaced by Compound 1 to 5. The device results are shown in Table 2.

**Table 2**

| Appl. Ex. | Host | Emitter | U (V) | EQE (%) | CIE x, y |
|---|---|---|---|---|---|
| Appl. Ex. 3 | Compound 1 | Compound C | 4.7 | 13.2 | 0.66, 0.34 |
| Appl. Ex. 4 | Compound 2 | Compound C | 5.0 | 18.1 | 0.66, 0.34 |
| Appl. Ex. 5 | Compound 3 | Compound C | 4.3 | 16.2 | 0.66, 0.34 |
| Appl. Ex. 6 | Compound 4 | Compound C | 4.8 | 15.2 | 0.66, 0.34 |
| Appl. Ex. 7 | Compound 5 | Compound C | 5.0 | 14.5 | 0.66. 0.34 |
| Appl. Ex. 8 | Compound 6 | Compound C | 4.8 | 12.5 | 0.66, 0.33 |

## Claims

1. A composition comprising
i) at least one Ir complex represented by the following formula (XX)
IrLg₁Lg₂Lg₃ (XX)
wherein
Lg₁, Lg₂ and Lg₃ are selected from the following groups A-1, A-2 and A-3 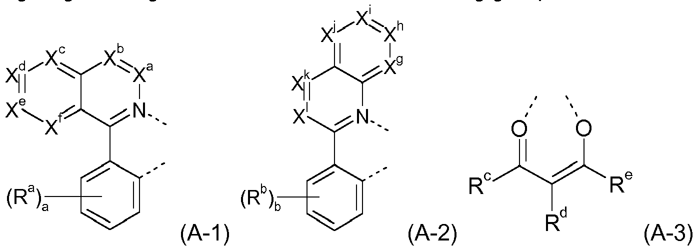
wherein one or two of Lg₁, Lg₂ and Lg₃, preferably one, are a group A-3, and one or two of Lg₁, Lg₂ and Lg₃, preferably two, are selected from the group consisting of A-1 and A-2,
wherein
X^{a} is CR^{a'} or N;
X^{b} is CR^{b'} or N;
X^{c} is CR^{c'} or N;
X^{d} is CR^{d'} or N;
X^{e} is CR^{e'} or N;
X^{f} is CR^{f} or N;
X^{g} is CR^{g} or N;
X^{h} is CR^{h} or N;
Xⁱ is CRⁱ or N;
X^{j} is CR^{j} or N;
X^{k} is CR^{k} or N;and
X^{l} is CR^{l} or N;
wherein at least one of X^{a}, X^{b}, X^{c}, X^{d}, X^{e} and X^{f} is CR^{a'}, CR^{b'}, CR^{c'}, CR^{d'}, CR^{e'} respectively CR^{f}, and at least one of X^{g}, X^{h}, Xⁱ, X^{j}, X^{k} and X^{l} is CR^{g}, CR^{h}, CRⁱ, CR^{j}, CR^{k} respectively CR^{l};
R^{a'}, R^{b'}, R^{c'}, R^{d'}, R^{e'}, R^{f}, R^{g}, R^{h}, Rⁱ, R^{j}, R^{k} and R^{l} each independently represent hydrogen, a halogen, a cyano group, a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group;
wherein at least one of R^{a'}, R^{b'}, R^{c'}, R^{d'}, R^{e'} and R^{f}, and at least one of R^{g}, R^{h}, Rⁱ, R^{j}, R^{k} and R^{l} is a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably a substituted or unsubstituted C₁-C₂₅alkyl group;
R^{a} to R^{e} each independently represent hydrogen, a halogen, a cyano group, a substituted or unsubstituted C₁-C₂₅alkyl group, a substituted or unsubstituted C₃-C₂₅cycloalkyl group, a substituted or unsubstituted C₁-C₂₅silyl group, a substituted or unsubstituted C₆-C₃₀aryl group, or a substituted or unsubstituted C₁-C₃₀ heteroaryl group, preferably, R^{a} to R^{e} each independently represent hydrogen, a substituted or unsubstituted C₁-C₁₀alkyl group, a substituted or unsubstituted C₃-C₁₀cycloalkyl group, a substituted or unsubstituted C₆-C₂₀ary group, or a substituted or unsubstituted C₁-C₂₀ heteroaryl group;
a and b each independently represent an integer of 0 to 5; and
each of R^{a} and R^{b} is the same or different;
wherein the dotted lines are bonding sites;
and
ii) at least one compound represented by the general formula (I) 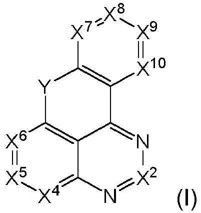 wherein
X² is CR²,
X⁴ is CR⁴ or N,
X⁵ is CR⁵ or N,
X⁶ is CR⁶ or N,
X⁷ is CR⁷ or N,
X⁸ is CR⁸ or N,
X⁹ is CR⁹ or N,
X¹⁰ is CR¹⁰ or N,
Y is O or S,
R², R⁴, R⁵, R⁶, R⁷, R⁸, R⁹, and R¹⁰ are independently of each other selected from H, E, a C₆-C₂₄aryl group which is unsubstituted or substituted by at least one group E, C₂-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
at least two of R⁴, R⁵, R⁶, R⁷, R⁸, R⁹ or R¹⁰, if present at adjacent carbon atoms, together form at least one C₆-C₁₈aryl or C₂-C₁₈heteroaryl ring or ring system,
p is 0 or 1, q is 0 or 1, r is 0 or 1 and s is 0 or 1,
D is independently of each other -CO-, -COO-, -S-, -SO-, -SO₂-, -O-,-CR¹⁵=CR¹⁶-, -NR¹⁷-, -SiR²²R²³-, -POR²⁵-, -C≡C-,
E is independently of each other -OR²¹,-SR²¹, -NR¹⁷R¹⁸, -COR²⁰, -COOR¹⁹, -CONR¹⁷R¹⁸, -CN, -SiR²²R²³R²⁴, POR²⁵R²⁷, halogen, a C₆-C₆aryl group which is unsubstituted or is substituted by at least one -F,-CF₃, -CF₂CF₃, -CF₂CF₂CF₃, -CF(CF₃)₂, -(CF₂)₃CF₃ or -C(CF₃)₃, a C₁-C₁₈ alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group, a C₁-C₆₀heteroaryl group which is unsubstituted or substituted by at least one -F,-CF₃, -CF₂CF₃, -CF₂CF₂CF₃, -CF(CF₃)₂, -(CF₂)₃CF₃, -C(CF₃)₃, a C₁-C₁₈alkyl group, a C₁-C₁₈alkyl group which is interrupted by at least one O, a C₆-C₁₈aryl group which is unsubstituted or substituted by at least one C₁-C₁₈alkyl group or at least one C₁-C₁₈alkoxy group or a C₁-C₁₃heteroaryl group which is unsubstituted or substituted by at least one C₁-C₁₈alkyl group or at least one C₁-C₁₈alkoxy group,
R¹⁵ and R¹⁶ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group which is interrupted by at least one O,
R¹⁷ and R¹⁸ are independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, or
R¹⁷ and R¹⁸ together form a five or six membered aliphatic, aromatic or heteroaromatic ring,
R¹⁹ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²⁰ is H or a C₆-C₁₈aryl group which is unsubstituted or substituted by at least one C₁-C₁₈alkyl group or at least one C₁-C₁₈alkoxy group, a C₁-C₁₈ alkyl group or a C₁-C₁₈alkyl group, which is interrupted by at least one O,
R²¹ is independently of each other H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O,
R²², R²³ and R²⁴ are independently of each other H, a C₁-C₁₈alkyl group, a C₆-C₁₈aryl group which is unsubstituted or substituted by at least one C₁-C₁₈alkyl group, and
R²⁵ and R²⁷ are independently of each other H, a C₁-C₁₈alkyl group, a C₆-C₁₈aryl group which is unsubstituted or substituted by at least one C₁-C₁₈alkyl group, a C₇-C₂₅aralkyl which is unsubstituted or substituted by at least one group E, a C₅-C₁₂cycloalkyl group which is unsubstituted or substituted by at least one group E,
wherein one of R², R⁴, R⁵, R⁶, R⁷, R⁸, R⁹, R¹⁰ in the compound of formula (I) is a N-heteroaryl group according to general formulae (XIII), (XV) or (XXI), preferably, R² in the compound of formula (I) is a N-heteroaryl group according to general formulae (XIII), (XV) or (XXI); 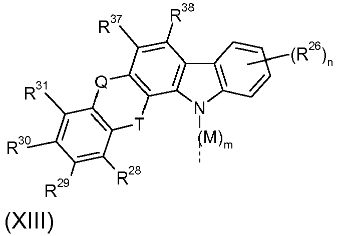
wherein n is 0, 1, 2, 3 or 4,
m is 0, 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused,
R²⁸, R²⁹, R³⁰, R³¹, R³⁷ and R³⁸ are independently of each other H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H, phenyl or biphenyl,
or
at least two of R²⁸, R²⁹, R³⁰, R³¹, R³⁷ or R³⁸, if present at adjacent carbon atoms, may form together at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, more preferably H, methyl, ethyl, or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E or a spiro group, or R³⁴ and R³⁵ together may form a five or six membered, substituted or unsubstituted, aliphatic ring, preferably R³⁴ and R³⁵ are independently of each other H, methyl, ethyl, phenyl or a spiro group, or R³⁴ and R³⁵ together may form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group,
wherein the dotted line is a bonding site; 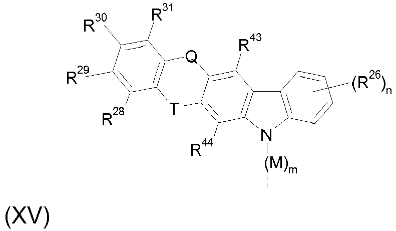
wherein n is 0, 1, 2, 3 or 4,
m is 0, 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D,
R²⁶ is independently of each other selected from E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D,
or at least two of R²⁶, if present at adjacent carbon atoms, may form at least one five or six membered, substituted or unsubstituted, saturated, unsaturated, aromatic or heteroaromatic ring or ring system, to which at least one further aromatic and/or heteroaromatic ring or ring system may be fused, R²⁸, R²⁹, R³⁰, R³¹, R⁴³ and R⁴⁴ are independently of each other selected from H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E, preferably H,
or
at least two of R²⁸, R²⁹, R³⁰ or R³¹, if present at adjacent carbon atoms, together may form at least one C₆-C₁₈aryl or C₂-C₁₈heteroaryl ring or ring system, and
Q and T are independently of each other selected from direct bond, S, O, SiR³²R³³, CR³⁴R³⁵ and NR³⁶, wherein Q and T are not at the same time a direct bond;
wherein R³² and R³³ are independently of each other H, a C₁-C₁₈alkyl group, a C₆-C₁₈aryl group which is unsubstituted or substituted by at least one C₁-C₁₈alkyl group, preferably H, a C₆-C₁₈aryl group which is unsubstituted or a C₁-C₁₈alkyl group, more preferably H, methyl, ethyl or phenyl,
R³⁴ and R³⁵ are independently of each other H, E, a C₆-C₂₄aryl group which is unsubstituted or substituted by at least one group E, C₁-C₂₄heteroaryl group which is unsubstituted or substituted by at least one group E, a C₁-C₂₅alkyl group which unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E or a spiro group, or R³⁴ and R³⁵ together may form a five or six membered, substituted or unsubstituted, aliphatic ring, preferably, R³⁴ and R³⁵ are independently of each other H, ethyl, ethyl, phenyl or a spiro group, or R³⁴ and R³⁵ together may form a five or six membered, substituted or unsubstituted, aliphatic ring,
R³⁶ is H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, for example phenyl,
wherein the dotted line is a bonding site, 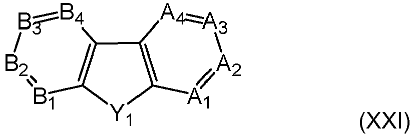
wherein
A¹ is CR⁶² or N,
A² is CR⁶³ or N,
A³ is CR⁶⁴ or N,
A⁴ is CR⁶⁵ or N,
B¹ is CR⁶⁶ or N,
B² is CR⁶⁷ or N,
B³ is CR⁶⁸ or N,
B⁴ is CR⁶⁹ or N,
Y¹ is NR⁷⁰, CR⁷¹R⁷², O or S;
R⁶², R⁶³, R⁶⁴, R⁶⁵, R⁶⁶, R⁶⁷, R⁶⁸ and R⁶⁹ are independently of each other H, direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
or
at least two of R⁶², R⁶³, R⁶⁴ and R⁶⁵ may be directly bonded to the moiety represented by the general formula (XXII) via the two *-locations; 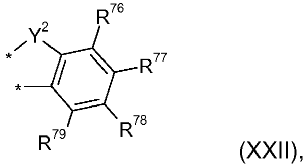
wherein
Y² is NR⁷³, CR⁷⁴R⁷⁵, O or S,
or
at least two of R⁶⁶, R⁶⁷, R⁶⁸ and R⁶⁹ may be directly bonded to the moiety represented by the general formula (XXIII) via the two *-locations. 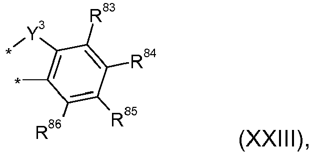
wherein
Y³ is NR⁸⁰, CR⁸¹R⁸², O or S,
R⁷⁰, R⁷³ and R⁸⁰ are independently of each other a direct bond, H, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E, R⁷¹, R⁷², R⁷⁴, R⁷⁵, R⁸¹ and R⁸² are, independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E or a C₅-C₁₂cycloalkyl group which is unsubstituted or substituted by at least one group E,
or R⁷¹ and R⁷², R⁷⁴ and R⁷⁵ and/or R⁸¹ and R⁸² together form at least one C₃-C₁₈-alkyl ring or ring system to which at least one C₆-C₁₈aryl ring or ring system may be attached, R⁷⁶, R⁷⁷, R⁷⁸, R⁷⁹, R⁸³, R⁸⁴, R⁸⁵ and R⁸⁶ are independently of each other H, a direct bond, E, a C₆-C₄₀aryl group which is unsubstituted or substituted by at least one group E, C₁-C₃₀heteroaryl group which is unsubstituted or substituted by at least one group E, a C₁-C₂₅alkyl group, which is unsubstituted or substituted by at least one group E and/or interrupted by at least one group D, a C₇-C₂₅aralkyl which is unsubstituted or substituted by at least one group E, a C₅-C₁₂cycloalkyl group which is unsubstituted or substituted by at least one group E,
or
R⁷⁶ and R⁷⁷, R⁷⁷ and R⁷⁸ and/or R⁷⁸ and R⁷⁹ together may form at least one C₆-C₁₈aryl or C₂-C₁₈heteroaryl ring or ring system, and/or
R⁸³ and R⁸⁴, R⁸⁴ and R⁸⁵ and/or R⁸⁵ and R⁸⁶ together form at least one C₆-C₁₈aryl or C₂-C₁₈heteroaryl ring or ring system,
wherein the heterocyclic group according to general formula (XXI) is connected to the compound according to general formula (I) via one of R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶, wherein this respective residue R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ or R⁸⁶ is a direct bond, optionally interrupted by a group of formula -(M)ₘ-, in this case,
m is 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

2. The composition according to claim 1, wherein the compound of formula (XX) is represented by the following formulae (XX-1) or (XX-2): 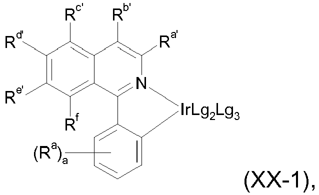 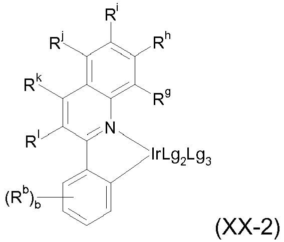 wherein Lg₂ and Lg₃ are a group A-1, A-2 or A-3, wherein in formula (XX-1), at least one of Lg₂ and Lg₃ is a group A-3, preferably, one of Lg₂ and Lg₃ is a group A-3 and the other is a group A-1; and in formula (XX-2), at least one of Lg₂ and Lg₃ is a group A-3, preferably, one of Lg₂ and Lg₃ is a group A-3 and the other is a group A-2.

3. The composition according to claim 1 or 2, wherein the compound of formula (XX) is represented by one of the following formulae (XX-3) and (XX-4):

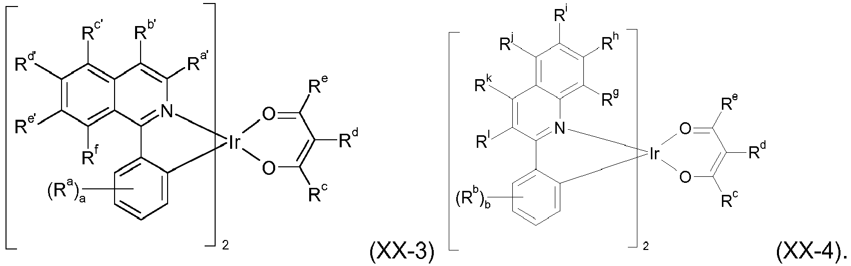

4. The composition according to claim 3, wherein the compounds of formulae (XX-3) and XX-4 are represented by the following formulae (XX-5) and XX-6: 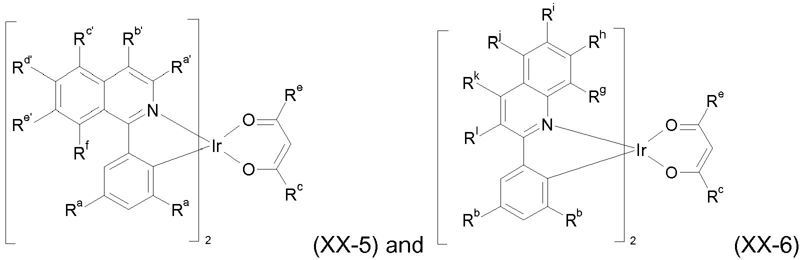 wherein
R^{a} and R^{b} each independently represent a substituted or unsubstituted C₁-C₂₅alkyl group.

5. The composition according to claim 4, wherein the compounds of formulae (XX-5) and XX-6 are represented by the following formulae (XX-7) and XX-8: 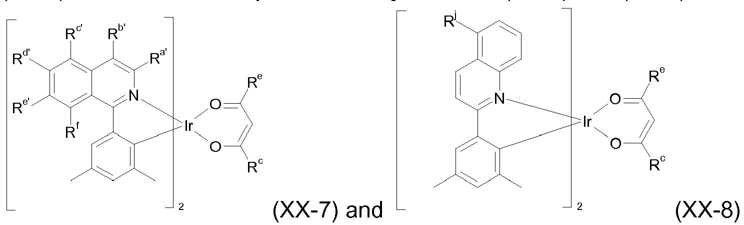 wherein
one or two of R^{a'}, R^{b'}, R^{c'}, R^{d'}, R^{e'} and R^{f} R^{j} each independently represent CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl;
and the remaining of R^{a'}, R^{b'}, R^{c'}, R^{d'}, R^{e'} and R^{f} are hydrogen;
R^{j} is CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyl, cyclohexyl, ethyl, trimethylsilyl, triethylsilyl, triisopropylsilyl, CH[Si(CH₃)₃]₂, trimethylgermyl, triethylgermyl or triisopropylgermyl;
and
R^{c} and R^{e} are independently of each other methyl, CH(CH₃)₂, CH(CH₂CH₃)₂ or CH₂CH(CH₃)₂.

6. The composition according to any one of claims 1 to 5, wherein wherein one of R², R⁴, R5, R⁶, R⁷, R⁸, R⁹, R¹⁰ in the compound of formula (I) is a N-heteroaryl group according to general formula (XIV) 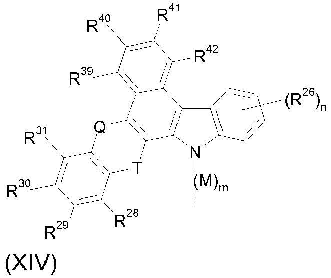 wherein
R³⁹, R⁴⁰, R⁴¹, R⁴² are independently of each other selected from H, a C₆-C₁₈aryl group which is unsubstituted or substituted by at least one C₁-C₁₈alkyl group or at least one C₁-C₁₈alkoxy group, a C₁-C₁₈alkyl group or a C₁-C₁₈alkyl group, which is interrupted by at least one O, preferably H, a C₆-C₁₈aryl group which is unsubstituted or a C₁-C₁₈alkyl group, more preferably R³⁹, R⁴⁰, R⁴¹ and R⁴² are H,
wherein the dotted line is a bonding site.

7. The composition according to any one of claims 1 to 5, wherein the heterocyclic group according to the general formula (XXI) is represented by any one of general formulae (XXIa), (XXIb) or (XXIc)

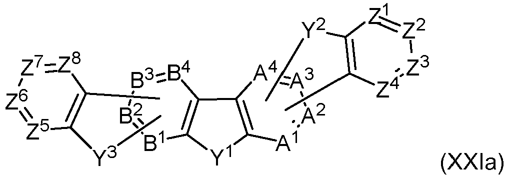

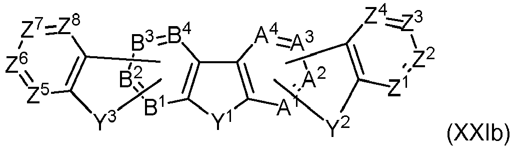

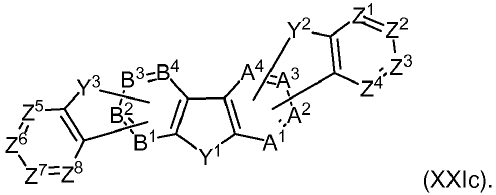

8. The composition according to claim 7 wherein one of Y¹, Y² and Y³ is NR⁷⁰, NR⁷³ or NR⁸⁰ respectively.

9. The composition according to claim 8, wherein two of Y¹, Y² and Y³ are NR⁷⁰, NR⁷³ or NR⁸⁰ respectively.

10. The composition according to any one of claims 1 to 5, 7 and 8 wherein the substituent according to general formula (XXI) is connected to the compound according to general formula (I) via R⁷⁰, R⁷³ or R⁸⁰, wherein this respective residue R⁷⁰, R⁷³ or R⁸⁰ is a direct bond, optionally interrupted by a group of formula -(M)ₘ-,
wherein in this case,
m is 1, 2, 3 or 4,
M is a C₆-C₄₀ arylene group which is unsubstituted or substituted by at least one group E, a C₁-C₂₄heteroarylene group which is unsubstituted or substituted by at least one group E or a C₁-C₂₅ alkylene group which unsubstituted or substituted by at least one group E and/or interrupted by at least one group D.

11. The composition according to any one of claims 1 to 10, wherein in formulae (XIII), (XIV), (XV), (XXI), (XXIa), (XXIb) or (XXIc) m is 0 or 1, preferably 0, M is a C₆-C₄₀arylene group which is unsubstituted or substituted by at least one group E, preferably phenylene, and in formulae (XIII), (XIV) or (XV) at least two of R²⁶, if present at adjacent carbon atoms, form at least one five or six membered, substituted or unsubstituted, saturated, unsaturated, aromatic or heteroaromatic ring.

12. The composition according to any one of claims 1 to11, wherein R⁴, R⁵, R⁶, R⁷, R⁸, R⁹ and R¹⁰ are H and R² is a N-heteroaryl group according to one of the formulae (XIII), (XIV), (XV), (XXI), (XXIa), (XXIb) or (XXIc).

13. The composition according to anyone of claims 1 to 6, 11 and 12, wherein
formula (XIII) is represented by 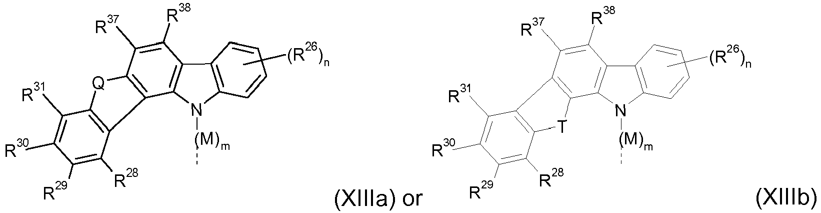 formula (XV) is represented by 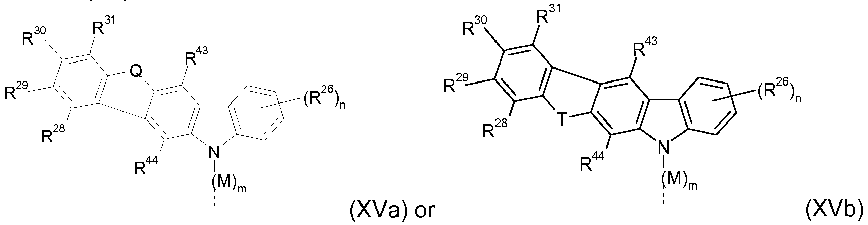 and formula (XIV) is represented by

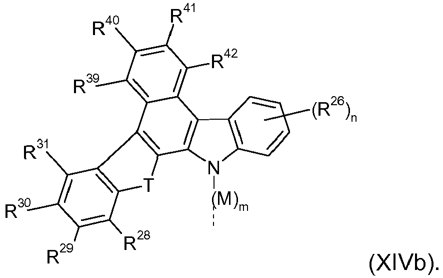

14. The composition according to claim 13, wherein
formula (XIII) is represented by 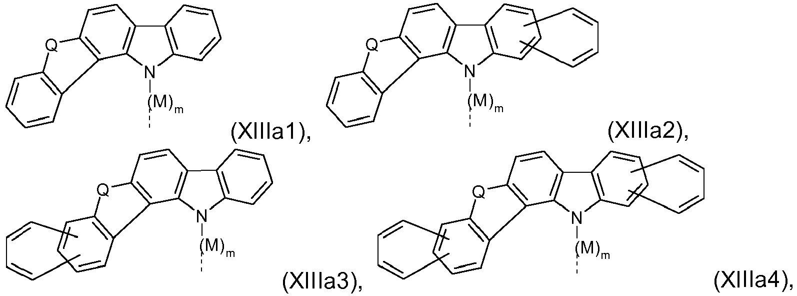 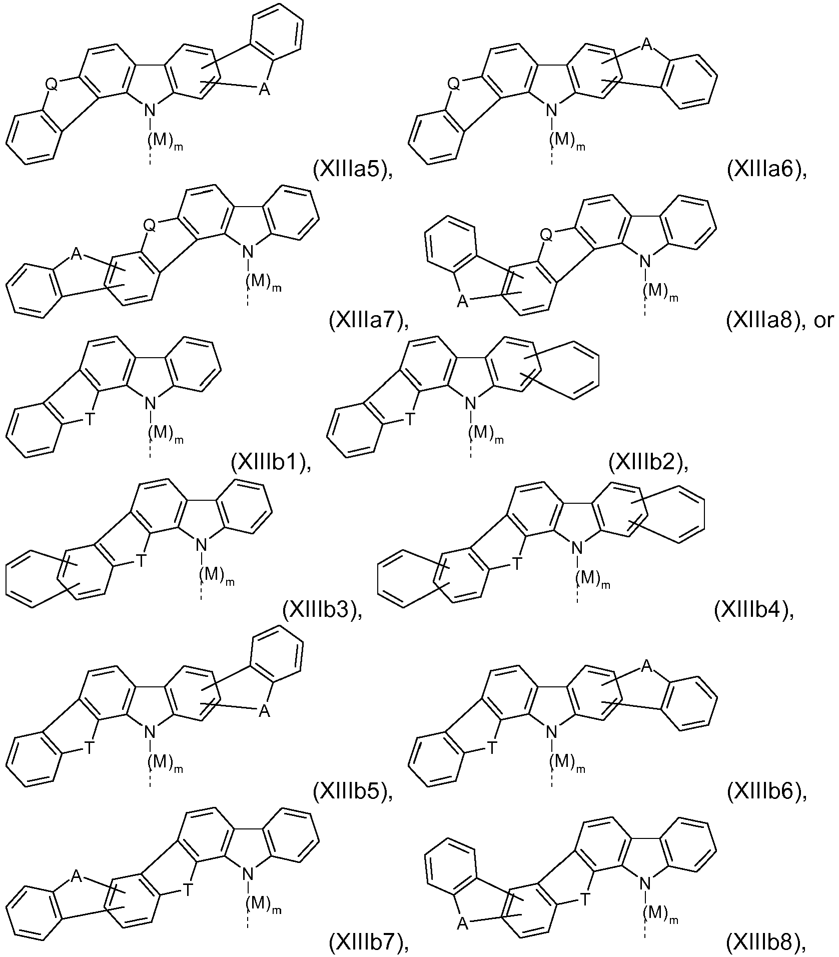 formula (XV) is represented by 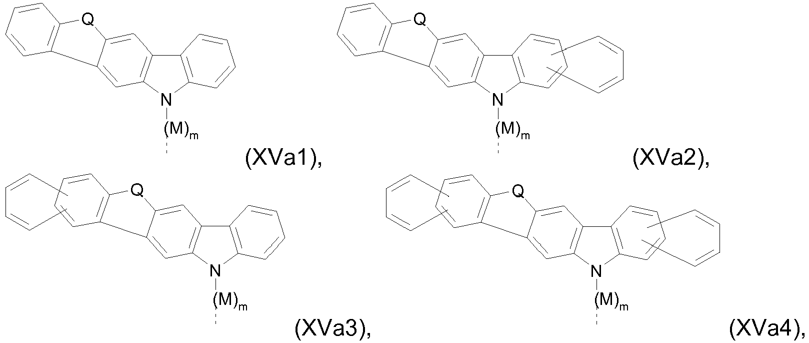 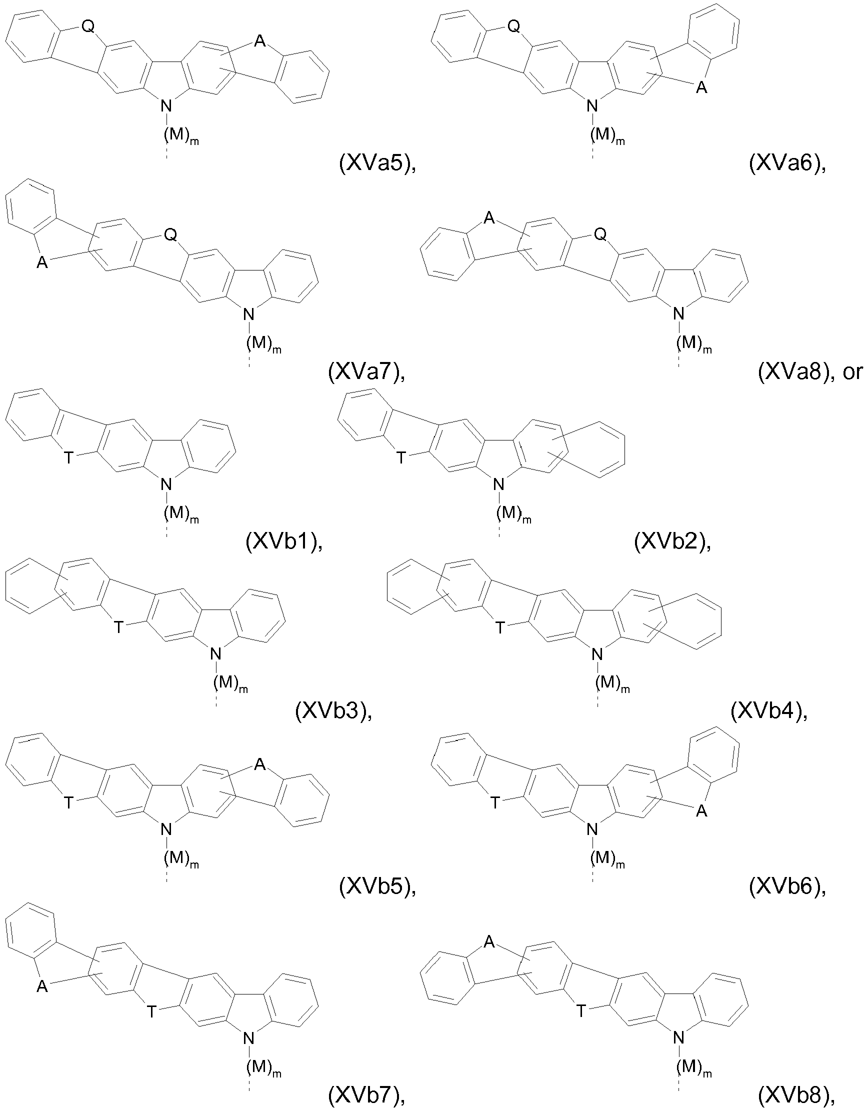 and formula (XIVb) is represented by 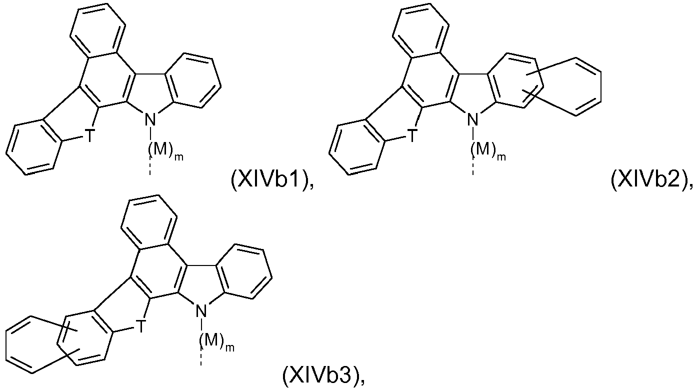 wherein A is S, O, SiR³²R³³, CR³⁴R³⁵ or NR³⁶.

15. The composition according to claim 14, comprising
i) At least one Ir complex represented by the formula 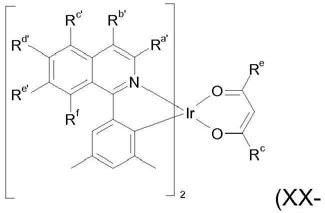 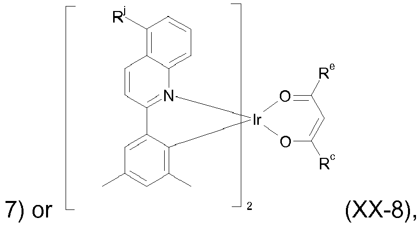 and
ii) at least one compound represented by one of the formulae (XIIIa1), (XIIIa2), (XIIIa3), (XIIIa4), (XIIIa5), (XIIIa6), (XIIIa7), (XIIIa8), (XIIIb1), (XIIIb2), (XIIIb3), (XIIIb4), (XIIIb5), (XIIIb6), (XIIIb7), (XIIIb8), (XIVb1), (XIVb2), (XIVb3), (XVa1), (XVa2), (XVa3), (XVa4), (XVa5), (XVa6), (XVa7), (XVa8), (XVb1), (XVb2), (XVb3), (XVb4), (XVb5), (XVb6), (XVb7) or (XVb8).

16. An emitting layer comprising at least one dopant material of formula (XX) as described in any one of claims 1 to 5 and at least one host material of formula (I) as described in any one of claims 1 or 6 to 14.

17. An electronic device comprising at least one dopant material of formula (XX) as described in any one of claims 1 to 5 and at least one host material of formula (I) as described in any one of claims 1 or 6 to 14,or comprising the emitting layer according to claim 16.

18. An electronic device, preferably an organic electroluminescence device, more preferably an organic light emitting diode (OLED), comprising a cathode, an anode, and a plurality of organic thin film layers provided between the cathode and the anode, the plurality of organic thin film layers comprising at least one emitting layer comprising at least one dopant material of formula (XX) as described in any one of claims 1 to 5 and at least one host material of formula (I) as described in any one of claims 1 or 6 to 14.

## Patentansprüche

1. Zusammensetzung, umfassend
i) mindestens einen Ir-Komplex, der durch die folgende Formel (XX) wiedergegeben wird:
IrLg₁Lg₂Lg₃ (XX)
wobei
Lg₁, Lg₂ und Lg₃ aus den folgenden Gruppen A-1, A-2 und A-3 ausgewählt sind: 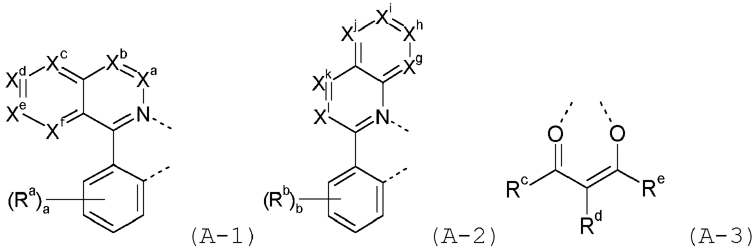
wobei eine oder zwei der Gruppen Lg₁, Lg₂ und Lg₃, vorzugsweise eine, für eine Gruppe A-3 stehen und eine oder zwei der Gruppen Lg₁, Lg₂ und Lg₃, vorzugsweise zwei, aus der Gruppe bestehend aus A-1 und A-2 ausgewählt sind,
wobei
X^{a} für CR^{a'} oder N steht;
X^{b} für CR^{b'} oder N steht;
X^{c} für CR^{c'} oder N steht;
X^{d} für CR^{d'} oder N steht;
X^{e} für CR^{e'} oder N steht;
X^{f} für CR^{f} oder N steht;
X^{g} für CR^{g} oder N steht;
X^{h} für CR^{h} oder N steht;
X¹ für CRⁱ oder N steht;
X^{j} für CR^{j} oder N steht;
X^{k} für CR^{k} oder N steht; und
X¹ für CR^{l} oder N steht;
wobei mindestens eine der Variablen X^{a}, X^{b}, X^{c}, X^{d}, X^{e} und X^{f} für CR^{a'}, CR^{b'}, CR^{c'}, CR^{d'}, CR^{e'} bzw. CR^{f} steht und mindestens eine der Variablen X^{g}, X^{h}, Xⁱ, x^{j}, X^{k} und X^{l} für CR^{g}, CR^{h}, CRⁱ, CR^{j}, CR^{k} bzw. CR^{l} steht;
R^{a'}, R^{b'}, R^{c'}, R^{d'}, R^{e'}, R^{f}, R^{g}, R^{h}, Rⁱ, R^{j}, R^{k} und R^{l} jeweils unabhängig für Wasserstoff, ein Halogen, eine Cyanogruppe, eine substituierte oder unsubstituierte C₁-C₂₅-Alkylgruppe, eine substituierte oder unsubstituierte C₃-C₂₅-Cycloalkylgruppe, eine substituierte oder unsubstituierte C₁-C₂₅-Silylgruppe, eine substituierte oder unsubstituierte C₆-C₃₀-Arylgruppe oder eine substituierte oder unsubstituierte C₁-C₃₀)-Het-eroarylgruppe stehen;
wobei mindestens eine der Variablen R^{a'}, R^{b'}, R^{c'}, R^{d'}, R^{e'} und R^{f} und mindestens eine der Variablen R^{g}, R^{h}, Rⁱ, R^{j}, R^{k} und R^{l} für eine substituierte oder unsubstituierte C₁-C₂₅-Alkylgruppe, eine substituierte oder unsubstituierte C₃-C₂₅-Cycloalkylgruppe, eine substituierte oder unsubstituierte C₁-C₂₅-Silylgruppe, eine substituierte oder unsubstituierte C₆-C₃₀-Arylgruppe oder eine substituierte oder unsubstituierte C₁-C₃₀-Heteroarylgruppe, vorzugsweise eine substituierte oder unsubstituierte C₁-C₂₅-Alkylgruppe, steht;
R^{a} bis R^{e} jeweils unabhängig für Wasserstoff, ein Halogen, eine Cyanogruppe, eine substituierte oder unsubstituierte C₁-C₂₅-Alkylgruppe, eine substituierte oder unsubstituierte C₃-C₂₅-Cycloalkylgruppe, eine substituierte oder unsubstituierte C₁-C₂₅-Silylgruppe, eine substituierte oder unsubstituierte C₆-C₃₀-Arylgruppe oder eine substituierte oder unsubstituierte C₁-C₃₀-Heteroarylgruppe stehen, vorzugsweise R^{a} bis R^{e} jeweils unabhängig für Wasserstoff, eine substituierte oder unsubstituierte C₁-C₁₀-Alkylgruppe, eine substituierte oder unsubstituierte C₃-C₁₀-Cycloalkylgruppe, eine substituierte oder unsubstituierte C₆-C₂₀-Arylgruppe oder eine substituierte oder unsubstituierte C₁-C₂₀-Heteroarylgruppe stehen;
a und b jeweils unabhängig für eine ganze Zahl von 0 bis 5 stehen und
R^{a} und R^{b} jeweils gleich oder verschieden sind; wobei es sich bei den gestrichelten Linien um Bindungsstellen handelt;
und
ii) mindestens eine Verbindung, die durch die allgemeine Formel (I) wiedergegeben wird: 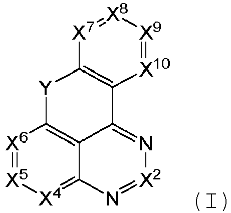 wobei
X² für CR² steht,
X⁴ für CR⁴ oder N steht,
X⁵ für CR⁵ oder N steht,
X⁶ für CR⁶ oder N steht,
X⁷ für CR⁷ oder N steht,
X⁸ für CR⁸ oder N steht,
X⁹ für CR⁹ oder N steht,
X¹⁰ für CR¹⁰ oder N steht,
Y für O oder S steht,
R², R⁴, R⁵, R⁶, R⁷, R⁸, R⁹ und R¹⁰ unabhängig voneinander aus H, E, einer C₆-C₂₄-Arylgruppe, die unsubstituiert oder durch mindestens eine Gruppe E substituiert ist, einer C₂-C₃₀-Heteroarylgruppe, die unsubstituiert oder durch mindestens eine Gruppe E substituiert ist, einer C₁-C₂₅-Alkylgruppe, die unsubstituiert oder durch mindestens eine Gruppe E substituiert und/oder durch mindestens eine Gruppe D unterbrochen ist, einer C₇-C₂₅-Aralkylgruppe, die unsubstituiert oder durch mindestens eine Gruppe E substituiert ist, eine C₅-C₁₂-Cycloalkylgruppe, die unsubstituiert oder durch mindestens eine Gruppe E substituiert ist, ausgewählt sind
oder
mindestens zwei der Variablen R⁴, R⁵, R⁶, R⁷, R⁸, R⁹ oder R¹⁰ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden,
p für 0 oder 1 steht, q für 0 oder 1 steht, r für 0 oder 1 steht und s für 0 oder 1 steht,
D unabhängig voneinander für -CO-, -COO-, -S-, -SO-, -SO₂-, -O-, -CR¹⁵=CR¹⁶-, -NR¹⁷-, -SiR²²R²³-, -POR²⁵-, -C=C- steht,
E unabhängig voneinander für -OR²¹, -SR²¹, -NR¹⁷R¹⁸, -COR²⁰, -COOR¹⁹, -CONR¹⁷R¹⁸, -CN, -SiR²²R²³R²⁴, P0R²⁵R²⁷, Halogen, eine C₆-C₆₀-Arylgruppe, die unsubstituiert oder durch mindestens ein -F, -CF₃, -CF₂CF₃, -CF₂CF₂CF₃, -CF(CF₃)₂, - (CF₂)₃CF₃ oder -C(CF₃)₃ substituiert ist, eine C₁-C₁₈-Alkylgruppe, eine C₁-C₁₈-Alkylgruppe, die durch mindestens ein O unterbrochen ist, eine C₆-C₁₈-Arylgruppe, die unsubstituiert oder durch mindestens eine C₁-C₁₈-Alkylgruppe oder mindestens eine C₁-C₁₈-Alkoxygruppe, oder eine C₁-C₁₃-Heteroarylgruppe, die unsubstituiert oder durch mindestens eine C₁-C₁₈-Alkylgruppe oder mindestens eine C₁-C₁₈-Alkoxygruppe substituiert ist, substituiert ist, eine C₁-C₆₀-Heteroarylgruppe, die unsubstituiert oder durch mindestens ein -F, -CF₃, -CF₂CF₃, -CF₂CF₂CF₃, -CF(CF₃)₂, -(CF₂)₃CF₃, -C(CF₃)₃, eine C₁-C₁₈-Alkylgruppe, eine C₁-C₁₈-Alkylgruppe, die durch mindestens ein O unterbrochen ist, C₆-C₁₈-Arylgruppe, die unsubstituiert oder durch mindestens eine C₁-C₁₈-Alkylgruppe oder mindestens eine C₁-C₁₈-Alkoxygruppe, oder eine C₁-C₁₃-Heteroarylgruppe, die unsubstituiert oder durch mindestens eine C₁-C₁₈-Alkylgruppe oder mindestens eine C₁-C₁₈-Alkoxygruppe substituiert ist, substituiert ist,
R¹⁵ und R¹⁶ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen, R¹⁷ und R¹⁸ unabhängig voneinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, stehen oder
R¹⁷ und R¹⁸ zusammen einen fünf- oder sechsgliedrigen aliphatischen, aromatischen oder heteroaromatischen Ring bilden,
R¹⁹ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²⁰ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht,
R²¹ unabhängig voreinander für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, steht, R²², R²³ und R²⁴ unabhängig voneinander für H, eine C₁-C₁₈-Alkylgruppe, eine C₆-C₁₈-Arylgruppe, die unsubstituiert oder durch mindestens eine C₁-C₁₈-Alkylgruppe substituiert ist, stehen und
R²⁵ und R²⁷ unabhängig voneinander für H, eine C₁-C₁₈-Alkylgruppe, eine C₆-C₁₈-Arylgruppe, die unsubstituiert oder durch mindestens eine C₁-C₁₈-Alkylgruppe substituiert ist, eine C₇-C₂₅-Aralkylgruppe, die unsubstituiert oder durch mindestens eine Gruppe E substituiert ist, oder eine C₅-C₁₂-Cycloalkylgruppe, die unsubstituiert oder durch mindestens eine Gruppe E substituiert ist, steht,
wobei eine der Variablen R², R⁴, R⁵, R⁶, R⁷, R⁸, R⁹, R¹⁰ in der Verbindung der Formel (I) für eine N-Heteroarylgruppe gemäß den allgemeinen Formeln (XIII), (XV) oder (XXI) steht, vorzugsweise R² in der Verbindung der Formel (I) für eine N-Heteroarylgruppe gemäß den allgemeinen Formeln (XIII), (XV) oder (XXI) steht; 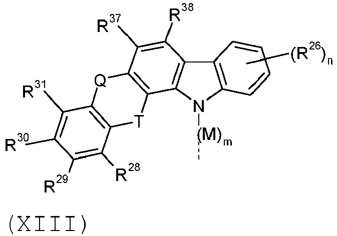
wobei n für 0, 1, 2, 3 oder 4 steht,
m für 0, 1, 2, 3 oder 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht,
R²⁶ unabhängig voneinander aus E, einer C₆-C₄₀-Arylgruppe, die unsubstituiert oder durch mindestens eine Gruppe E substituiert ist, einer C₁-C₃₀)-Heteroarylgruppe, die unsubstituiert oder durch mindestens eine Gruppe E substituiert ist, oder eine C₁-C₂₅-Alkylgruppe, die unsubstituiert oder durch mindestens eine Gruppe E substituiert und/oder durch mindestens eine Gruppe D unterbrochen ist, ausgewählt ist
oder mindestens zwei der Variablen R²⁶ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bzw. ein solches Ringsystem, woran mindestens ein weiterer aromatischer und/oder heteroaromatischen Ring bzw. ein solches Ringsystem anelliert sein kann, bilden können,
R²⁸, R²⁹, R³⁰, R³¹, R³⁷ und R³⁸ unabhängig voneinander für H, E, eine C₆-C₄₀-Arylgruppe, die unsubstituiert oder durch mindestens eine Gruppe E substituiert ist, eine C₁-C₃₀)-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E substituiert ist, oder eine C₅-C₁₂-Cycloalkylgruppe, die unsubstituiert oder durch mindestens eine Gruppe E substituiert ist, vorzugsweise H, Phenyl oder Biphenyl, stehen
oder
mindestens zwei der Variablen R²⁸, R²⁹, R³⁰, R³¹, R³⁷ und R³⁸ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden können und
Q und T unabhängig voneinander aus einer direkten Bindung, S, O, SiR³²R³³, CR³⁴R³⁵ und NR³⁶ ausgewählt sind, wobei Q und T nicht gleichzeitig für eine direkte Bindung steht;
wobei R³² und R³³ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe substituiert ist, vorzugsweise H, eine C₆-C₁₈-Arylgruppe, die unsubstituiert ist, oder eine C₁-C₁₈-Alkylgruppe, weiter bevorzugt H, Methyl, Ethyl oder Phenyl, stehen,
R³⁴ und R³⁵ unabhängig voneinander für H, E, eine C₆-C₂₄-Arylgruppe, die unsubstituiert oder durch mindestens eine Gruppe E substituiert ist, eine C₁-C₂₄-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E substituiert ist, eine C₅-C₁₂-Cycloalkylgruppe, die unsubstituiert oder durch mindestens eine Gruppe E substituiert ist, oder eine Spirogruppe stehen oder R³⁴ und R³⁵ zusammen einen fünf- oder sechsgliedrigen, substituierten oder unsubstituierten, aliphatischen Ring bilden können, vorzugsweise R³⁴ und R³⁵ unabhängig voneinander für H, Methyl, Ethyl, Phenyl oder eine Spirogruppe stehen oder R³⁴ und R³⁵ zusammen einen fünf- oder sechsgliedrigen, substituierten oder unsubstituierten, aliphatischen Ring bilden können,
R³⁶ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, vorzugsweise H, eine C₆-C₁₈-Arylgruppe, die unsubstituiert ist, oder eine C₁-C₁₈-Alkylgruppe, steht,
wobei es sich bei der gestrichelten Linie um eine Bindungsstelle handelt; 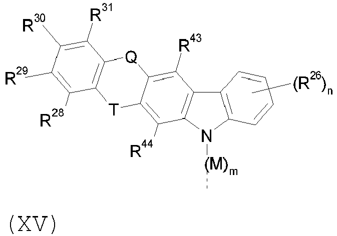
wobei n für 0, 1, 2, 3 oder 4 steht,
m für 0, 1, 2, 3 oder 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht,
R²⁶ unabhängig voneinander aus E, einer C₆-C₄₀-Arylgruppe, die unsubstituiert oder durch mindestens eine Gruppe E substituiert ist, einer C₁-C₃₀)-Heteroarylgruppe, die unsubstituiert oder durch mindestens eine Gruppe E substituiert ist, oder eine C₁-C₂₅-Alkylgruppe, die unsubstituiert oder durch mindestens eine Gruppe E substituiert und/oder durch mindestens eine Gruppe D unterbrochen ist, ausgewählt ist
oder mindestens zwei der Variablen R²⁶ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bzw. ein solches Ringsystem, woran mindestens ein weiterer aromatischer und/oder heteroaromatischen Ring bzw. ein solches Ringsystem anelliert sein kann, bilden können,
R²⁸, R²⁹, R³⁰, R³¹, R⁴³ und R⁴⁴ unabhängig voneinander für H, E, eine C₆-C₄₀-Arylgruppe, die unsubstituiert oder durch mindestens eine Gruppe E substituiert ist, eine C₁-C₃₀-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E substituiert ist, oder eine C₅-C₁₂-Cycloalkylgruppe, die unsubstituiert oder durch mindestens eine Gruppe E substituiert ist, vorzugsweise H, stehen
oder
mindestens zwei der Variablen R²⁸, R²⁹, R³⁰ oder R³¹ dann, wenn sie an benachbarten Kohlenstoffatomen vorliegen, zusammen mindestens einen C₆-C₁₈-Aryl- oder C₂-C₁₈-Heteroarylring bzw. ein solches Ringsystem bilden können und
Q und T unabhängig voneinander aus einer direkten Bindung, S, O, SiR³²R³³, CR³⁴R³⁵ und NR³⁶ ausgewählt sind, wobei Q und T nicht gleichzeitig für eine direkte Bindung steht;
wobei R³² und R³³ unabhängig voneinander für H, eine C₁-C₁₈-Alkylgruppe oder eine C₆-C₁₈-Arylgruppe, die unsubstituiert oder durch mindestens eine C₁-C₁₈-Alkylgruppe, substituiert ist, vorzugsweise H, eine C₆-C₁₈-Arylgruppe, die unsubstituiert ist, oder eine C₁-C₁₈-Alkylgruppe, weiter bevorzugt H, Methyl, Ethyl oder Phenyl, stehen,
R³⁴ und R³⁵ unabhängig voneinander für H, E, eine C₆-C₂₄-Arylgruppe, die unsubstituiert oder durch mindestens eine Gruppe E substituiert ist, eine C₁-C₂₄-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E substituiert ist, eine C₅-C₁₂-Cycloalkylgruppe, die unsubstituiert oder durch mindestens eine Gruppe E substituiert ist, oder eine Spirogruppe stehen oder R³⁴ und R³⁵ zusammen einen fünf- oder sechsgliedrigen, substituierten oder unsubstituierten, aliphatischen Ring bilden können, vorzugsweise R³⁴ und R³⁵ unabhängig voneinander für H, Methyl, Ethyl, Phenyl oder eine Spirogruppe stehen oder R³⁴ und R³⁵ zusammen einen fünf- oder sechsgliedrigen, substituierten oder unsubstituierten, aliphatischen Ring bilden können,
R³⁶ für H, eine C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, vorzugsweise H, eine C₆-C₁₈-Arylgruppe, die unsubstituiert ist, oder eine C₁-C₁₈-Alkylgruppe, beispielsweise Phenyl, steht,
wobei es sich bei der gestrichelten Linie um eine Bindungsstelle handelt; 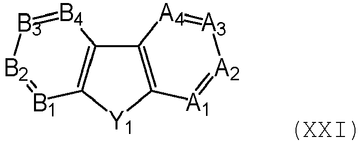
wobei
A¹ für CR⁶² oder N steht,
A² für CR⁶³ oder N steht,
A³ für CR⁶⁴ oder N steht,
A⁴ für CR⁶⁵ oder N steht,
B¹ für CR⁶⁶ oder N steht,
B² für CR⁶⁷ oder N steht,
B³ für CR⁶⁸ oder N steht,
B⁴ für CR⁶⁹ oder N steht,
Y¹ für NR⁷⁰, CR⁷¹R⁷² , O oder S steht,
R⁶², R⁶³, R⁶⁴, R⁶⁵, R⁶⁶, R⁶⁷, R⁶⁸ und R⁶⁹ unabhängig voneinander aus H, einer direkten Bindung, E, einer C₆-C₄₀-Arylgruppe, die unsubstituiert oder durch mindestens eine Gruppe E substituiert ist, einer C₁-C₃₀-Heteroarylgruppe, die unsubstituiert oder durch mindestens eine Gruppe E substituiert ist, einer C₁-C₂₅-Alkylgruppe, die unsubstituiert oder durch mindestens eine Gruppe E substituiert und/oder durch mindestens eine Gruppe D unterbrochen ist, einer C₇-C₂₅-Aralkylgruppe, die unsubstituiert oder durch mindestens eine Gruppe E unterbrochen ist, oder einer C₅-C₁₂-Cycloalkylgruppe, die unsubstituiert oder durch mindestens eine Gruppe E substituiert ist, ausgewählt sind
oder
mindestens zwei der Variablen R⁶², R⁶³, R⁶⁴ und R⁶⁵ über die beiden *-Positionen direkt an die durch die allgemeine Formel (XXII) wiedergegebene Gruppierung gebunden sein können; 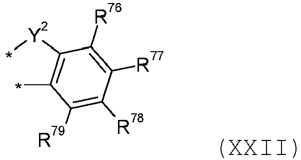
wobei
Y² für NR⁷³, CR⁷⁴R⁷⁵, O oder S steht,
oder
mindestens zwei der Variablen R⁶⁶, R⁶⁷, R⁶⁸ und R⁶⁹ über die beiden *-Positionen direkt an die durch die allgemeine Formel (XXIII) wiedergegebene Gruppierung gebunden sein können; 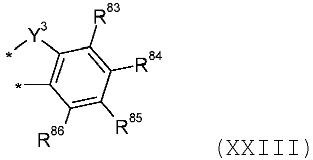
wobei
Y³ für NR⁸⁰, CR⁸¹R⁸², O oder S steht,
R⁷⁰, R⁷³ und R⁸⁰ unabhängig voneinander aus einer direkten Bindung, H, E, einer C₆-C₄₀-Arylgruppe, die unsubstituiert oder durch mindestens eine Gruppe E substituiert ist, einer C₁-C₃₀-Heteroarylgruppe, die unsubstituiert oder durch mindestens eine Gruppe E substituiert ist, einer C₁-C₂₅-Alkylgruppe, die unsubstituiert oder durch mindestens eine Gruppe E substituiert und/oder durch mindestens eine Gruppe D unterbrochen ist, einer C₇-C₂₅-Aralkylgruppe, die unsubstituiert oder durch mindestens eine Gruppe E unterbrochen ist, oder einer C₅-C₁₂-Cycloalkylgruppe, die unsubstituiert oder durch mindestens eine Gruppe E substituiert ist, ausgewählt sind,
R⁷¹, R⁷², R⁷⁴, R⁷⁴, R⁸¹ und R⁸² unabhängig voneinander für H, eine direkte Bindung, E, eine C₆-C₄₀-Arylgruppe, die unsubstituiert oder durch mindestens eine Gruppe E substituiert ist, eine C₁-C₃₀-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E unterbrochen ist, oder eine C₅-C₁₂-Cycloalkylgruppe, die unsubstituiert oder durch mindestens eine Gruppe E substituiert ist, stehen
oder R⁷¹ und R⁷², R⁷⁴ und R⁷⁵ und/oder R⁸¹ und R⁸² zusammen mindestens einen C₃-C₁₈-Alkylring bzw. ein solches Ringsystem, woran mindestens ein C₆-C₁₈-Arylring bzw. ein solches Ringsystem gebunden sein kann, bilden,
R⁷⁶, R⁷⁷, R⁷⁸, R⁷⁹, R⁸³, R⁸⁴, R⁸⁵ und R⁸⁶ unabhängig voneinander für H, eine direkte Bindung, E, eine C₆-C₄₀-Arylgruppe, die unsubstituiert oder durch mindestens eine Gruppe E substituiert ist, eine C₁-C₃₀-Heteroarylgruppe, die unsubstituiert oder durch mindestens eine Gruppe E substituiert ist, eine C₁-C₂₅-Alkylgruppe, die unsubstituiert oder durch mindestens eine Gruppe E substituiert und/oder durch mindestens eine Gruppe D unterbrochen ist, eine C₇-C₂₅-Aralkylgruppe, die unsubstituiert oder durch mindestens eine Gruppe E unterbrochen ist, oder eine C₅-C₁₂-Cycloalkylgruppe, die unsubstituiert oder durch mindestens eine Gruppe E substituiert ist, stehen
oder
R⁷⁶ und R⁷⁷, R⁷⁷ und R⁷⁸ und/oder R⁷⁸ und R⁷⁹ zusammen mindestens einen C₆-C₁₈-Aryl- oder C₂-C₁₈-Hetero-arylring bzw. ein solches Ringsystem bilden können und/oder
R⁸³ und R⁸⁴, R⁸⁴ und R⁸⁵ und/oder R⁸⁵ und R⁸⁶ zusammen mindestens einen C₆-C₁₈-Aryl- oder C₂-C₁₈-Hetero-arylring bzw. ein solches Ringsystem bilden,
wobei die heterocyclische Gruppe gemäß der allgemeinen Formel (XXI) über eine der Variablen R⁷⁰ R⁷³, R⁸⁰, R⁶², R⁶³, R⁶⁴, R⁶⁵, R⁶⁶, R⁶⁷, R⁶⁸, R⁶⁹, R⁷⁶ , R⁷⁷ , R⁷⁸, R⁷⁹, R⁷¹, R⁷², R⁷⁴, R⁷⁵, R⁸¹, R⁸², R⁸³, R⁸⁴, R⁸⁵ oder R⁸⁶ an die Verbindung gemäß der allgemeinen Formel (I) gebunden ist, wobei dieser jeweilige Rest R⁷⁰, R⁷³, R⁸⁰, R⁶², R⁶³, R⁶⁴, R⁶⁵, R⁶⁶, R⁶⁷ , R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸³, R⁸⁴, R⁸⁵ oder R⁸⁶ für eine direkte Bindung, die gegebenenfalls durch eine Gruppe der Formel -(M)ₘ- unterbrochen ist, steht, wobei in diesem Fall
m für 1, 2, 3 oder 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht.

2. Zusammensetzung nach Anspruch 1, wobei die Verbindung der Formel (XX) durch die folgenden Formeln (XX-1) oder (XX-2) wiedergegeben wird: 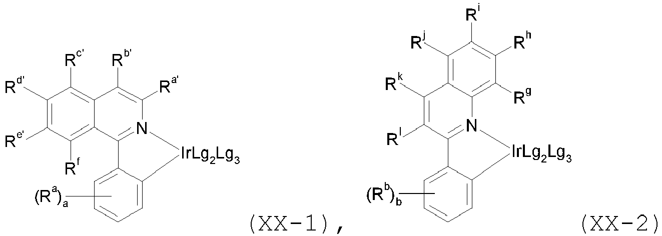 wobei Lg₂ und Lg₃ für eine Gruppe A-1, A-2 oder A-3 stehen, wobei in Formel (XX-1) mindestens eine der Gruppen Lg₂ und Lg₃ für eine Gruppe A-3 steht, vorzugsweise eine der Gruppen Lg₂ und Lg₃ für eine Gruppe A-3 steht und die andere für eine Gruppe A-1 steht; und in Formel (XX-2) mindestens eine der Gruppen Lg₂ und Lg₃ für eine Gruppe A-3 steht, vorzugsweise eine der Gruppen Lg₂ und Lg₃ für eine Gruppe A-3 steht und die andere für eine Gruppe A-2 steht.

3. Zusammensetzung nach Anspruch 1 oder 2, wobei die Verbindung der Formel (XX) durch eine der folgenden Formeln (XX-3) und (XX-4) wiedergegeben wird:

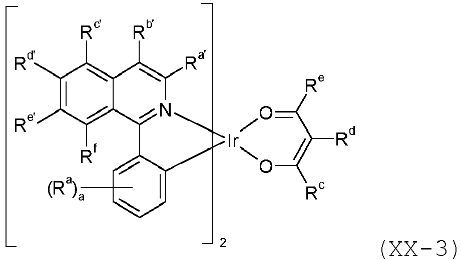

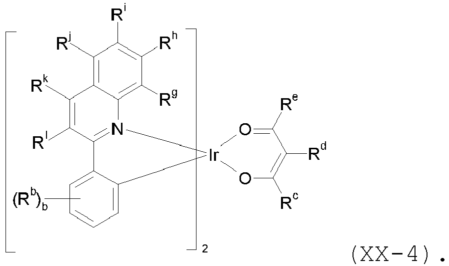

4. Zusammensetzung nach Anspruch 3, wobei die Verbindungen der Formeln (XX-3) und (XX-4) durch die folgenden Formeln (XX-5) und (XX-6) wiedergegeben werden: 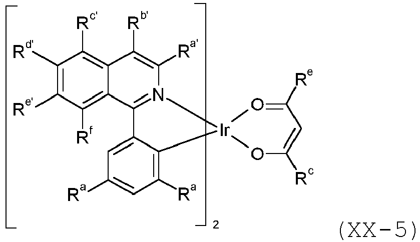 und 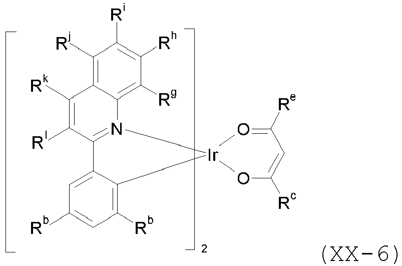 wobei
R^{a} und R^{b} jeweils unabhängig für eine substituierte oder unsubstituierte C₁-C₂₅-Alkylgruppe stehen.

5. Zusammensetzung nach Anspruch 4, wobei die Verbindungen der Formel (XX-5) und (XX-6) durch die folgenden Formeln (XX-7) und (XX-8) wiedergegeben werden: 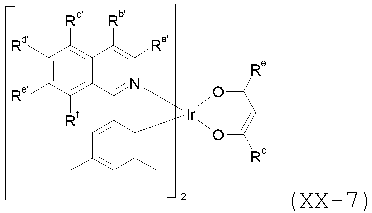 und 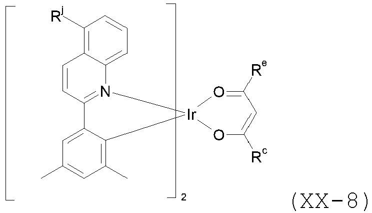 wobei
eine oder zwei der Variablen R^{a'}, R^{b'}, R^{c'}, R^{d'}, R^{e'} und R^{f} R^{j} jeweils unabhängig für CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, Cyclopentyl, Cyclohexyl, Ethyl, Trimethylsilyl, Triethylsilyl, Triisopropylsilyl, CH[Si(CH₃)₃]₂, Trimethylgermyl, Triethylgermyl oder Triisopropylgermyl stehen
und die übrigen der Variablen R^{a'}, R^{b'}, R^{c'}, R^{d'}, R^{e'} und R^{f} für Wasserstoff stehen;
R^{j} für CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, Cyclopentyl, Cyclohexyl, Ethyl, Trimethylsilyl, Triethylsilyl, Triisopropylsilyl, CH[Si(CH₃)₃]₂, Trimethylgermyl, Triethylgermyl oder Triisopropylgermyl steht
und
R^{c} und R^{e} unabhängig voneinander für Methyl, CH(CH₃)₂, CH(CH₂CH₃)₂ oder CH₂CH(CH₃)₂ stehen.

6. Zusammensetzung nach einem der Ansprüche 1 bis 5, wobei eine der Variablen R², R⁴, R⁵, R⁶, R⁷, R⁸, R⁹, R¹⁰ in der Verbindung der Formel (I) für eine N-Heteroarylgruppe gemäß der allgemeinen Formel (XIV) steht: 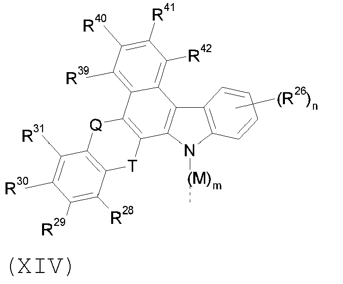 wobei
R³⁹, R⁴⁰, R⁴¹ und R⁴² unabhängig voneinander aus H, einer C₆-C₁₈-Arylgruppe, die unsubstituiert oder durch mindestens eine C₁-C₁₈-Alkylgruppe oder mindestens eine C₁-C₁₈-Alkoxygruppe substituiert ist, eine C₁-C₁₈-Alkylgruppe oder eine C₁-C₁₈-Alkylgruppe, die durch mindestens ein O unterbrochen ist, vorzugsweise H, eine C₆-C₁₈-Arylgruppe, die unsubstituiert ist, oder eine C₁-C₁₈-Alkylgruppe ausgewählt sind, weiter bevorzugt R³⁹, R⁴⁰, R⁴¹ und R⁴² für H stehen,
wobei es sich bei der gestrichelten Linie um eine Bindungsstelle handelt.

7. Zusammensetzung nach einem der Ansprüche 1 bis 5, wobei die heterocyclische Gruppe gemäß der allgemeinen Formel (XXI) durch eine der allgemeinen Formeln (XXIa), (XXIb) oder (XXIc) wiedergegeben wird:

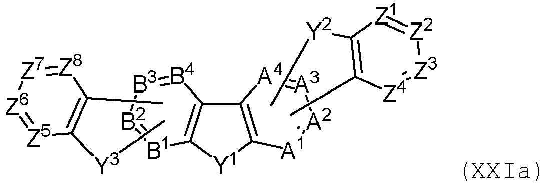

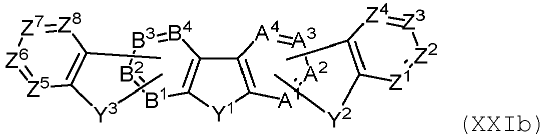

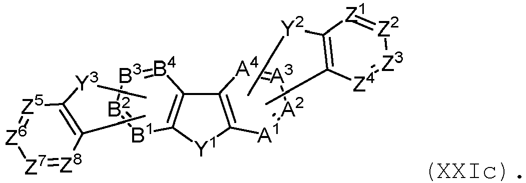

8. Zusammensetzung nach Anspruch 7, wobei eine der Variablen Y¹, Y² und Y³ für NR⁷⁰, NR⁷³ bzw. NR⁸⁰ steht.

9. Zusammensetzung nach Anspruch 8, wobei zwei der Variablen Y¹, Y² und Y³ für NR⁷⁰, NR⁷³ bzw. NR⁸⁰ stehen.

10. Zusammensetzung nach einem der Ansprüche 1 bis 5, 7 und 8, wobei der Substituent gemäß der allgemeinen Formel (XXI) über R⁷⁰, R⁷³ oder R⁸⁰ an die Verbindung gemäß der allgemeinen Formel (I) gebunden ist, wobei dieser jeweilige Rest R⁷⁰, R⁷³ oder R⁸⁰ für eine direkte Bindung, die gegebenenfalls durch eine Gruppe der Formel -(M)ₘ-unterbrochen ist, steht,
wobei in diesem Fall
m für 1, 2, 3 oder 4 steht,
M für eine C₆-C₄₀-Arylengruppe, die unsubstituiert oder durch mindestens eine Gruppe E substituiert ist, eine C₁-C₂₄-Heteroarylengruppe, die unsubstituiert oder durch mindestens eine Gruppe E substituiert ist, oder eine C₁-C₂₅-Alkylengruppe, die unsubstituiert oder durch mindestens eine Gruppe E substituiert und/oder durch mindestens eine Gruppe D unterbrochen ist, steht

11. Verbindung nach einem der Ansprüche 1 bis 10, wobei in den Formeln (XIII), (XIV), (XV), (XXI), (XXIa), (XXIb) oder (XXIc) m für 0 oder 1, vorzugsweise 0, steht, M für eine C₆-C₄₀-Arylen-gruppe, die unsubstituiert oder durch mindestens eine Gruppe E substituiert ist, vorzugsweise Phenylen, steht und in den Formeln (XIII), (XIV) oder (XV) mindestens zwei der Variablen R²⁶ dann, wenn sie an benachbarten Kohlenstoffatomen vorliegen, mindestens einen fünf- oder sechsgliedrigen, substituierten oder unsubstituierten, gesättigten, ungesättigten, aromatischen oder heteroaromatischen Ring bilden.

12. Zusammensetzung nach einem der Ansprüche 1 bis 11, wobei R⁴, R⁵, R⁶, R⁷, R⁸, R⁹ und R¹⁰ für H stehen und R² für eine N-Heteroarylgruppe gemäß einer der Formeln (XIII), (XIV), (XV), (XXI), (XXIa), (XXIb) oder (XXIc) steht.

13. Zusammensetzung nach einem der Ansprüche 1 bis 6, 11 und 12, wobei
Formel (XIII) durch 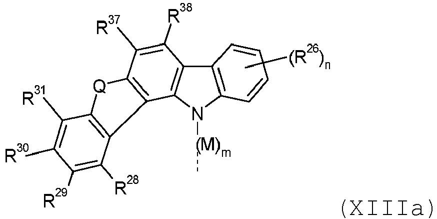 oder 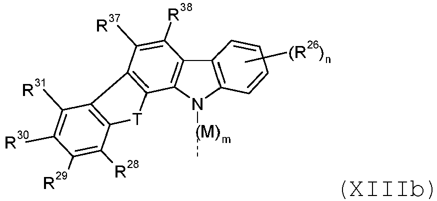 wiedergegeben wird,
Formel (XV) durch 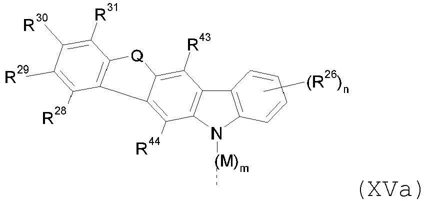 oder 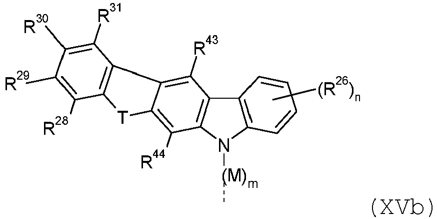 wiedergegeben wird
und Formel (XIV) durch 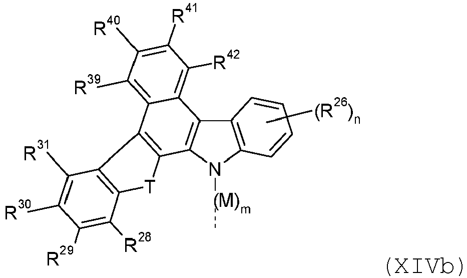 wiedergegeben wird.

14. Verbindung nach Anspruch 13, wobei
Formel (XIII) durch 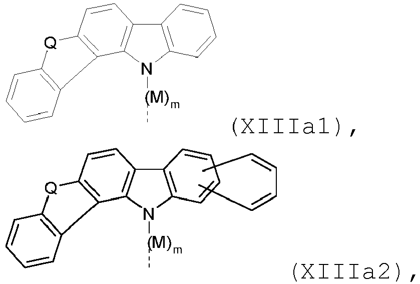 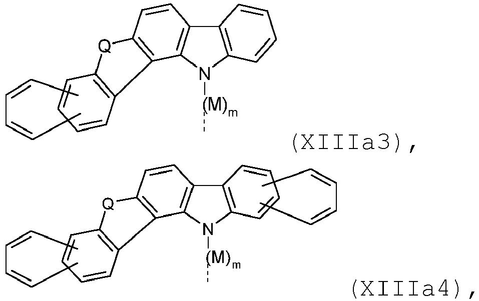 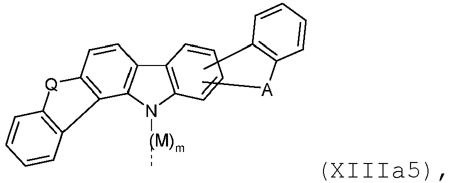 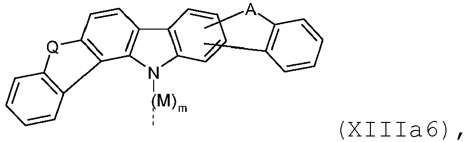 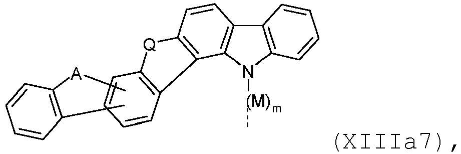 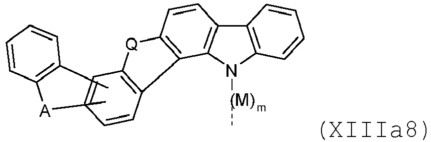 oder 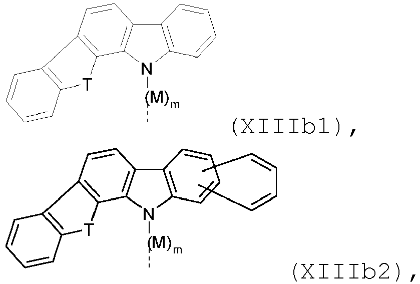 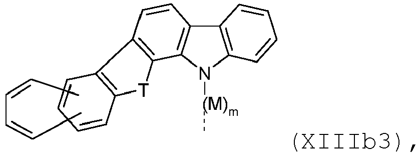 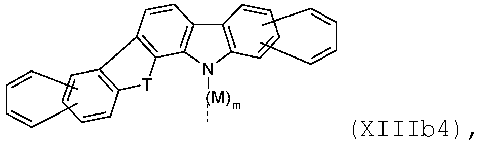 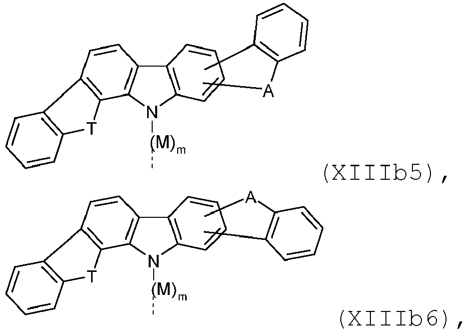 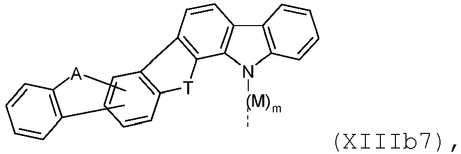 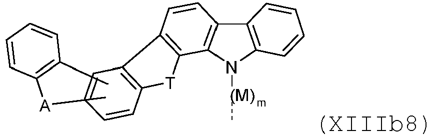 wiedergegeben wird,
Formel (XV) durch 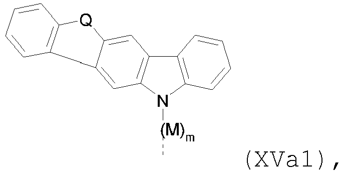 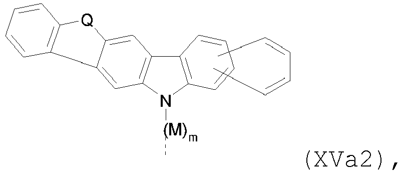 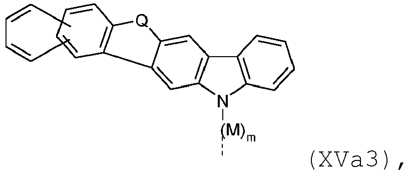 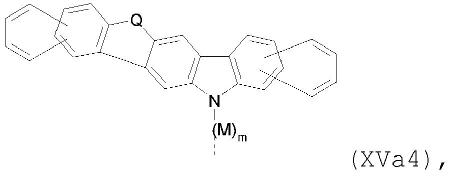 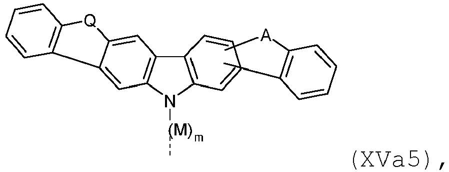 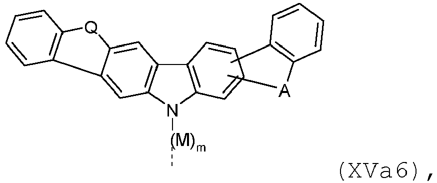 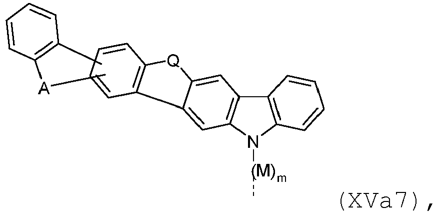 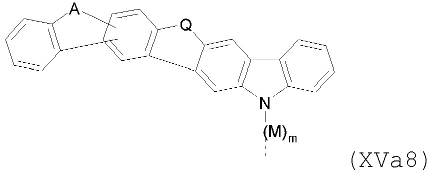 oder 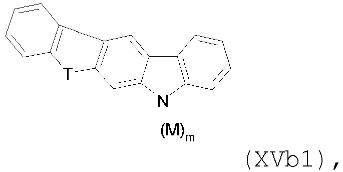 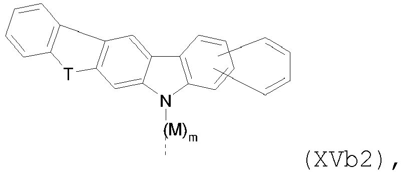 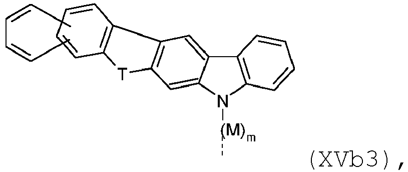 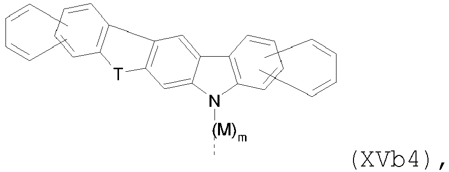 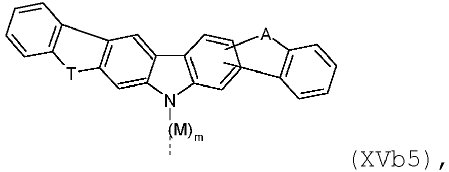 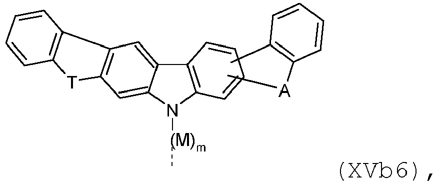 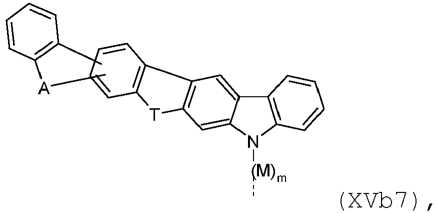 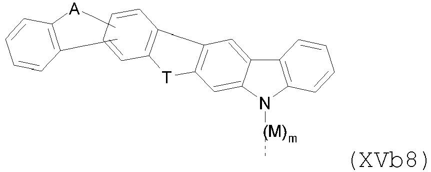 wiedergegeben wird
und Formel (XIVb) durch 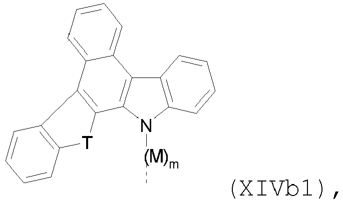 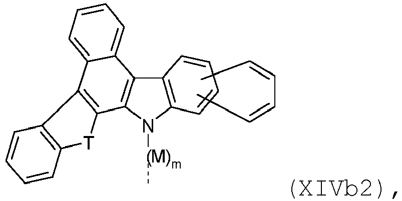 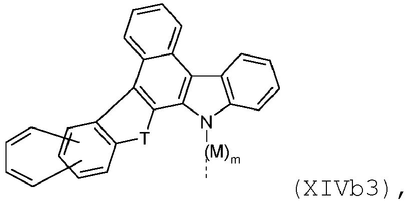 wiedergegeben wird,
wobei A für S, O, SiR³²R³³, CR³⁴R³⁵ oder NR³⁶ steht.

15. Zusammensetzung nach Anspruch 14, umfassend
i) mindestens eine Ir-Komplex, der durch die Formel 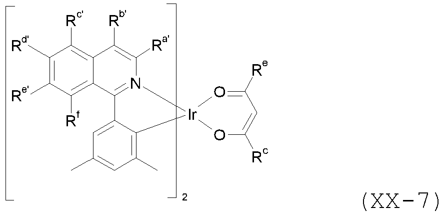 oder 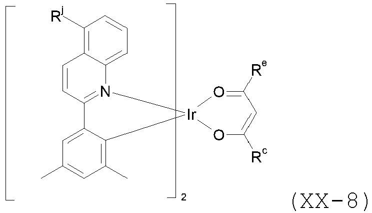 wiedergegeben wird, und
ii) mindestens eine Verbindung, die durch eine der Formeln (XIIIa1), (XIIIa2), (XIIIa3), (XIIIa4), (XIIIa5), (XIIIa6), (XIIIa7), (XIIIa8), (XIIIb1), (XIIIb2), (XIIIb3), (XIIIb4), (XIIIb5), (XIIIb6), (XIIIb7), (XIIIb8), (XIVb1), (XIVb2), (XIVb3), (XVa1), (XVa2), (XVa3), (XVa4), (XVa5), (XVa6), (XVa7), (XVa8), (XVb1), (XVb2), (XVb3), (XVb4), (XVb5), (XVb6), (XVb7) oder (XVb8) wiedergegeben wird.

16. Emissionsschicht, umfassend mindestens ein Dotiermaterial der Formel (XX) gemäß einem der Ansprüche 1 bis 5 und mindestens ein Wirtsmaterial der Formel (I) gemäß einem der Ansprüche 1 oder 6 bis 14.

17. Elektronische Vorrichtung, umfassend mindestens ein Dotiermaterial der Formel (XX) gemäß einem der Ansprüche 1 bis 15 und mindestens ein Wirtsmaterial der Formel (I) gemäß einem der Ansprüche 1 oder 6 bis 14 oder umfassend die Emissionsschicht nach Anspruch 16.

18. Elektronische Vorrichtung, vorzugsweise organische Elektrolumineszenzvorrichtung, weiter bevorzugt eine organische Leuchtdiode (OLED), mit einer Kathode, einer Anode und mehreren organischen Dünnfilmschichten zwischen der Kathode und der Anode, wobei die mehreren organischen Dünnfilmschichten mindestens eine Emissionsschicht umfassen, die mindestens ein Dotiermaterial der Formel (XX) gemäß einem der Ansprüche 1 bis 5 und mindestens ein Wirtsmaterial der Formel (I) gemäß einem der Ansprüche 1 oder 6 bis 14 umfasst.

## Revendications

1. Composition comprenant
i) au moins un complexe d'Ir représenté par la formule suivante (XX)
IrLg₁Lg₂Lg₃ (XX)
Lg₁, Lg₂ et Lg₃ étant choisis parmi les groupes suivants A-1, A-2 et A-3 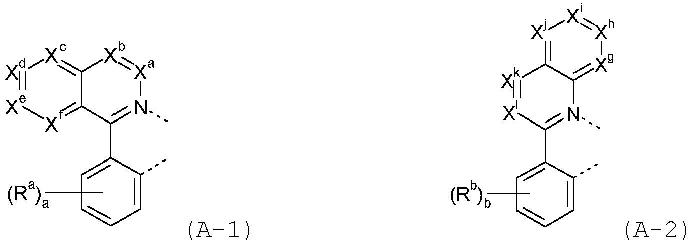 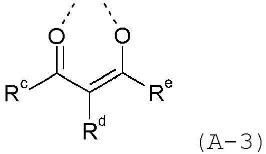
un ou deux parmi Lg₁, Lg₂ et Lg₃, préférablement un, étant un groupe A-3, et un ou deux parmi Lg₁, Lg₂ et Lg₃, préférablement deux, étant choisis dans le groupe constitué par A-1 et A-2,
X^{a} étant CR^{a'} ou N ;
X^{b} étant CR^{b'} ou N ;
X^{c} étant CR^{c'} ou N ;
X^{d} étant CR^{d'} ou N ;
X^{e} étant CR^{e'}ou N ;
X^{f} étant CR^{f} ou N ;
X^{g} étant CR^{g} ou N ;
X^{h} étant CR^{h} ou N ;
Xⁱ étant CRⁱ ou N ;
X^{j} étant CR^{j} ou N ;
X^{k} étant CR^{k} ou N ; et
X^{l} étant CR^{l} ou N ;
au moins l'un parmi X^{a}, X^{b}, X^{c}, X^{d}, X^{e} et X^{f} étant respectivement CR^{a'}, CR^{b'}, CR^{c'}, CR^{d'}, CR^{e'}, CR^{f}, et au moins l'un parmi X^{g}, X^{h}, Xⁱ, X^{j}, X^{k} et X^{l} étant respectivement CR^{g}, CR^{h}, CRⁱ, CR^{j}, CR^{k}, CR^{l} ;
R^{a'}, R^{b'}, R^{c'}, R^{d'}, R^{e'}, R^{f}, R^{g}, R^{h}, Rⁱ, R^{j}, R^{k} et R^{l} représentant chacun indépendamment hydrogène, un halogène, un groupe cyano, un groupe C₁-C₂₅alkyle substitué ou non substitué, un groupe C₃-C₂₅cycloalkyle substitué ou non substitué, un groupe C₁-C₂₅silyle substitué ou non substitué, un groupe C₆-C₃₀aryle substitué ou non substitué, ou un groupe C₁-C₃₀hétéroaryle substitué ou non substitué ;
au moins l'un parmi R^{a'}, R^{b'}, R^{c'}, R^{d'}, R^{e'} et R^{f}, et au moins l'un parmi R^{g}, R^{h}, Rⁱ, R^{j}, R^{k} et R^{l} étant un groupe C₁-C₂₅alkyle substitué ou non substitué, un groupe C₃-C₂₅cycloalkyle substitué ou non substitué, un groupe C₁-C₂₅silyle substitué ou non substitué, un groupe C₆-C₃₀aryle substitué ou non substitué, ou un groupe C₁-C₃₀hétéroaryle substitué ou non substitué, préférablement un groupe C₁-C₂₅alkyle substitué ou non substitué ;
R^{a} à R^{e} représentant chacun indépendamment hydrogène, un halogène, un groupe cyano, un groupe C₁-C₂₅alkyle substitué ou non substitué, un groupe C₃-C₂₅cycloalkyle substitué ou non substitué, un groupe C₁-C₂₅silyle substitué ou non substitué, un groupe C₆-C₃₀aryle substitué ou non substitué, ou un groupe C₁-C₃₀hétéroaryle substitué ou non substitué, préférablement R^{a} à R^{e} représentant chacun indépendamment hydrogène, un groupe C₁-C₁₀alkyle substitué ou non substitué, un groupe C₃-C₁₀cycloalkyle substitué ou non substitué, un groupe C₆-C₂₀aryle substitué ou non substitué, ou un groupe C₁-C₂₀hétéroaryle substitué ou non substitué ;
a et b représentant chacun indépendamment un entier de 0 à 5 ; et
chacun parmi R^{a} et R^{b} étant identique ou différent ;
les lignes en pointillés étant des sites de liaison ;
et
ii) au moins un composé représenté par la formule générale (I) 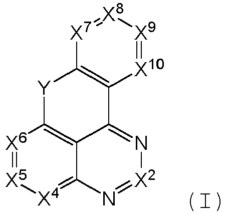
X² étant CR²,
X⁴ étant CR⁴ ou N,
X⁵ étant CR⁵ ou N,
X⁶ étant CR⁶ ou N,
X⁷ étant CR⁷ ou N,
X⁸ étant CR⁸ ou N,
X⁹ étant CR⁹ ou N,
X¹⁰ étant CR¹⁰ ou N,
Y étant O ou S,
R², R⁴, R⁵, R⁶, R⁷, R⁸, R⁹, et R¹⁰ étant indépendamment les uns des autres choisis parmi H, E, un groupe C₆₋₂₄aryle qui est non substitué ou substitué par au moins un groupe E, groupe C₂₋₃₀hétéroaryle qui est non substitué ou substitué par au moins un groupe E, un groupe C₁₋₂₅alkyle, qui est non substitué ou substitué par au moins un groupe E et/ou interrompu par au moins un groupe D, un C₇₋₂₅aralkyle qui est non substitué ou substitué par au moins un groupe E, un groupe un C₅₋₁₂cycloalkyle qui est non substitué ou substitué par au moins un groupe E,
ou
au moins deux parmi R⁴, R⁵, R⁶, R⁷, R⁸, R⁹ et R¹⁰, si présents au niveau d'atomes de carbone adjacents, formant ensemble au moins un cycle ou système cyclique C₆-C₁₈aryle ou C₂-C₁₈hétéroaryle,
p étant 0 ou 1, q étant 0 ou 1, r étant 0 ou 1 et s étant 0 ou 1,
D étant indépendamment les uns des autres -CO-, -COO-, -S-, -SO-, -SO₂-, -O-, -CR¹⁵=CR¹⁶-, -NR¹⁷-, -SiR²²R²³-, -POR²⁵-, -C≡C-,
E étant indépendamment les uns des autres -OR²¹, -SR²¹, -NR¹⁷R¹⁸, -COR²⁰, -COOR¹⁹, -CONR¹⁷R¹⁸, -CN, -SiR²²R²³R²⁴, POR²⁵R²⁷, halogène, un groupe C₆₋₆₀aryle qui est non substitué ou substitué par au moins un -F, , -CF₃, -CF₂CF₃, -CF₂CF₂CF₃, -CF (CF₃)₂, - (CF₂)₃CF₃ ou -C (CF₃)₃, un groupe C₁-C₁₈alkyle, un groupe C₁-C₁₈alkyle qui est interrompu par au moins un O, un groupe C₆-C₁₈aryle qui est non substitué ou substitué par au moins un groupe C₁-C₁₈alkyle ou au moins un groupe C₁-C₁₈alcoxy ou un groupe C₁-C₁₃hétéroaryle qui est non substitué ou substitué par au moins un groupe C₁-C₁₈alkyle ou au moins un groupe C₁-C₁₈alcoxy, un groupe C₁₋₆₀hétéroaryle qui est non substitué ou substitué par au moins un -F, -CF₃, -CF₂CF₃, -CF₂CF₂CF₃, -CF(CF₃)₂, -(CF₂)₃CF₃, -C(CF₃)₃, un groupe C₁-C₁₈alkyle, un groupe C₁-C₁₈alkyle qui est interrompu par au moins un O, un groupe C₆-C₁₈aryle qui est non substitué ou substitué par au moins un groupe C₁-C₁₈alkyle ou au moins un groupe C₁-C₁₈alcoxy ou un groupe C₁-C₁₃hétéroaryle qui est non substitué ou substitué par au moins un groupe C₁-C₁₈alkyle ou au moins un groupe C₁-C₁₈alcoxy,
R¹⁵ et R¹⁶ étant indépendamment l'un de l'autre H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O,
R¹⁷ et R¹⁸ étant indépendamment l'un de l'autre H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O, ou R¹⁷ et R¹⁸ formant ensemble un cycle aliphatique, aromatique ou hétéroaromatique à cinq ou six chaînons,
R¹⁹ étant H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O,
R²⁰ étant H ou un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O,
R²¹ étant indépendamment les uns des autres H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O,
R²², R²³ et R²⁴ étant indépendamment les uns des autres H, un groupe C₁-C₁₈alkyle, un groupe C₆-C₁₈aryle qui est non substitué ou substitué par au moins un groupe C₁-C₁₈alkyle, et
R²⁵ et R²⁷ étant indépendamment les uns des autres H, un groupe C₁-C₁₈alkyle, un groupe C₆-C₁₈aryle qui est non substitué ou substitué par au moins un groupe C₁-C₁₈alkyle, un C₇₋₂₅aralkyle qui est non substitué ou substitué par au moins un groupe E, un groupe C₅₋₁₂cycloalkyle qui est non substitué ou substitué par au moins un groupe E,
l'un parmi R², R⁴, R⁵, R⁶, R⁷, R⁸, R⁹, R¹⁰ dans le composé de formule (I) étant un groupe N-hétéroaryle selon les formules générales (XIII), (XV) ou (XXI) préférablement, R2 dans le composé de formule (I) étant un groupe N-hétéroaryle selon les formules (XIII), (XV) ou (XXI) ; 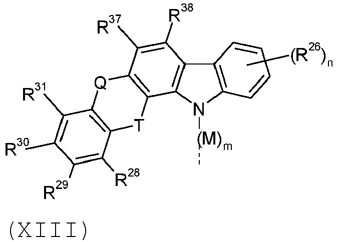
n étant 0, 1, 2, 3 ou 4,
m étant 0, 1, 2, 3 ou 4,
M étant un groupe C₆₋₄₀arylène qui est non substitué ou substitué par au moins un groupe E ou un groupe C₁₋₂₄hétéroarylène qui est non substitué ou substitué par au moins un groupe E, un groupe C₁₋₂₅alkylène qui est non substitué ou substitué par au moins un groupe E et/ou interrompu par au moins un groupe D, R²⁶ étant indépendamment les uns des autres choisi parmi E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, ou au moins deux parmi R²⁶, si présents au niveau d'atomes de carbone adjacents, pouvant former au moins un cycle ou système cyclique saturé, insaturé, aromatique ou hétéroaromatique, substitué ou non substitué, à cinq ou six chaînons, auquel au moins un cycle ou système cyclique aromatique et/ou hétéroaromatique peut être condensé, R²⁸, R²⁹, R³⁰, R³¹, R³⁷ et R³⁸ étant indépendamment les uns des autres H, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E, préférablement H, phényle ou biphényle,
ou
au moins deux parmi R²⁸, R²⁹, R³⁰, R³¹, R³⁷ et R³⁸, si présents au niveau d'atomes de carbone adjacents, pouvant former ensemble au moins un cycle ou système cyclique C₆₋₁₈aryle ou C₂₋₁₈hétéroaryle, et
Q et T étant indépendamment l'un de l'autre choisis parmi une liaison directe, S, O, SiR³²R³³, CR³⁴R³⁵ et NR³⁶, Q et T n'étant pas en même temps une liaison directe ;
R³² et R³³ étant indépendamment l'un de l'autre H, un groupe C₁-C₁₈alkyle, un groupe C₆-C₁₈aryle qui est non substitué ou substitué par au moins un groupe C₁-C₁₈alkyle, préférablement H, un groupe C₆-C₁₈aryle qui est non substitué ou un groupe C₁-C₁₈alkyle, plus préférablement H, méthyle, éthyle, ou phényle,
R³⁴ et R³⁵ étant indépendamment l'un de l'autre H, E, un groupe C₆-C₂₄aryle qui est non substitué ou substitué par au moins un groupe E, un groupe C₁₋₂₄hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E ou un groupe spiro, ou R³⁴ et R³⁵ ensemble pouvant former un cycle aliphatique substitué ou non substitué, à cinq ou six chaînons, préférablement R³⁴ et R³⁵ étant indépendamment l'un de l'autre H, méthyle, éthyle, phényle ou un groupe spiro, ou R³⁴ et R³⁵ ensemble pouvant former un cycle aliphatique substitué ou non substitué, à cinq ou six chaînons,
R³⁶ étant H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle, qui est interrompu par au moins un O, préférablement H, un groupe C₆-C₁₈aryle qui est non substitué ou un groupe C₁-C₁₈alkyle,
la ligne en pointillés étant un site de liaison ; 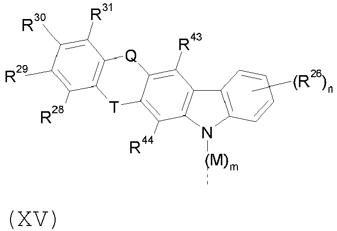
n étant 0, 1, 2, 3 ou 4,
m étant 0, 1, 2, 3 ou 4,
M étant un groupe C₆₋₄₀arylène qui est non substitué ou substitué par au moins un groupe E, un groupe C₁₋₂₄hétéroarylène qui est non substitué ou substitué par au moins un groupe E, un groupe C₁₋₂₅alkylène qui est non substitué ou substitué par au moins un groupe E et/ou interrompu par au moins un groupe D, R²⁶ étant indépendamment les uns des autres choisi parmi E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D,
ou au moins deux parmi R²⁶, si présents au niveau d'atomes de carbone adjacents, pouvant former au moins un cycle ou système cyclique saturé, insaturé, aromatique ou hétéroaromatique, substitué ou non substitué, à cinq ou six chaînons, auquel au moins un cycle ou système cyclique aromatique et/ou hétéroaromatique peut être condensé,
R²⁸, R²⁹, R³⁰, R³¹, R⁴³ et R⁴⁴ étant indépendamment les uns des autres H, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E, préférablement H,
ou
au moins deux parmi R²⁸, R²⁹, R³⁰ et R³¹, si présents au niveau d'atomes de carbone adjacents, pouvant former ensemble au moins un cycle ou système cyclique C₆₋₁₈aryle ou C₂₋₁₈hétéroaryle, et
Q et T étant indépendamment l'un de l'autre choisis parmi une liaison directe, S, O, SiR³²R³³, CR³⁴R³⁵ et NR³⁶, Q et T n'étant pas en même temps une liaison directe ;
R³² et R³³ étant indépendamment l'un de l'autre H, un groupe C₁-C₁₈alkyle, un groupe C₆-C₁₈aryle qui est non substitué ou substitué par au moins un groupe C₁-C₁₈alkyle, préférablement H, un groupe C₆-C₁₈aryle qui est non substitué ou un groupe C₁-C₁₈alkyle, plus préférablement H, méthyle, éthyle, ou phényle,
R³⁴ et R³⁵ étant indépendamment l'un de l'autre H, E, un groupe C₆-C₂₄aryle qui est non substitué ou substitué par au moins un groupe E, un groupe C₁₋₂₄hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E ou un groupe spiro, ou R³⁴ et R³⁵ ensemble pouvant former un cycle aliphatique substitué ou non substitué, à cinq ou six chaînons, préférablement R³⁴ et R³⁵ étant indépendamment l'un de l'autre H, méthyle, éthyle, phényle ou un groupe spiro, ou R³⁴ et R³⁵ ensemble pouvant former un cycle aliphatique substitué ou non substitué, à cinq ou six chaînons,
R³⁶ étant H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle, qui est interrompu par au moins un O, préférablement H, un groupe C₆-C₁₈aryle qui est non substitué ou un groupe C₁-C₁₈alkyle, par exemple phényle,
la ligne en pointillés étant un site de liaison, 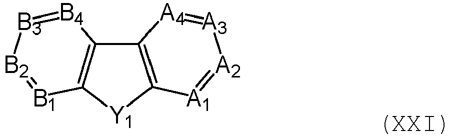
A¹ étant CR⁶² ou N,
A² étant CR⁶³ ou N,
A³ étant CR⁶⁴ ou N,
A⁴ étant CR⁶⁵ ou N,
B¹ étant CR⁶⁶ ou N,
B² étant CR⁶⁷ ou N,
B³ étant CR⁶⁸ ou N,
B⁴ étant CR⁶⁹ ou N,
Y¹ étant NR⁷⁰, CR⁷¹R⁷², O ou S ;
R⁶², R⁶³, R⁶⁴, R⁶⁵, R⁶⁶, R⁶⁷, R⁶⁸ et R⁶⁹ étant indépendamment les uns des autres H, une liaison directe, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E,
ou
au moins deux parmi R⁶², R⁶³, R⁶⁴ et R⁶⁵ pouvant être directement liés au fragment représenté par la formule générale (XXII) via les deux sites *; 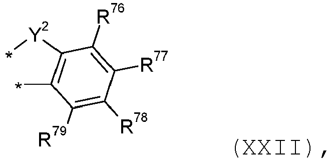
Y² étant NR⁷³, CR⁷⁴R⁷⁵, O ou S,
ou
au moins deux parmi R⁶⁶, R⁶⁷, R⁶⁸ et R⁶⁹ pouvant être directement liés au fragment représenté par la formule générale (XXIII) via les deux sites *, 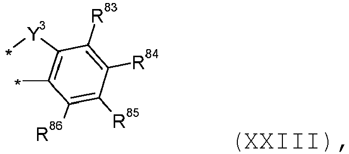
Y³ étant NR⁸⁰, CR⁸¹R⁸², O ou S,
R⁷⁰, R⁷³ et R⁸⁰ étant indépendamment les uns des autres une liaison directe, H, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E ou
R⁷¹, R⁷², R⁷⁴, R⁷⁵, R⁸¹ et R⁸² étant indépendamment les uns des autres H, une liaison directe, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E ou un groupe C₅₋₁₂cycloalkyle qui est non substitué ou substitué par au moins un groupe E,
ou R⁷¹ et R⁷², R⁷⁴ et R⁷⁵ et/ou R⁸¹ et R⁸² formant ensemble au moins un cycle ou système cyclique C₃₋₁₈alkyle auquel au moins un cycle ou système cyclique C₆₋₁₈aryle peut être fixé, R⁷⁶, R⁷⁷, R⁷⁸, R⁷⁹, R⁸⁴, R⁸⁵ et R⁸⁶ étant indépendamment les uns des autres H, une liaison directe, E, un groupe C₆₋₄₀aryle qui est non substitué ou substitué par au moins un groupe E, un groupe C₁₋₃₀hétéroaryle qui est non substitué ou substitué par au moins un groupe E, un groupe C₁₋₂₅alkyle qui est non substitué ou substitué par au moins un groupe E et/ou interrompu par au moins un groupe D, un C₇₋₂₅aralkyle qui est non substitué ou substitué par au moins un groupe E, un groupe C₅₋₁₂cycloalkyle qui est non substitué ou substitué par au moins un groupe E,
ou
R⁷⁶ et R⁷⁷, R⁷⁷ et R⁷⁸ et/ou R⁷⁸ et R⁷⁹ formant ensemble au moins un cycle ou système cyclique C₆-C₁₈aryle ou C₂-C₁₈hétéroaryle, et/ou
R⁸³ et R⁸⁴, R⁸⁴ et R⁸⁵ et/ou R⁸⁵ et R⁸⁶ ensemble pouvant former au moins un cycle ou système cyclique C₆-C₁₈aryle ou C₂-C₁₈hétéroaryle,
le groupe hétérocyclique selon la formule générale (XXI) étant relié au composé selon la formule générale (I) via l'un parmi R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸⁴, R⁸⁵ et R⁸⁶, ce résidu respectif R⁷⁰, R⁷³, R⁸⁰, R⁶², R⁶³, R⁶⁴, R⁶⁵, R⁶⁶, R⁶⁷, R⁶⁸, R⁶⁹, R⁷⁶, R⁷⁷, R⁷⁸, R⁷⁹, R⁷¹, R⁷², R⁷⁴, R⁷⁵, R⁸¹, R⁸², R⁸⁴, R⁸⁵ ou R⁸⁶ étant une liaison directe, éventuellement interrompue par un groupe de formule -(M)ₘ-, dans ce cas,
m étant 1, 2, 3 ou 4,
M étant un groupe C₆₋₄₀arylène qui est non substitué ou substitué par au moins un groupe E, un groupe C₁₋₂₄hétéroarylène qui est non substitué ou substitué par au moins un groupe E ou un groupe C₁₋₂₅alkylène qui est non substitué ou substitué par au moins un groupe E et/ou interrompu par au moins un groupe D.

2. Composition selon la revendication 1, le composé de formule (XX) étant représenté par les formules suivantes (XX-1) ou (XX-2) : 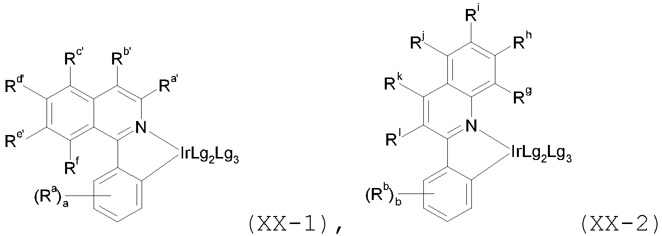 Lg₂ et Lg₃ étant un groupe A-1, A-2 ou A-3, où dans la formule (XX-1), au moins l'un parmi Lg₂ et Lg₃ est un groupe A-3, préférablement l'un parmi Lg₂ et Lg₃ est un groupe A-3 et l'autre est un groupe A-1 ; et dans la formule (XX-2), au moins l'un parmi Lg₂ et Lg₃ est un groupe A-3, préférablement l'un parmi Lg₂ et Lg₃ est un groupe A-3 et l'autre est un groupe A-2.

3. Composition selon la revendication 1 ou 2, le composé de formule (XX) étant représenté par l'une des formules suivantes (XX-3) et (XX-4) :

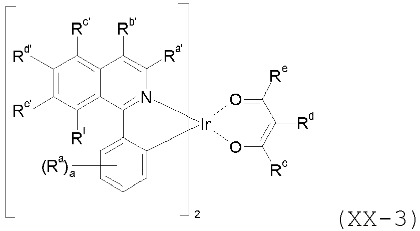

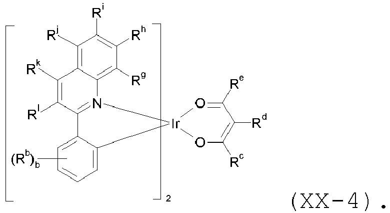

4. Composition selon la revendication 3, les composés des formules (XX-3) et (XX-4) étant représentés par les formules suivantes (XX-5) et (XX-6) : 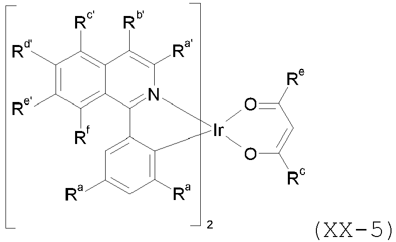 et 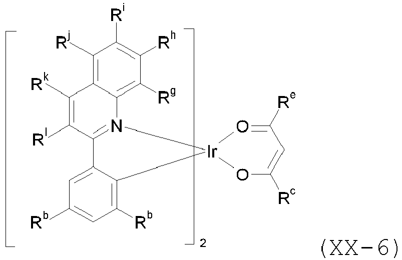 R^{a} et R^{b} représentant chacun indépendamment un groupe C₁-C₂₅alkyle substitué ou non substitué.

5. Composition selon la revendication 4, les composés des formules (XX-5) et (XX-6) étant représentés par les formules suivantes (XX-7) et (XX-8) : 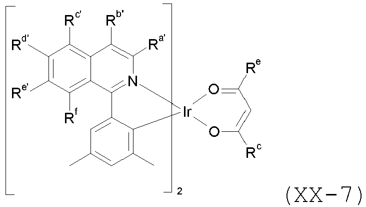 et 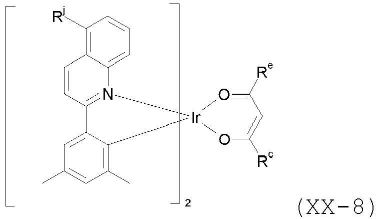
un ou deux parmi R^{a'}, R^{b'}, R^{c'}, R^{d'}, R^{e'} et R^{f} représentant chacun indépendamment CH(CH₃)₂, CH₂CH(CH₃)₂, CH₂C(CH₃)₃, CH(CH₂CH₃)₂, CF₃, CH₂CH₂CF₃, CH₂CH₂CH(CF₃)₂, cyclopentyle, cyclohexyle, éthyle, triméthylsilyle, triéthylsilyle, triisopropylsilyle, CH[Si(CH₃)₃]₂, triméthylgermyle, triéthylgermyle ou triisopropylgermyle ;
et les groupes restants parmi R^{a'}, R^{b'}, R^{c'}, R^{d'}, R^{e'} et R^{f} étant hydrogène ;
R^{j} étant CH(CH₃)₂, CH₂CH(CH₃)₂, CH₂C (CH₃)₃, CH(CH₂CH₃)₂, CF₃, CH₂CH₂CF₃, CH₂CH₂CH(CF₃)₂, cyclopentyle, cyclohexyle, éthyle, triméthylsilyle, triéthylsilyle, triisopropylsilyle, CH[Si(CH₃)₃]₂, triméthylgermyle, triéthylgermyle ou triisopropylgermyle ;
et
R^{c} et R^{e} étant indépendamment l'un de l'autre méthyle, CH(CH₃)₂, CH(CH₂CH₃)₂ ou CH₂CH(CH₃)₂.

6. Composition selon l'une quelconque des revendications 1 à 5, l'un parmi R², R⁴, R⁵, R⁶, R⁷, R⁸, R⁹, R¹⁰ dans le composé de formule (I) étant un groupe N-hétéroaryle selon la formule générale (XIV) 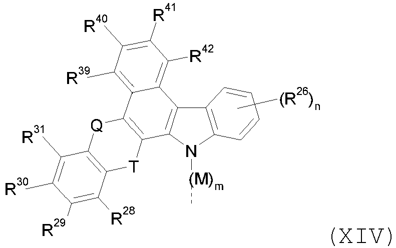
R³⁹, R⁴⁰, R⁴¹, R⁴² étant indépendamment les uns des autres choisis parmi H, un groupe C₆-C₁₈aryle qui est non substitué ou substitué par au moins un groupe C₁-C₁₈alkyle ou au moins un groupe C₁-C₁₈alcoxy, un groupe C₁-C₁₈alkyle ou un groupe C₁-C₁₈alkyle qui est interrompu par au moins un O, préférablement H, un groupe C₆-C₁₈aryle qui est non substitué ou un groupe C₁-C₁₈alkyle, plus préférablement R³⁹, R⁴⁰, R⁴¹ et R⁴² étant H,
la ligne en pointillés étant un site de liaison.

7. Composition selon l'une quelconque des revendications 1 à 5, le groupe hétérocyclique selon la formule générale (XXI) étant représenté par l'une quelconque parmi les formules générales (XXIa), (XXIb) et (XXIc)

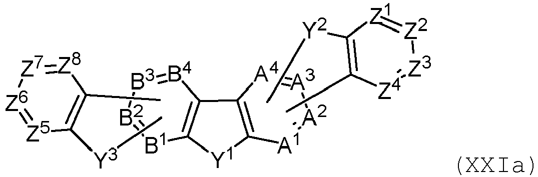

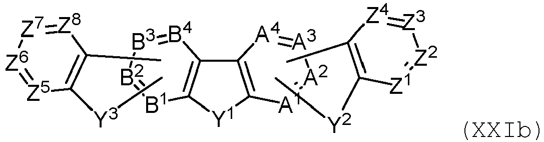

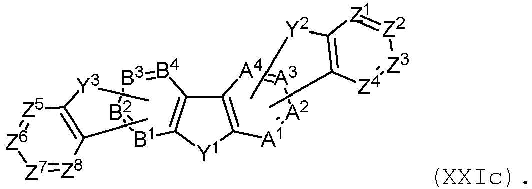

8. Composition selon la revendication 7, l'un parmi Y¹, Y² et Y³ étant respectivement NR⁷⁰, NR⁷³ ou NR⁸⁰.

9. Composition selon la revendication 8, deux parmi Y¹, Y² et Y³ étant respectivement NR⁷⁰, NR⁷³ ou NR⁸⁰.

10. Composition selon l'une quelconque des revendications 1 à 5, 7 et 8, le substituant selon la formule générale (XXI) étant relié au composé selon la formule générale (I) via R⁷⁰, R⁷³ ou R⁸⁰, ce résidu respectif R⁷⁰, R⁷³ ou R⁸⁰ étant une liaison directe, éventuellement interrompue par un groupe de formule -(M)ₘ-, dans ce cas,
m étant 1, 2, 3 ou 4,
M étant un groupe C₆₋₄₀arylène qui est non substitué ou substitué par au moins un groupe E, un groupe C₁₋₂₄hétéroarylène qui est non substitué ou substitué par au moins un groupe E, ou un groupe C₁₋₂₅alkylène qui est non substitué ou substitué par au moins un groupe E et/ou interrompu par au moins un groupe D.

11. Composition selon l'une quelconque des revendications 1 à 10, où dans les formules (XIII), (XIV), (XV), (XXI), (XXIa), (XXIb) ou (XXIc), m est 0 ou 1, préférablement 0, M est un groupe C₆₋₄₀arylène qui est non substitué ou substitué par au moins un groupe E, préférablement phénylène, et dans les formules (XIII), (XIV) ou (XV) au moins deux parmi R²⁶, si présents au niveau d'atomes de carbone adjacents, forment au moins un cycle saturé, insaturé, aromatique ou hétéroaromatique, substitué ou non substitué, à cinq ou six chaînons.

12. Composition selon l'une quelconque des revendications 1 à 11, R⁴, R⁵, R⁶, R⁷, R⁸, R⁹ et R¹⁰ étant H et R² étant un groupe N-hétéroaryle selon l'une des formules (XIII), (XIV), (XV), (XXI), (XXIa), (XXIb) et (XXIc) .

13. Composition selon l'une quelconque des revendications 1 à 6, 11 et 12,
la formule (XIII) étant représentée par 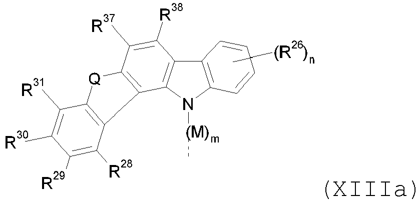 ou 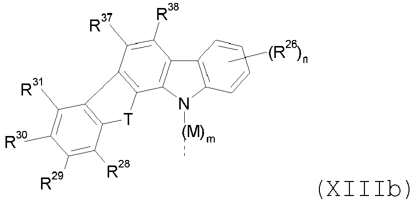 la formule (XV) étant représentée par 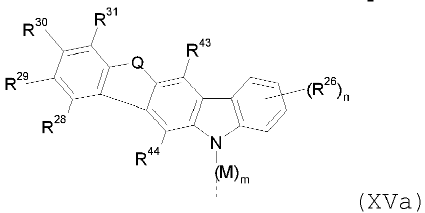 ou 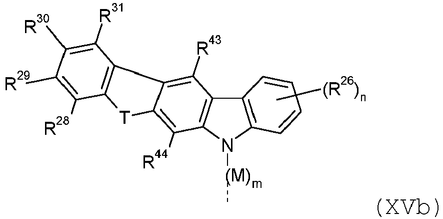 et la formule (XIV) étant représentée par

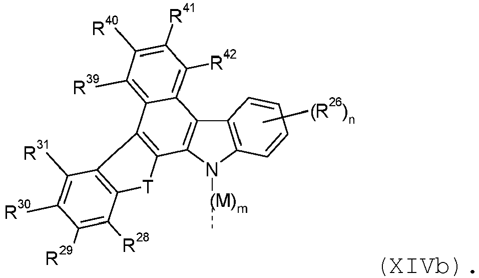

14. Composition selon la revendication 13,
la formule (XIII) étant représentée par 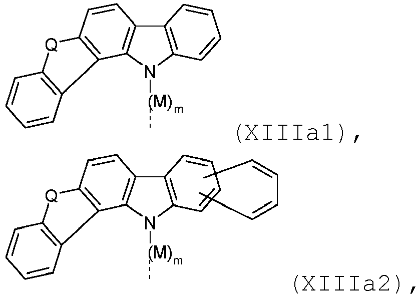 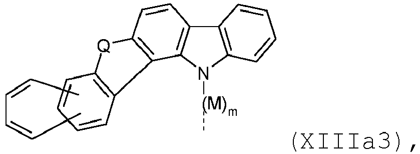 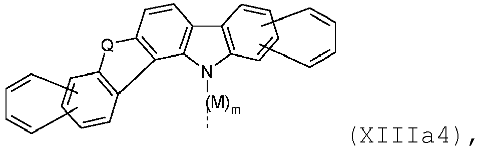 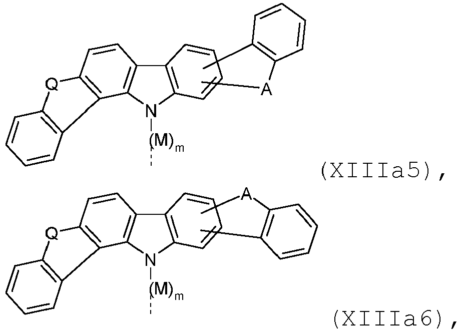 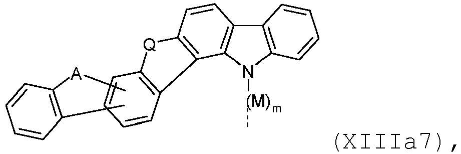 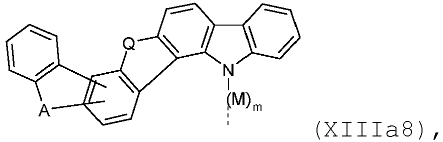 ou 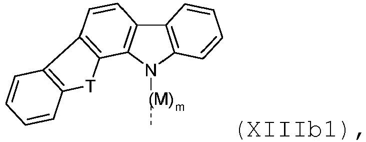 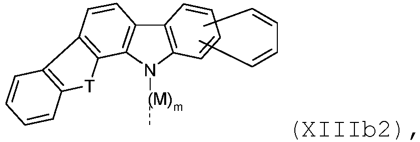 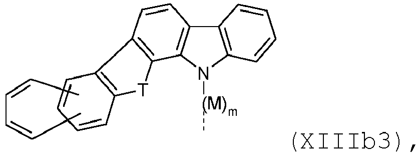 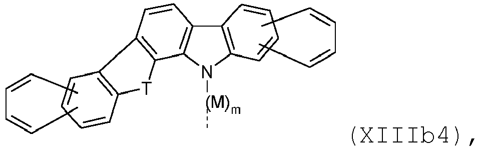 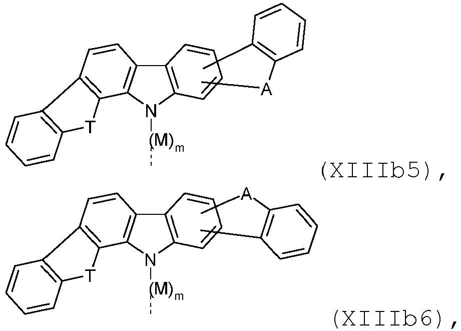 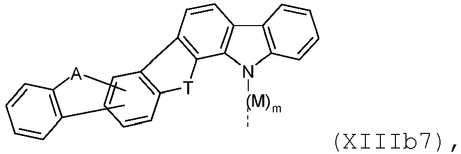 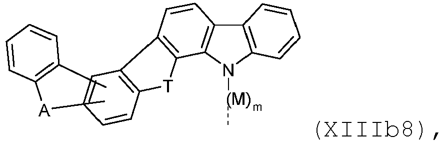 la formule (XV) étant représentée par 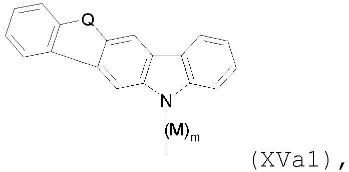 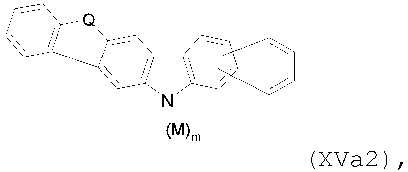 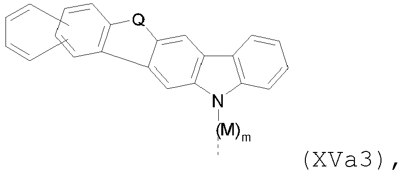 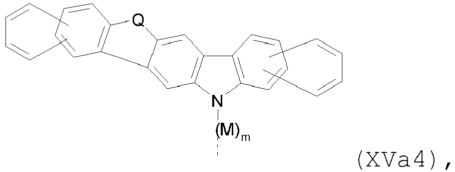 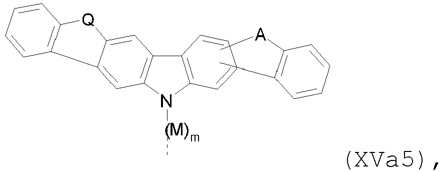 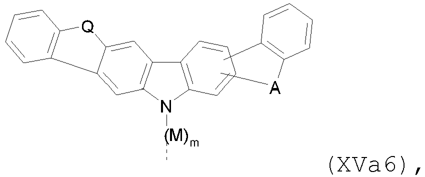 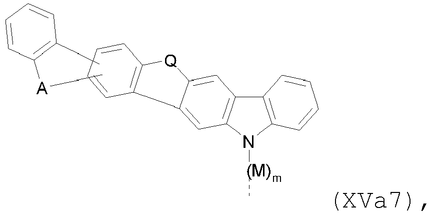 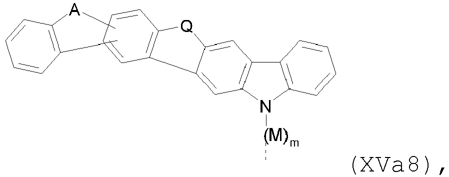 ou 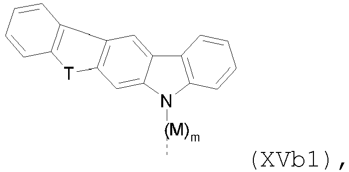 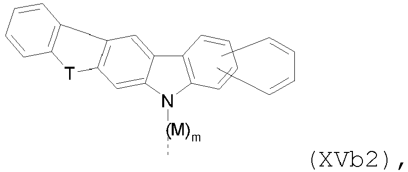 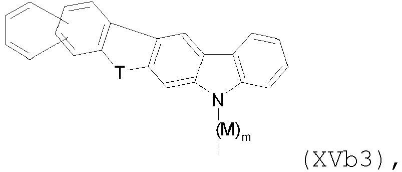 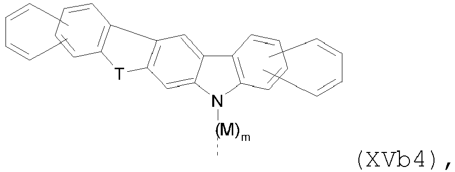 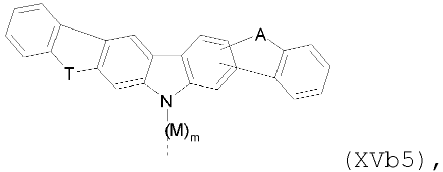 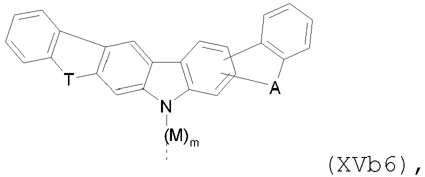 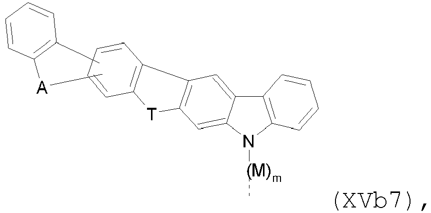 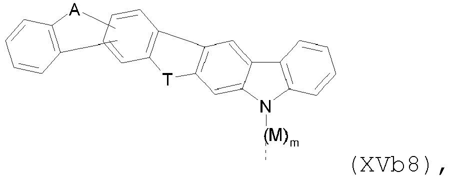 et la formule (XIVb) étant représentée par 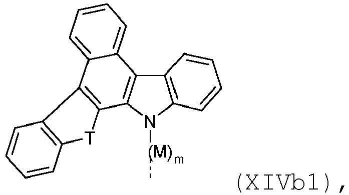 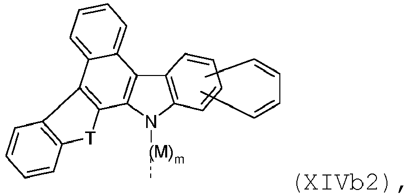 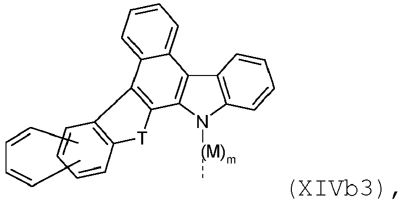 A étant S, O, SiR³²R³³, CR³⁴R³⁵ ou NR³⁶.

15. Composition selon la revendication 14, comprenant
i) au moins un complexe d'Ir représenté par la formule 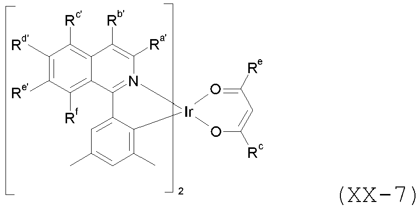 ou 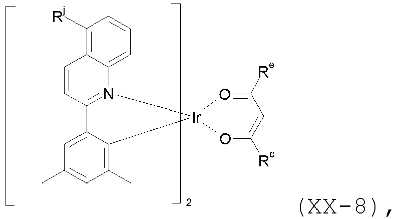 et
ii) au moins un composé représenté par l'une des formules (XIIIa1), (XIIIa2), (XIIIa3), (XIIIa4), (XIIIa5), (XIIIa6), (XIIIa7), (XIIIa8), (XIIIb1), (XIIIb2), (XIIIb3), (XIIIb4), (XIIIb5), (XIIIb6), (XIIIb7), (XIIIb8), (XIVb1), (XIVb2), (XIVb3), (XVa1), (XVa2), (XVa3), (XVa4), (XVa5), (XVa6), (XVa7), (XVa8), (XVb1), (XVb2), (XVb3), (XVb4), (XVb5), (XVb6), (XVb7) ou (XVb8).

16. Couche émettrice comprenant au moins un matériau dopant de formule (XX) tel que décrit selon l'une quelconque des revendications 1 à 5 et au moins un matériau hôte de formule (I) tel que décrit selon l'une quelconque des revendications 1 ou 6 à 14.

17. Dispositif électronique comprenant au moins un matériau dopant de formule (XX) tel que décrit selon l'une quelconque des revendications 1 à 5 et au moins un matériau hôte de formule (I) tel que décrit selon l'une quelconque des revendications 1 ou 6 à 14, ou comprenant la couche émettrice selon la revendication 16.

18. Dispositif électronique, préférablement dispositif organique d'électroluminescence, plus préférablement diode luminescente organique (OLED), comprenant une cathode, une anode, et une pluralité de couches de film mince organique disposée entre la cathode et l'anode, la pluralité de couches de film mince organique comprenant au moins une couche émettrice comprenant au moins un matériau dopant de formule (XX) tel que décrit selon l'une quelconque des revendications 1 à 5 et au moins un matériau hôte de formule (I) tel que décrit selon l'une quelconque des revendications 1 ou 6 à 14.
